# EUROPEAN PATENT SPECIFICATION

(11) **EP 3 568 470 B1**
(45) Date of publication and mention of the grant of the patent: **06.07.2022**
(21) Application number: 18738591.9
(22) Date of filing: 09.01.2018
(51) Int. Cl.: C12N 15/00, C12N 15/64, C12N 15/87, C12N 9/16, C12N 9/22

(54) **METHODS FOR IN VITRO SITE-DIRECTED MUTAGENESIS USING GENE EDITING TECHNOLOGIES**
VERFAHREN ZUR STELLENGERICHTETEN IN-VITRO-MUTAGENESE UNTER VERWENDUNG VON GEN-EDITING-TECHNOLOGIEN
PROCÉDÉS DE MUTAGENÈSE DIRIGÉE IN VITRO FAISANT APPEL À DES TECHNOLOGIES D'ÉDITION DE GÈNES

(30) Priority: 10.01.2017 US 201762444629 P; 02.06.2017 US 201762514494 P; 17.07.2017 US 201762533170 P
(43) Date of publication of application: 20.11.2019
(73) Proprietor: Christiana Care Health Services, Inc., Newark, DE 19713 (US); F.O.R.E Biotherapeutics Ltd, 91120 Jerusalem (IL)
(72) Inventor: KMIEC, Eric, Brian, Middletown, DE 19709 (US); VIDNE, Michael, 91120 Jerusalem (IL); TARCIC, Gabi, 91120 Jerusalem (IL)
(74) Representative: Straus, Alexander
(86) International application number: PCT/US2018/013009
(87) International publication number: WO 2018/132390

(56) References cited:
- WO-A1-2015/123339
- WO-A1-2016/100819
- CN-A- 105 132 451
- US-A1- 2003 199 091
- RIVERA-TORRES ET AL.: 'Insertional Mutagenesis by CRISPR/Cas9 Ribonucleoprotein Gene Editing in Cells Targeted for Point Mutation Repair Directed by Short Single-Stranded DNA Oligonucleotides' PLOS ONE vol. 12, no. 1, 04 January 2017, pages 1 - 18, XP055511561

<110> Christiana Care Health Services, Inc.
   NovelusDx Ltd.
   Kmiec, Eric
   Vidne, Michael
   Tarcic, Gabi
<120> Methods for In Vitro Site-Directed Mutagenesis Using Gene Editing
   Technologies
<130> 368477.00028
<150> 62/444,629
   <151> 2017-01-10
<150> 62/514,494
   <151> 2017-06-02
<150> 62/533,170
   <151> 2017-07-17
<160> 2
<170> PatentIn version 3.5
<210> 1
   <211> 15
   <212> DNA
   <213> Artificial sequence
<220>
   <223> Oligonucleotide
<400> 1
   aatggttgcg gccgc 15
<210> 2
   <211> 15
   <212> DNA
   <213> Artificial seqeunce
<220>
   <223> Oligonucleotide
<400> 2
   ccattgcggc cgcaa 15

## Description

### CROSS-REFERENCE TO RELATED APPLICATION

The present application claims priority under 35 U.S.C. § 119(e) to U.S. Provisional Patent Application No. 62/444,629, filed January 10, 2017, U.S. Provisional Patent Application No. 62/514,494, filed June 2, 2017, and U.S. Provisional Patent Application No. 62/533,170, filed July 17, 2017.

### BACKGROUND OF THE INVENTION

Mounting evidence indicates that growth of pathologically identical cancers in each individual patient is fueled by different sets of driving mutations. The need to identify these drivers stems from the recognized necessity for tailoring therapy and scheduling future surveillance. A major advancement in patient diagnosis is the use of next-generation sequencing to identify the cancer-causing mutations. However, functional characterization of patient mutations and their sensitivity to different targeted therapy drugs is needed.

One possible way to address this issue is to monitor the activity levels of signaling pathways by means of a transfected cell-based assay. As a functional platform, this system reveals activated pathways regardless of the type of mutation, *i.e.,* whether it is a known mutation or a Variant of Unknown Significance (VUS). A central step in this process is the synthesis of the patient mutations into plasmids, ready to be expressed in live cells that are then tested in the assay. Currently, there is a lack of *in-vitro* tools to generate mutations in an automated robust manner. Instead, a PCR-based site-directed mutagenesis is used, but this method requires multiple PCR steps and is therefore time consuming.

As an alternative to the lengthy serial procedure associated with PCR based methods, a technique that allows precise and rapid mutagenesis of numerous mutations at the same time is needed. One of the most progressive and promising gene editing methods is CRISPR/Cas9. This system is constructed by either using a plasmid base system from which the guide RNAs and Cas9 are expressed or using a ribonucleoprotein (RNP) complex in which the guide RNAs are coupled with purified Cas9 protein prior to inclusion in the reaction mixture.

WO 2015/123339 discloses methods and vectors for manipulation of chromosomes within open reading frames. WO 2015/123339 discloses methods comprising introducing components of the CRISPR system, including CRISPR-associated nuclease Cas9 and a sequence- specific guide RNA (gRNA), into cells, resulting in sequence-directed double stranded breaks using the ability of the CRISPR system to induce such breaks.

CN 105132451 discloses methods and products for gene editing. CN 105132451 discloses CRISPR/Cas9 single transcription unit for directed modification of a skeleton vector and its application. The CRISPR/Cas9 single transcription unit is designed for eukaryotes directed genetic modification.

Rivera-Torres et al. (2017) PLoS One., 12(1): 1-18, report mutagenesis by CRISPR/Cas9 ribonucleoprotein gene editing in cells targeted for point mutation repair directed by short single-stranded DNA oligonucleotides.

US 2003/0199091 discloses methods of enhancing oligonucleotide-mediated nucleic acid sequence alteration in vivo, ex vivo, and in vitro using cells or extracts with altered levels or activity of at least one protein from the RAD52 epistasis group, the mismatch repair group or the nucleotide excision repair group.

There is an unmet need in the art for a rapid and robust *in vitro* technique that allows simultaneous generation of mutations without requiring sequencing. The present invention satisfies this need.

### SUMMARY OF THE INVENTION

As described herein, the present invention relates to a kit and methods for *in vitro* mutagenesis.

One aspect of the invention includes a method of performing *in vitro* mutagenesis of a targeted sequence. In one embodiment, the method comprises incubating a mixture comprising an isolated ribonucleotide particle (RNP), a first plasmid, an oligonucleotide, and a cell extract having enzymatic activity for editing genes. The RNP comprises a crRNA and a Cas9 enzyme. The RNP may further comprises a tracrRNA. The tracrRNA may be annealed with the crRNA. The first plasmid comprises a nucleotide sequence of the targeted sequence, and the oligonucleotide comprises a nucleotide sequence that is complementary to the targeted sequence but contains at least one mismatched nucleotide, thus generating a second mutated plasmid. The second plasmid is administered to a plurality of non-germ line cells. At least one cell is selected from the plurality of non-germ line cells wherein *in vitro* mutagenesis has occurred in the targeted sequence.

Another aspect of the invention includes a method of performing *in vitro* mutagenesis of a plurality of targeted sequences. The method comprises incubating a mixture comprising a plurality of ribonucleotide particles (RNPs), a plurality of first plasmids, a plurality of oligonucleotides, and a cell-free extract. In one embodiment, the plurality of RNPs comprise a plurality of tracrRNAs, a plurality of crRNAs and a Cas9 enzyme, wherein the plurality of tracrRNAs are annealed with the plurality of crRNAs. In one embodiment, the first plurality of plasmids comprises a plurality of nucleotide sequences of the plurality of targeted sequences. In one embodiment, the plurality of oligonucleotides comprise a plurality of nucleotide sequences that are complementary to the plurality of targeted sequences but contain at least one mismatched nucleotide per targeted sequence, thus generating a plurality of second mutated plasmids. In one embodiment, the plurality of second mutated plasmids are administered to a plurality of non-germ line cells. In one embodiment, the plurality of cells wherein *in vitro* mutagenesis has occurred in the targeted sequences is selected.

In another aspect, the invention includes an *in vitro* mutagenesis kit for a targeted sequence comprising an isolated ribonucleotide particle (RNP), an oligonucleotide, a plasmid, a cell extract having enzymatic activity for editing genes The RNP comprises a crRNA and a Cas-endonuclease. The plasmid comprises a nucleotide sequence of a targeted sequence. The oligonucleotide comprises a nucleotide sequence that is complementary to the targeted sequence but contains at least one mismatched nucleotide as to the targeted sequence therein.

Yet another aspect of the invention includes a method of performing *in vitro* mutagenesis of a targeted sequence comprising incubating a first mixture comprising an isolated RNP and a plasmid. In one embodiment, the RNP comprises a crRNA and a Cpfl enzyme, wherein the crRNA is complementary to the targeted sequence. In one embodiment, the RNP generates a double stranded break in the plasmid. In one embodiment, the single stranded oligonucleotide comprises 5' overhangs complementary to the Cpfl cut site. In one embodiment, a re-circularized plasmid is generated. In one embodiment, the re-circularized plasmid is administered to a plurality of non-germ line cells. In one embodiment, selected from the plurality of cells is at least one cell wherein *in vitro* mutagenesis has occurred in the targeted sequence.

In another aspect, the invention includes a method of performing *in vitro* mutagenesis of a targeted sequence comprising incubating a mixture comprising an isolated RNP, a plasmid, a single stranded oligonucleotide, a cell extract having enzymatic activity for editing genes. In one embodiment, selected from the plurality of cells is at least one cell wherein *in vitro* mutagenesis has occurred in the targeted sequence.

In yet another aspect, the invention includes a method of performing *in vitro* mutagenesis of a targeted sequence comprising incubating a first mixture comprising a first isolated RNP, a second isolated RNP and a plasmid. In one embodiment, the first RNP comprises a crRNA complementary to a first target sequence and a first Cpfl enzyme. In one embodiment, the second RNP comprises a second crRNA complementary to a second target sequence and a second Cpfl enzyme. In one embodiment, the first RNP generates a first double stranded break in the plasmid and the second RNP generates a second double stranded break in the plasmid.

The invention includes a method of performing *in vitro* mutagenesis of a targeted sequence comprising incubating a mixture comprising an isolated ribonucleotide particle (RNP), a first plasmid, an oligonucleotide, and a cell extract having enzymatic activity for editing genes. The RNP comprises a crRNA and a Cas endonuclease. The first plasmid comprises a nucleotide sequence of the targeted sequence. The oligonucleotide comprises a nucleotide sequence that is complementary to the targeted sequence but contains at least one mismatched nucleotide. Thus, a second, mutated plasmid is generated. The second plasmid is administered to a plurality of non-germ line cells. From the plurality of cells at least one cell is selected, wherein *in vitro* mutagenesis has occurred in the targeted sequence.

Another aspect of the invention includes a method of performing *in vitro* mutagenesis of a plurality of targeted sequences comprising incubating a mixture comprising a plurality of ribonucleotide particles (RNPs), a plurality of first plasmids, a plurality of oligonucleotides, and a cell-free extract. In one embodiment, the plurality of RNPs comprise a plurality of tracrRNAs, a plurality of crRNAs and a Cas endonuclease. In one embodiment, the plurality of tracrRNAs are annealed with the plurality of crRNAs. In one embodiment, the first plurality of plasmids comprise a plurality of nucleotide sequences of the plurality of targeted sequences. In one embodiment, the plurality of oligonucleotides comprise a plurality of nucleotide sequences that are complementary to the plurality of targeted sequences but contain at least one mismatched nucleotide per targeted sequence. In one embodiment, a plurality of second mutated plasmids is generated. In one embodiment, the plurality of second plasmids are administered to a plurality of non-germ line cells. In one embodiment, the plurality of cells wherein *in vitro* mutagenesis has occurred in the targeted sequences is selected.

Yet another aspect of the invention includes a method of performing *in vitro* mutagenesis of a targeted sequence comprising incubating a first mixture comprising an isolated ribonucleotide particle (RNP) and a plasmid. In one embodiment, the RNP comprises a crRNA and a Cas endonuclease, wherein the crRNA is complementary to the targeted sequence. In one embodiment, the RNP generates a double stranded break in the plasmid. In one embodiment, the double stranded oligonucleotide comprises 5' overhangs complementary to the RNP cut site. In one embodiment, a re-circularized plasmid is generated. In one embodiment, the re-circularized plasmid is administered to a plurality of non-germ line cells. In one embodiment, selected from the plurality of cells is at least one cell wherein *in vitro* mutagenesis has occurred in the targeted sequence.

Still another aspect of the invention includes a method of performing *in vitro* mutagenesis of a plurality of targeted sequences comprising incubating a mixture comprising a plurality of ribonucleotide particles (RNPs), a plurality of first plasmids, a plurality of oligonucleotides, and a cell extract having enzymatic activity for editing genes. In one embodiment, the plurality of RNPs comprise a plurality of tracrRNAs, a plurality of crRNAs and a Cas endonuclease. In one embodiment, the plurality of tracrRNAs are annealed with the plurality of crRNAs. In one embodiment, the first plurality of plasmids comprise a plurality of nucleotide sequences of the plurality of targeted sequences. In one embodiment, the plurality of oligonucleotides comprise a plurality of nucleotide sequences that are complementary to the plurality of targeted sequences but contain at least one mismatched nucleotide per targeted sequence. In one embodiment, a plurality of second mutated plasmids is generated. In one embodiment, the plurality of second plasmids is administered to a plurality of non-germ line cells. In one embodiment, the plurality of cells wherein *in vitro* mutagenesis has occurred in the targeted sequences is selected.

In yet another aspect, the invention includes a method of performing *in vitro* mutagenesis of a targeted sequence comprising incubating a mixture comprising an isolated RNP, a plasmid, a single stranded oligonucleotide, a cell extract having enzymatic activity for editing genes. In one embodiment, the RNP comprises a crRNA and a Cas endonuclease, wherein the crRNA is complementary to the targeted sequence. In one embodiment, the RNP generates a double stranded break in the plasmid. In one embodiment, the single stranded oligonucleotide comprises 5' overhangs complementary to the RNP cut site. In one embodiment, a re-circularized plasmid is generated. In one embodiment, the re-circularized plasmid is administered to a plurality of non-germ line cells. In one embodiment, selected from the plurality of cells is at least one cell wherein *in vitro* mutagenesis has occurred in the targeted sequence.

Another aspect of the invention includes an *in vitro* mutagenesis kit for a targeted sequence comprising an isolated ribonucleotide particle (RNP), an oligonucleotide, a plasmid, a buffer, a cell extract having enzymatic activity for editing genes and instructional material for use thereof. The RNP comprises a tracrRNA, a crRNA and a Cas endonuclease. The plasmid comprises a nucleotide sequence of a targeted sequence. The oligonucleotide comprises a nucleotide sequence that is complementary to the targeted sequence but contains at least one mismatched nucleotide as to the targeted sequence therein.

Yet another aspect of the invention includes an *in vitro* mutagenesis kit for a targeted sequence comprising an isolated ribonucleotide particle (RNP), an oligonucleotide, a plasmid, a buffer, a cell extract, and instructional material for use thereof. In one embodiment, the RNP comprises a crRNA and a Cas endonuclease, wherein the crRNA is complementary to the targeted sequence. In one embodiment, the oligonucleotide comprises 5' overhangs complementary to the RNP cut site.

In various embodiments of the above aspects or any other aspect of the invention delineated herein, the *in vitro* mutagenesis comprises at least one mutation in the nucleotide sequence of the targeted sequence selected from the group consisting of a single base nucleotide modification, a deletion, and an insertion. In another embodiment, the cell extract is derived from at least one cell selected from the group consisting of HEK, HUH-7, DLD1, HCT116, and S. cerevisiae.

In one embodiment, the mixture is incubated at about 37°C for about 120 minutes. In another embodiment, each oligonucleotide is independently between about 25 and about 200 bases in length. In yet another embodiment, each oligonucleotide is independently about 72 bases in length. In still another embodiment, each oligonucleotide independently further comprises a chemically modified terminal linkage. In one embodiment, the chemically modified terminal linkage comprises a 2'-O-methyl modification.

In certain embodiments, the oligonucleotide further comprises a NotI restriction site. In one embodiment, the selecting comprises digesting the cell with a NotI enzyme.

In certain embodiments, the kit or method of the invention further comprises a second RNP comprising a second crRNA complementary to a second targeted sequence.

In certain embodiments, the Cas endonuclease is selected from the group consisting of Cas9, Cas3, Cas8a, Cas8b, Cas10d, Cse1, Csy1, Csn2, Cas4, Cas10, Csm2, Cmr5, Fok1, T7, spCas9, Cpfl, Cpf2, CasY, CasX, and saCas9.

### BRIEF DESCRIPTION OF THE DRAWINGS

For the purpose of illustrating the invention, there are depicted in the drawings certain embodiments of the invention. However, the invention is not limited to the precise arrangements and instrumentalities of the embodiments depicted in the drawings.
FIG. 1 is a schematic depicting an illustrative RNP assembly method used in the present invention.
FIG. 2 is a gel image showing DNA cleavage by a purified RNP complex.
FIG. 3 displays results of an experiment designed to show the specificity of RNP-directed cleavage.
FIG. 4 is a gel image demonstrating enzyme activity after subtracting individual components of the RNP complex.
FIG. 5 is a gel image demonstrating the activity of the mammalian cell free extract on the product of RNP cleavage.
FIG. 6 is a schematic displaying the initialization and validation strategy of the genetic readout system of the present invention.

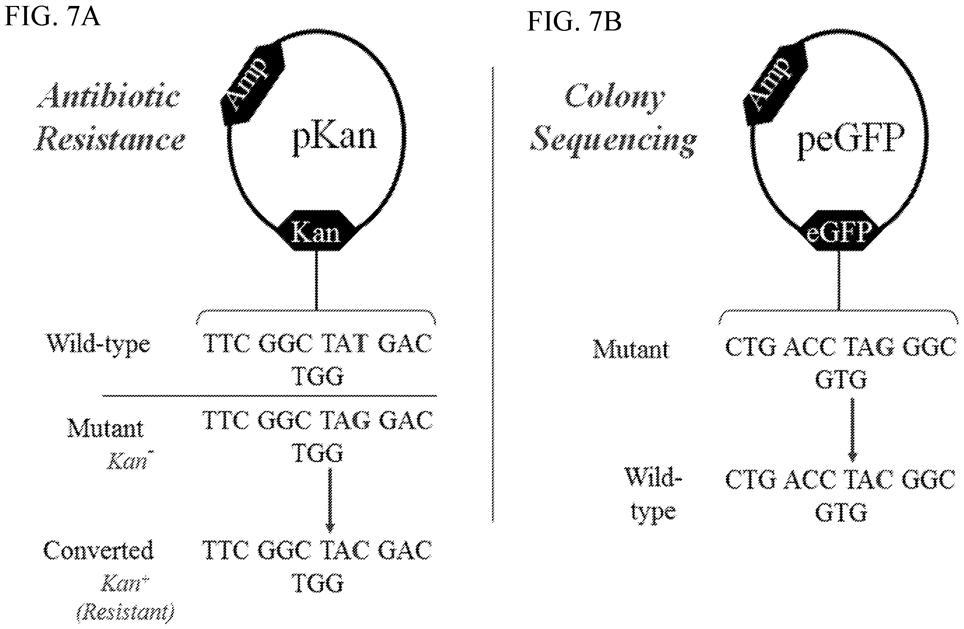

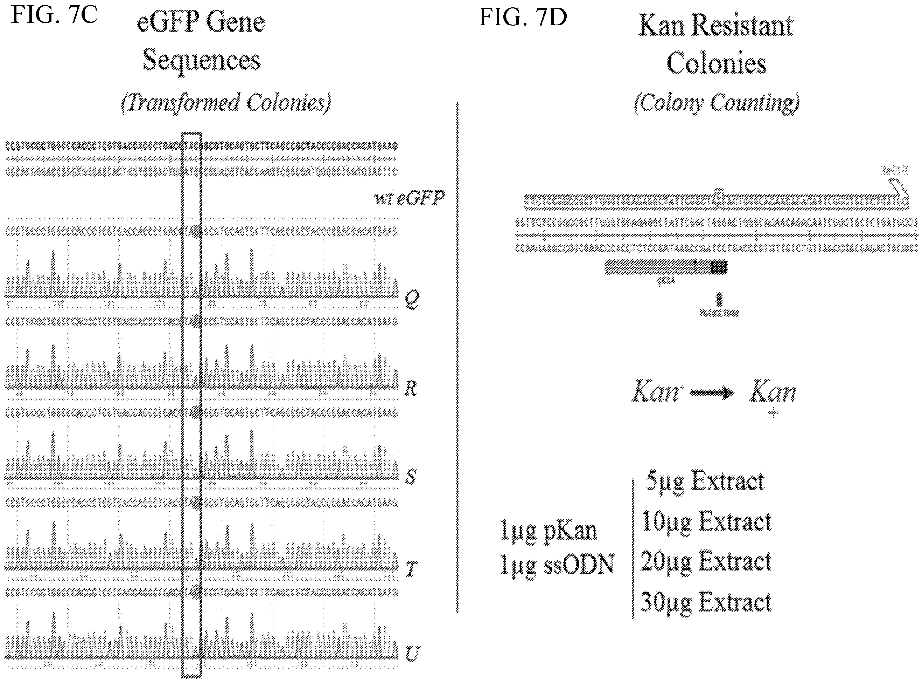

FIGs. 7A-7D are a set of images displaying targeted nucleotides for two exemplary genetic readouts demonstrated in the present invention. FIG. 7A shows the first readout system: conversion of kanamycin sensitivity to kanamycin resistance through correction of a G residue to a T residue in the plasmid pKan. FIG. 7B shows the other plasmid, which does not contain an antibiotic resistance gene for easy selection, but bears an ampicillin resistance gene in wild type form for screening of cells that have received the plasmid after transformation. In this case, the target is an eGFP gene bearing a stop codon whereupon conversion from the third-base, G, to a C, generates the wild type eGFP. FIG. 7C illustrates a panel of sequences that show the target base in the eGFP *in vitro* mutagenesis system. Starting plasmid clones listed as Q, R, S, T and U represent reference sequences for the targeted base which, in this case is G . Conversion to C (wild type sequence) produces the predicted outcome and is illustrated at the top. FIG. 7D illustrates the DNA sequence of the mutant kanamycin target and the conversion from kanamycin sensitivity to kanamycin resistance (G to C). The composition of a typical reaction mixture is provided below.
FIGs. 8A-8B are a series of images displaying representative DNA sequencing results from plasmid DNA modified *in vitro* and isolated from clones.
FIG. 9 is a gel image illustrating optimization of reaction conditions.
FIGs. 10A-10C illustrate a non-limiting proposed mechanism of NotI insertion. FIG. 10A shows the NotI restriction enzyme cut site. FIG. 10B shows the Cpf1-1228 RNP staggered double stranded cut site on the lacZ gene, indicated by arrows. The duplexed NotI insertion segment is incorporated into the gene sequence utilizing complementary arms to the overhangs produced by Cpfl cleavage. FIG. 10C shows the outlined steps of the *in vitro* reaction used herein to incorporate the NotI insertion into a gene segment.
FIGs. 11A-11B illustrate a NotI digestion assay to confirm the insertion of the NotI site. Plasmid isolated from *in vitro* duplexed NotI insertion reactions was transformed into DH5α competent *E. coli.* FIG. 11A shows results from pooled bacterial colony DNA subjected to NotI enzyme digestion. FIG. 11B shows results from individual bacterial colony DNA subjected to NotI enzyme digestion.
FIG. 12 illustrates NotI control assays to confirm the insertion of the NotI site. Without the addition of CFE and ligase to the *in vitro* NotI insertion reactions, there is no cleavage activity seen after bacterial colony DNA is subject to NotI enzyme digestion, indicating no NotI insertion had occurred. Additional reactions show NotI insertions were seen at a lower frequency when either of the single-stranded NotI oligonucleotides, NotI-S or NotI-NS, were incorporated into the *in vitro* reactions instead of the duplexed NotI insert.
FIG. 13 illustrates a NotI digestion assay to confirm the insertion of the NotI site using NotI-S ssODN. Plasmid isolated from *in vitro* single-stranded NotI oligonucleotide NotI-S insertion reactions was transformed into DH5α competent *E. coli.* Pooled and individual bacterial colony DNA was subject to NotI enzyme digestion. DNA containing NotI cut sites showing cleavage activity after NotI digestion are indicated by a star.
FIG. 14 illustrates a NotI digestion assay to confirm the insertion of the NotI site using NotI-NS ssODN. Plasmid isolated from *in vitro* single-stranded NotI oligonucleotide NotI-NS insertion reactions was transformed into DH5α competent *E*. *coli.* Pooled and individual bacterial colony DNA was subject to NotI enzyme digestion. DNA containing NotI cut sites showing cleavage activity after NotI digestion are indicated by a star.
FIG. 15 illustrates sequencing analysis showing two clones containing a perfect NotI site insertion at the Cpfl RNP cut site.
FIG. 16 illustrates NotI insertions containing deletions. Sequencing analysis shows two clones containing a single NotI site insertion that had also incurred a 1 bp deletion at the Cpfl RNP cut site.
FIG. 17 illustrates NotI insertions containing deletions. Sequencing analysis shows one clone containing a single NotI site insertion that had also incurred a 6 bp deletion upstream from the Cpfl RNP cut site.
FIG. 18 illustrates NotI insertions containing deletions. Sequencing analysis shows one clone containing a single NotI site insertion that had also incurred a 7 bp deletion upstream from the Cpfl RNP cut site.
FIG. 19 illustrates NotI insertions containing deletions. Sequencing analysis shows one clone containing a single NotI site insertion that had also incurred a 9 bp deletion upstream from the Cpfl RNP cut site.
FIG. 20 illustrates NotI insertions containing deletions. Sequencing analysis shows one clone containing a single NotI site insertion that had also incurred a 7 bp deletion downstream from the Cpfl RNP cut site.
FIG. 21 illustrates multiple NotI insertions containing deletions. Sequencing analysis shows three clones containing double NotI site insertions that had also incurred a 1 bp deletion at the Cpfl RNP cut site.
FIG. 22 illustrates NotI insertions containing deletions. Sequencing analysis shows one clone containing a triple NotI site insertion that had also incurred a 1 bp deletion at the Cpfl RNP cut site.

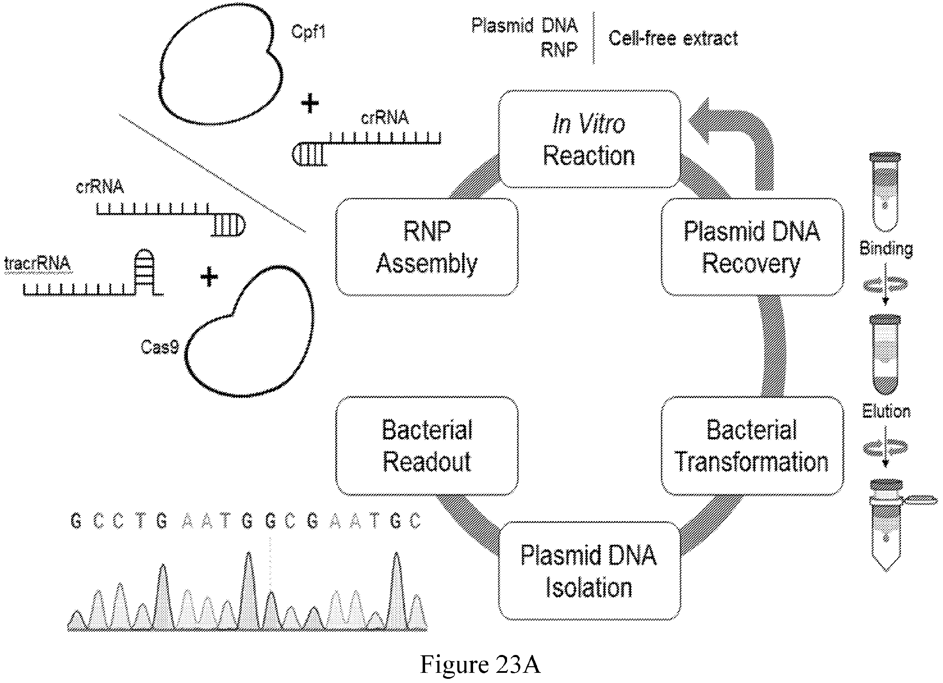

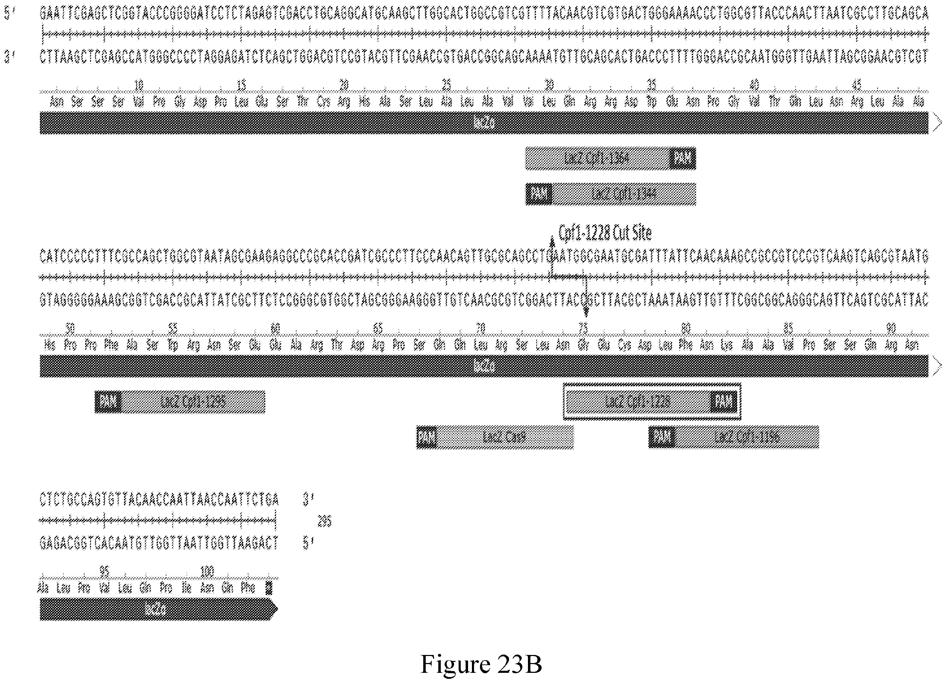

FIGs. 23A-23B are a series of images illustrating an *in vitro* gene editing experimental protocol and tools used herein. FIG. 23A shows Cpfl or Cas9 RNPs are complexed and added to a first *in vitro* cleavage reaction mixture with plasmid DNA. Plasmid DNA is recovered and added to a second *in vitro* recircularizing reaction mixture with cell extract. After the reaction is complete, plasmid DNA is recovered from the reaction and transformed into competent *E. coli.* DNA is then isolated from transformed cells and sequenced to identify modifications made *in vitro.* FIG. 23B shows a variety of Cpfl and Cas9 RNP sites across the lacZ gene region of pHSG299. One Cas9 binding site and five Cpfl binding sites are shown. The Cpfl site used for *in vitro* reactions described herein is indicated within a box, and the associated cut site is marked by a staggered arrow.

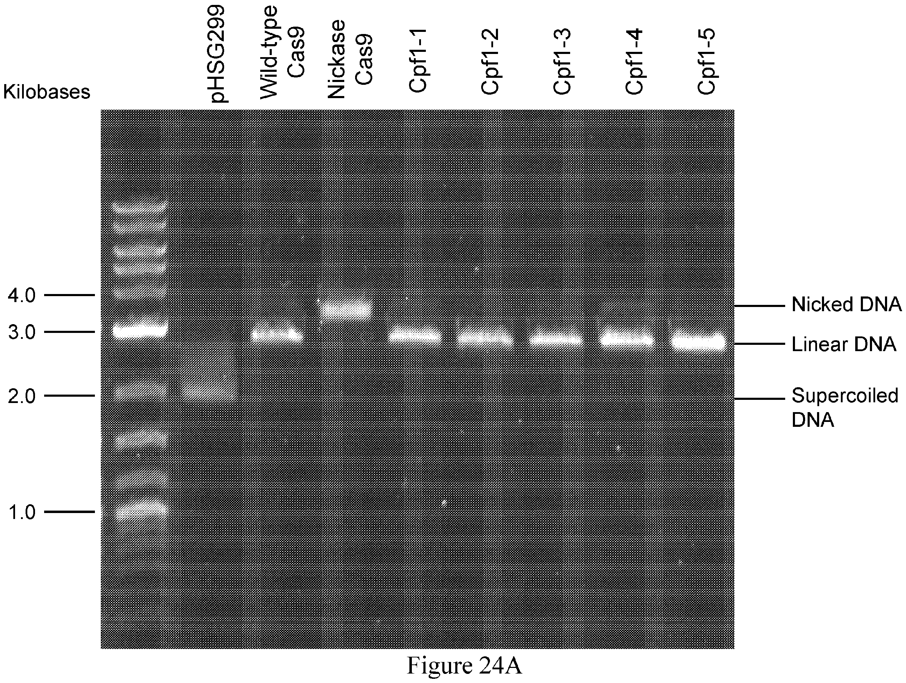

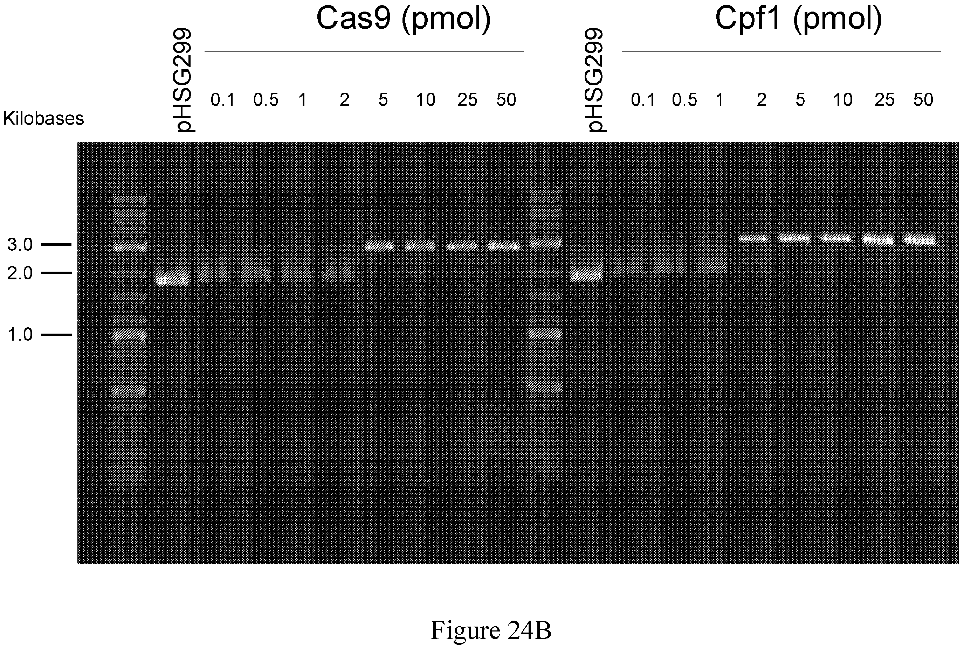

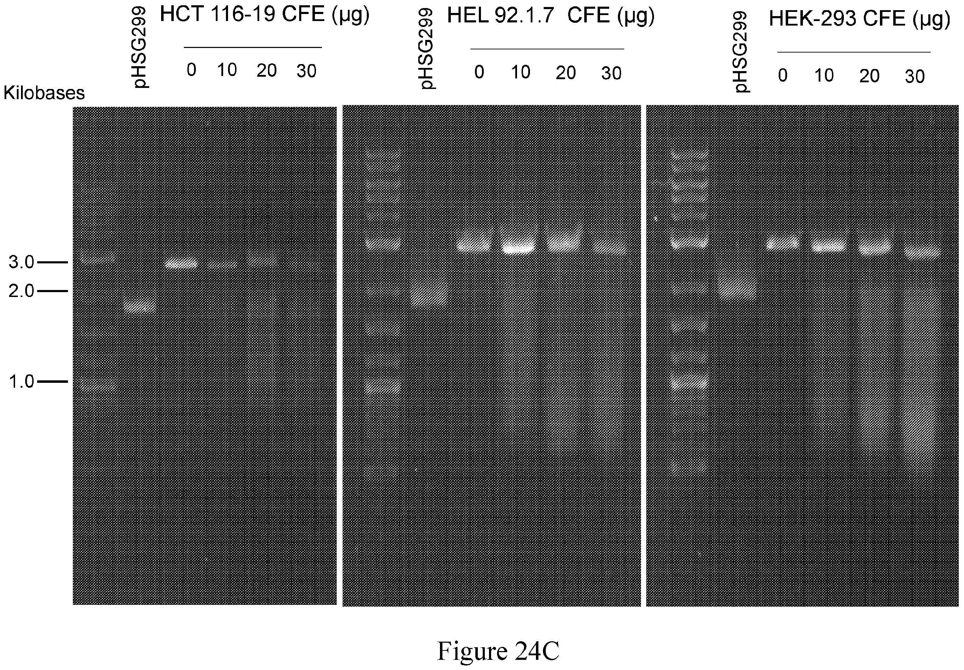

FIGs. 24A-24C are a series of gel images illustrating RNP cleavage and cell-free extract activity *in vitro.* FIG. 24A shows cleavage of pHSG299 was achieved using wild-type Cas9, nickase Cas9 and five Cpfl RNPs, each having distinct crRNA sequences. The varied conformations of DNA cleavage products can be distinguished by the degree of migration of supercoiled, linear and nicked DNA through the agarose gel, as shown on the right. FIG. 24B shows the relative cleavage activities of Cas9 and Cpfl assessed under *in vitro* reaction conditions at increasing RNP amounts ranging from 0.1 - 50pmol. FIG. 24C shows increasing amounts of cell-free extracts from three mammalian cell line sources (HCT 116-19 (colon), HEL 92.1.7 (erythroblast) and HEK-293 (kidney)) were added to *in vitro* cleavage reactions containing a Cpfl RNP, plasmid DNA, and increasing amounts of respective cell-free extract. As the amount of each cell-free extract was increased, DNA fragments were generated at the RNP cut site as the exposed DNA ends were degraded and reaction activity was visible as smeared lines running down gel lanes.

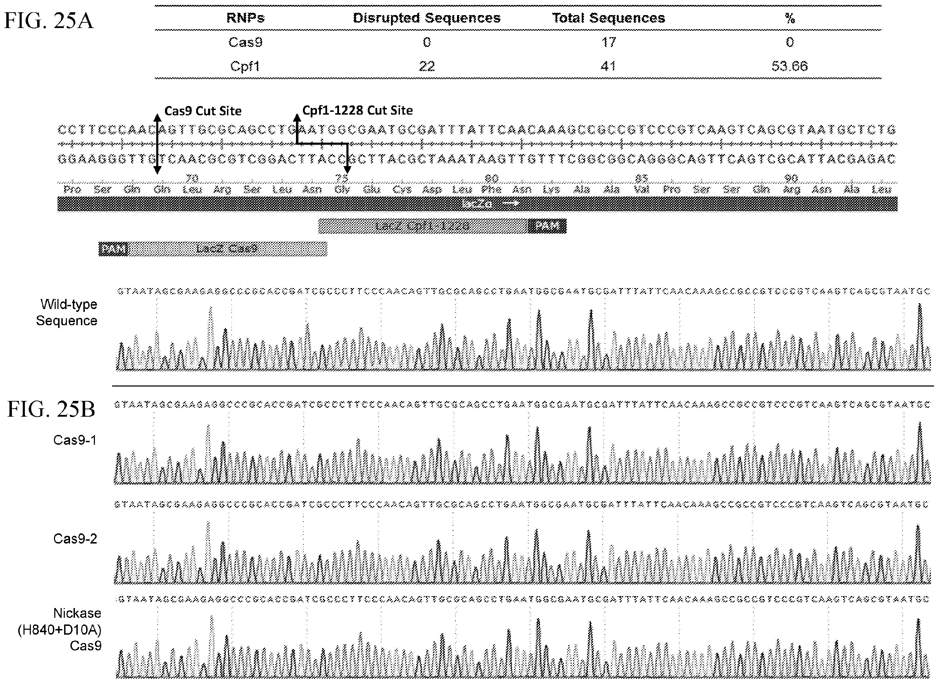

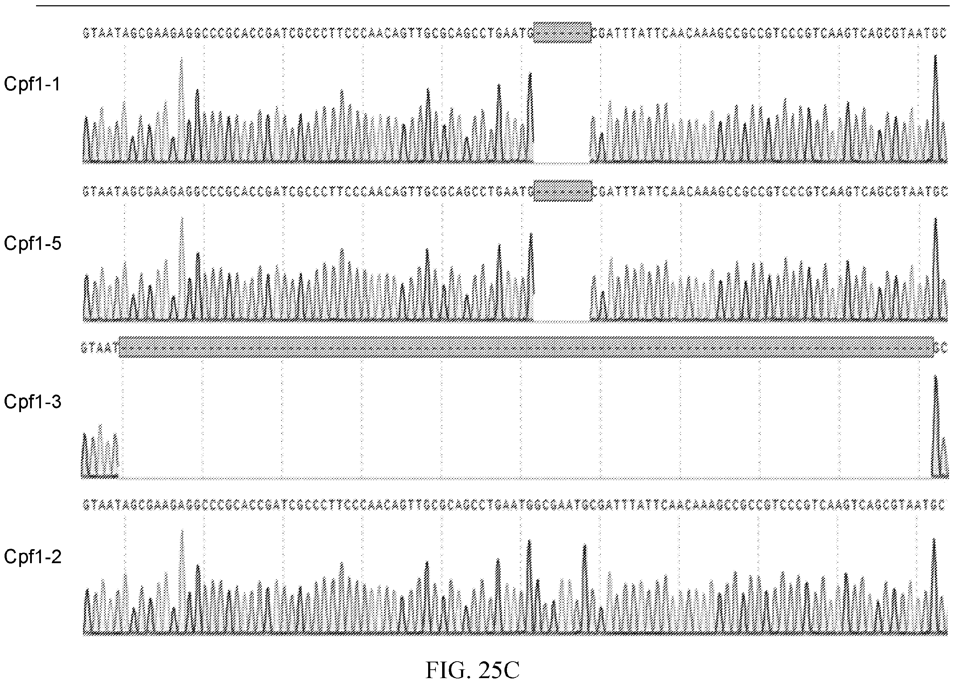

FIGs. 25A-25C are a series of images illustrating Cas9 and Cpfl RNP activity *in vitro.* FIG. 25A shows the frequency of DNA disruption by Cas9 and Cpfl RNPs as a percentage of the number of disrupted DNA sequences detected in relation to the total number of sequences analyzed from bacterial colonies transformed with plasmid DNA recovered from *in vitro* reactions. Cpfl and Cas9 sites are shown along the lacZ gene region with associated cleavage sites marked by staggered and straight arrows, respectively. The wild-type sequence of the lacZ gene region is shown. FIG. 25B shows three sequences representative of the total number assessed from *in vitro* reactions containing Cas9 RNPs with no DNA disruption around the cleavage site. FIG. 25C shows five sequences representative of the total number of sequences assessed from *in vitro* reactions containing Cpfl RNPs displaying a variety of DNA disruption around the cleavage site.

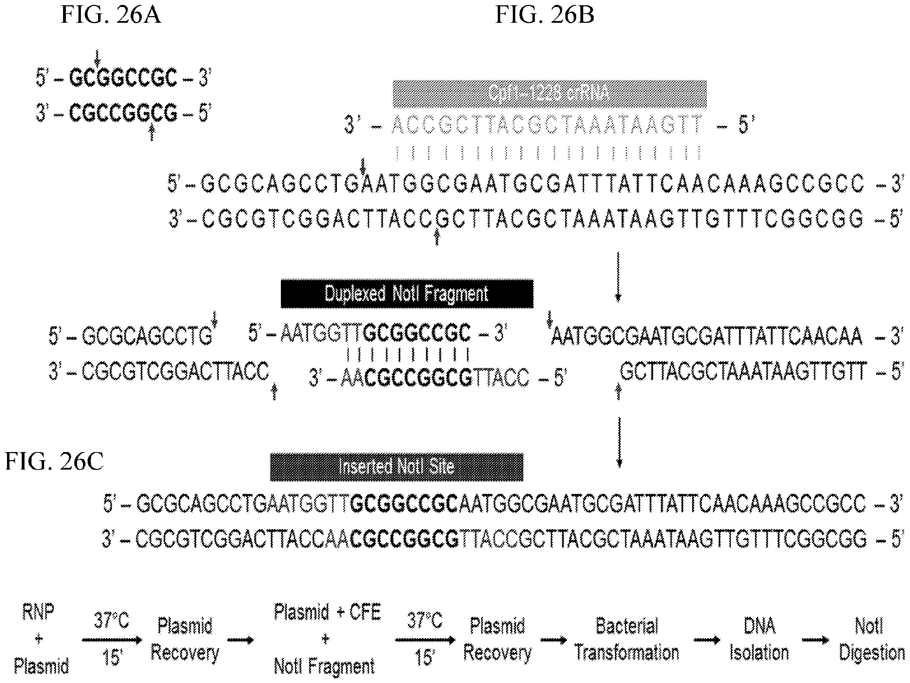

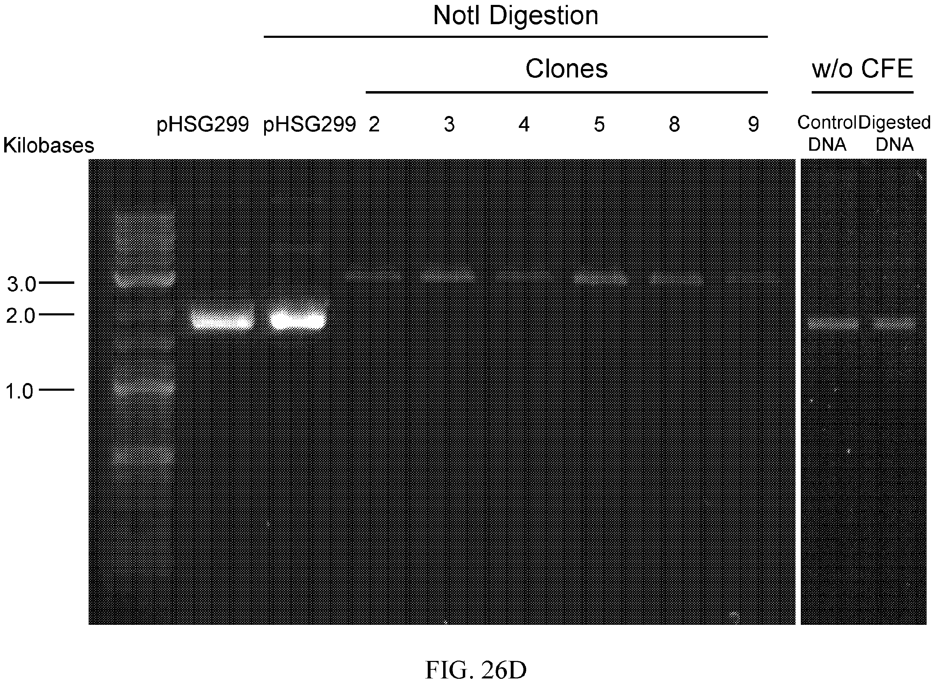

FIGs. 26A-26D are a series of images illustrating a proposed mechanism and verification of duplexed NotI fragment insertion. FIG. 26A is an illustration of the NotI restriction cut site. FIG. 26B shows the Cpfl RNP staggered double-stranded cleavage site on the lacZ gene indicated by arrows. The duplexed NotI fragment is inserted into the gene sequence utilizing two arms complementary to the overhangs produced by Cpfl cleavage. FIG. 26C shows the outlined steps of the *in vitro* reaction inserting the NotI fragment into the lacZ gene region. FIG. 26D shows plasmid DNA isolated from selected bacterial colonies transformed with plasmids recovered from *in vitro* duplexed NotI fragment insertion reactions subject to NotI enzyme digestion to confirm the integration of the NotI site into the lacZ gene region. An additional control was carried out to confirm that in the absence of the modified cell-free extract as a component of the *in vitro* reaction mixture; NotI fragment insertion would not occur.

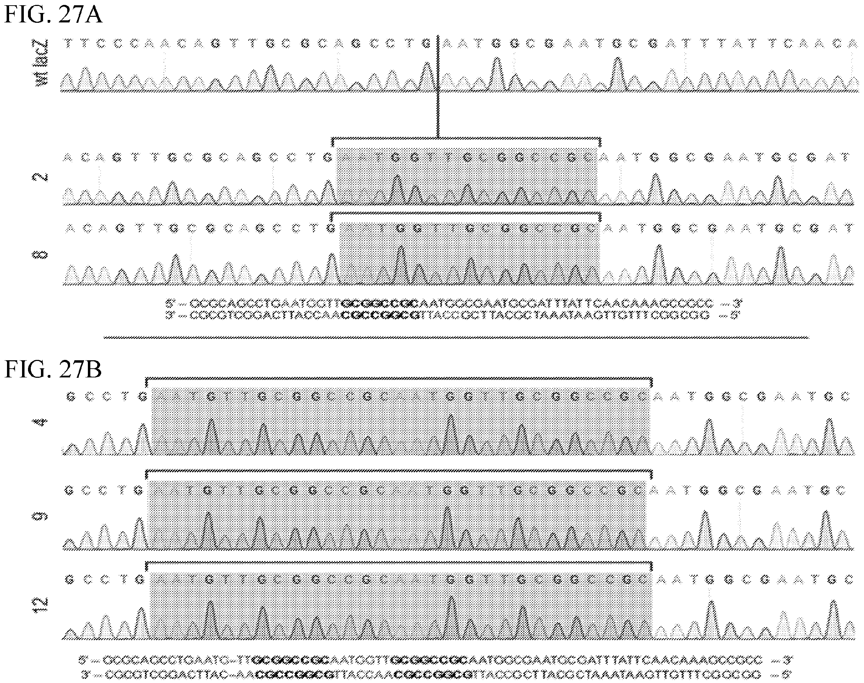

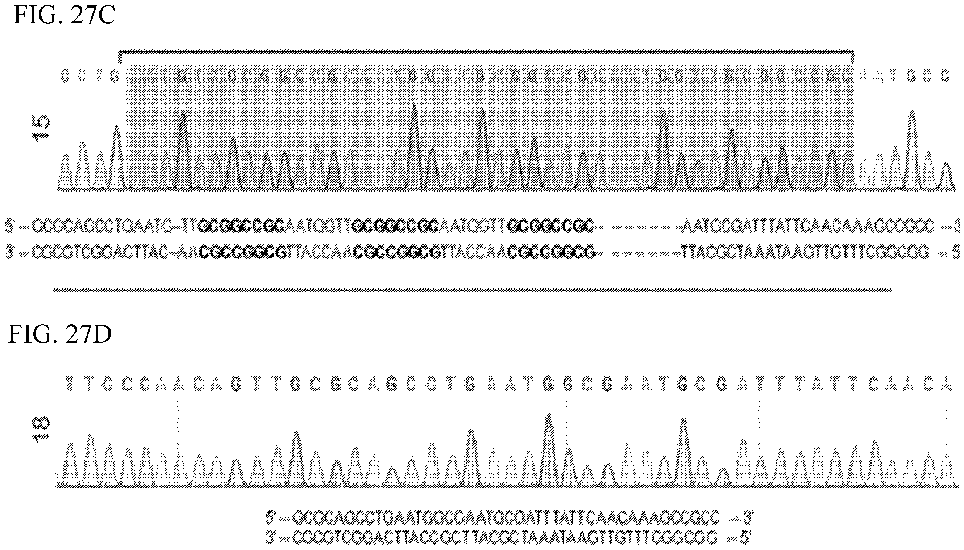

FIGs. 27A-27D are a series of traces illustrating duplexed NotI fragment insertion sequences. The sequence of the wild-type lacZ gene region and selected bacterial colonies transformed with plasmid DNA recovered from *in vitro* duplexed NotI fragment insertion reactions are shown. FIG. 27A shows sequencing analysis revealed two sequences that contained perfect NotI fragment insertion at the cleavage site. FIG. 27B shows three sequences that contained two NotI site fragment inserts accompanied by a 1bp deletion upstream from the cleavage site. FIG. 27C shows one sequence that contained three NotI site fragment inserts accompanied by a 1bp deletion upstream and a 7bp deletion downstream from the cleavage site. FIG. 27D shows one sequence that did not contain a NotI site fragment insertion at the cleavage site.

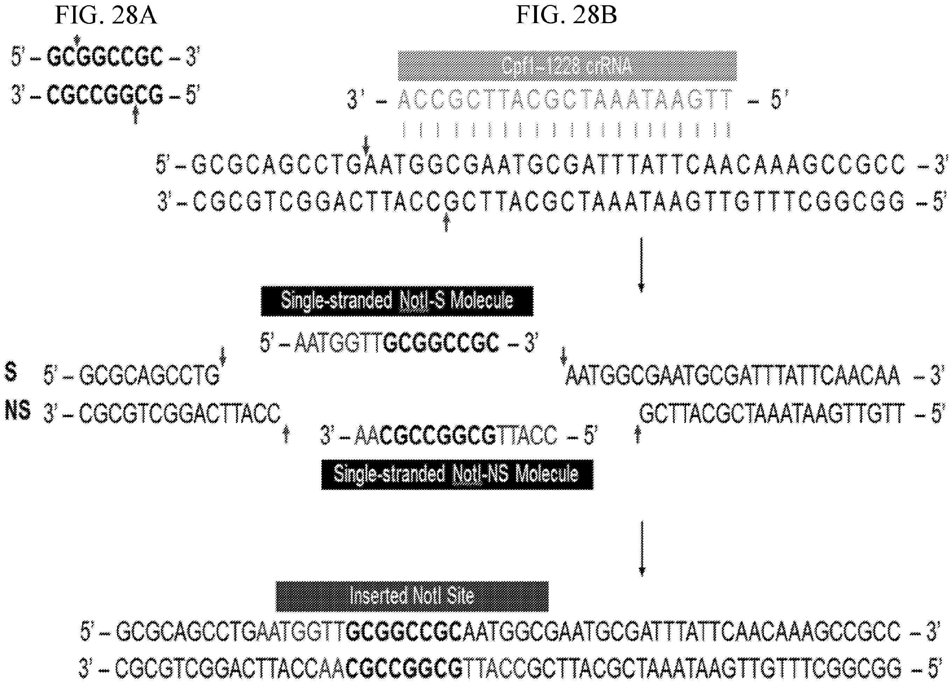

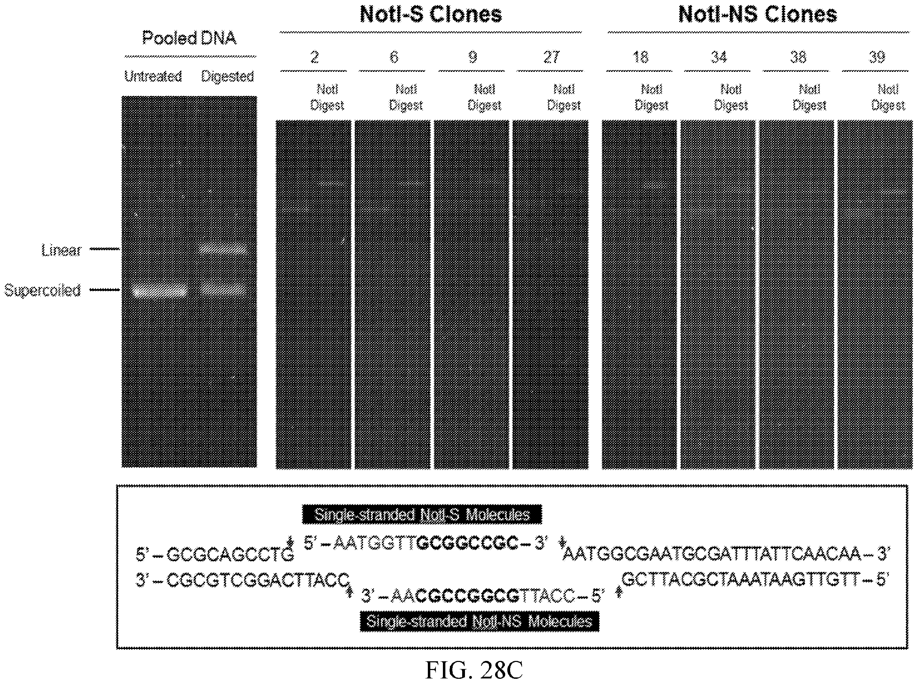

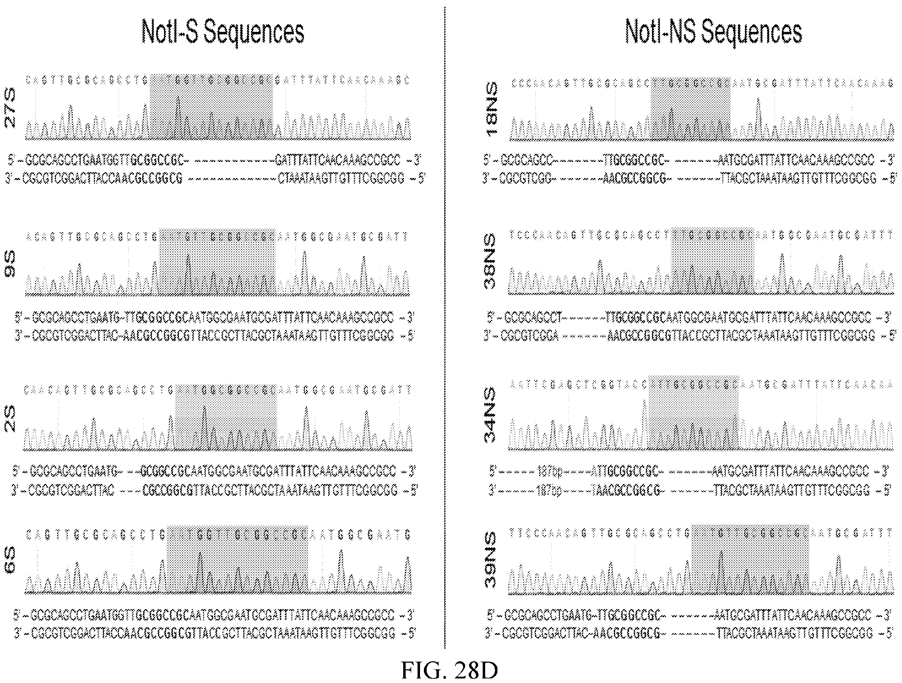

FIGs. 28A-28D are a series of images illustrating a proposed mechanism and verification of single-stranded NotI molecule insertion. FIG. 28A is an illustration of the NotI restriction cut site. FIG. 28B shows the Cpfl RNP staggered double-stranded cleavage site on the lacZ gene indicated by arrows. The single-stranded NotI molecules are inserted into one of two strands, the sense strand (S) or nonsense (NS) strand, by utilizing arms complementary to the overhangs produced by Cpfl cleavage. FIG. 28C shows pooled and isolated plasmid DNA from selected bacterial colonies transformed with plasmids recovered from *in vitro* single-stranded NotI reactions subject to NotI enzyme digestion to confirm the integration of the NotI site into the lacZ gene region. FIG. 28D shows four representative sequences from plasmid DNA isolated from selected bacterial colonies transformed with plasmid recovered from each of the *in vitro* single-stranded NotI-S and NotI-NS insertion reactions.
FIGs. 29A-29D are a series of images illustrating insertion of a 186 bp fragments using two Cpfl enzymes. FIG. 29A shows two Cpfl nucleases, cutting at different sites, were used to excise a fragment from the parent plasmid and replace it with a fragment of 186 base pairs, created by the annealing of two complementary oligonucleotides with complementary overhangs. FIGs. 29B-29C display the sequences obtained for the *in vitro* reaction with readout in bacteria. FIG. 29D illustrates the type of outcomes obtained, including mostly deletions and one successful insertion clone.

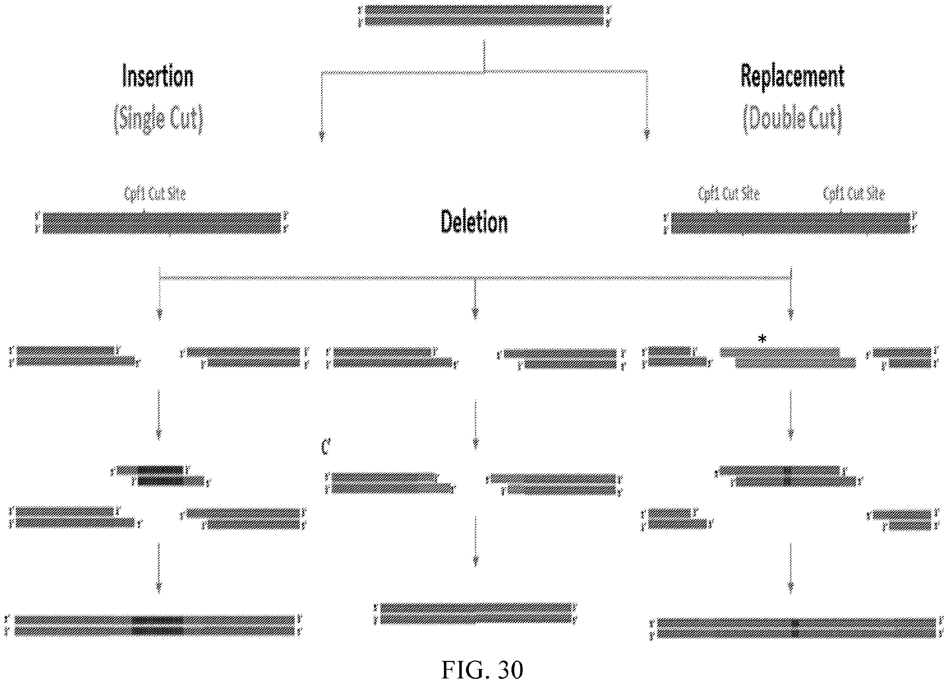

FIG. 30 illustrates insertion or replacement reactions and a deletion event. An insertion is generated in which a Cpfl RNP creates a staggered double-stranded DNA break and a double-stranded DNA fragment is then inserted at the break site, extending the original DNA segment. A deletion event occurs when a Cpfl RNP generates a staggered double-stranded DNA break and the exposed ends are resected before the DNA is re-ligated. A replacement reaction occurs when two Cpfl RNPs generate distinct double-stranded staggered cleavage sites along a gene segment after which the segment between the two cleavage sites is removed, shown by an ^{∗}, and a double-stranded DNA fragment is inserted and replaces the original DNA segment.

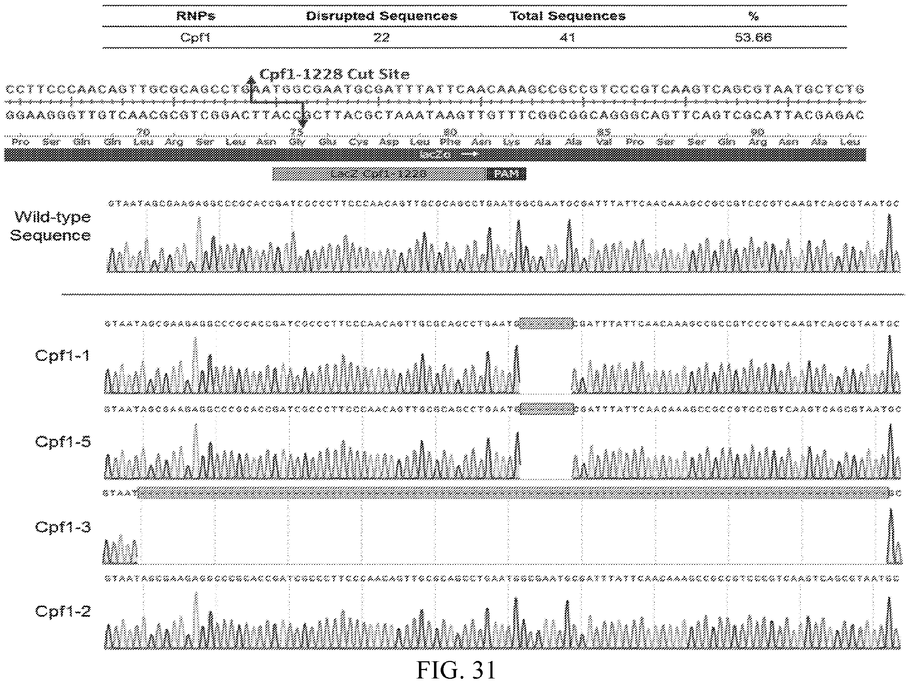

FIG. 31 illustrates Cpfl RNP activity *in vitro.* The frequency of DNA disruption by the Cpfl RNP is shown as a percentage of the number of disrupted DNA sequences detected in relation to the total number of sequences analyzed from bacterial colonies transformed with plasmid DNA recovered from *in vitro* reactions. The Cpfl site is shown along the lacZ gene region with associated cleavage sites marked by a staggered red arrow along the wild type lacZ gene region. The five sequences shown are representative of the total number of sequences assessed from *in vitro* reactions containing Cpfl RNPs displaying a variety of DNA disruption around the cleavage site.
FIGs. 32A-32B are a series of images showing additional sequences from plasmid DNA isolated from selected bacterial colonies transformed with plasmid recovered from the *in vitro* single-stranded NotI-S reaction.
FIGs. 33A-33C are a series of images showing base replacement in the lacZ gene.

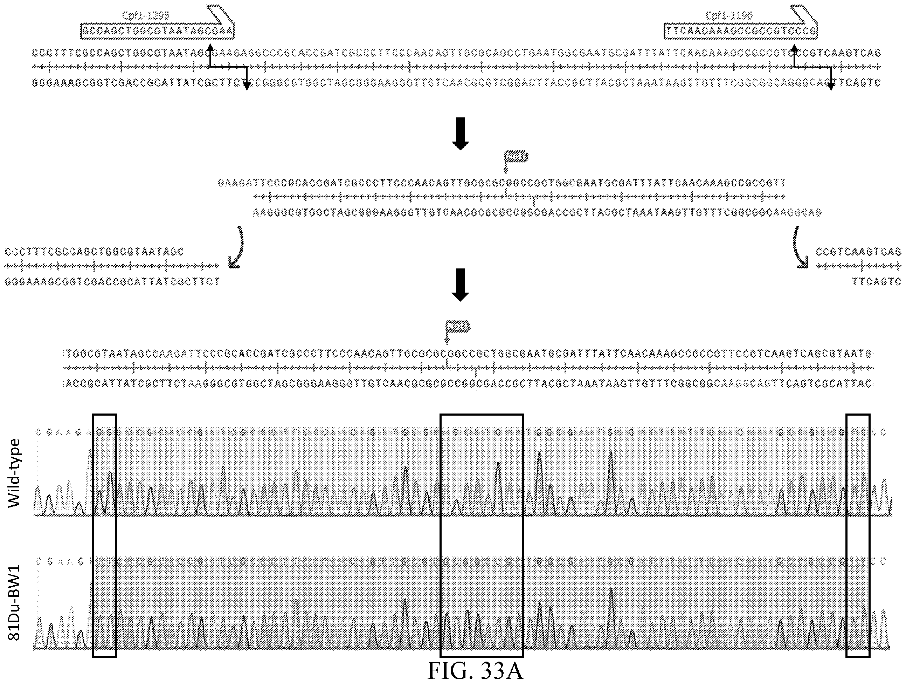

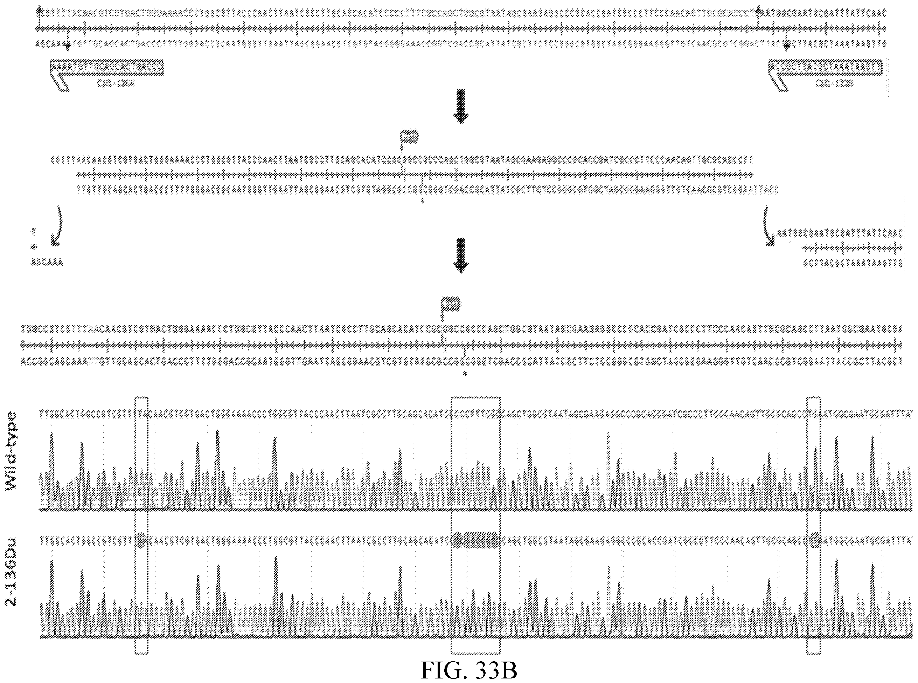

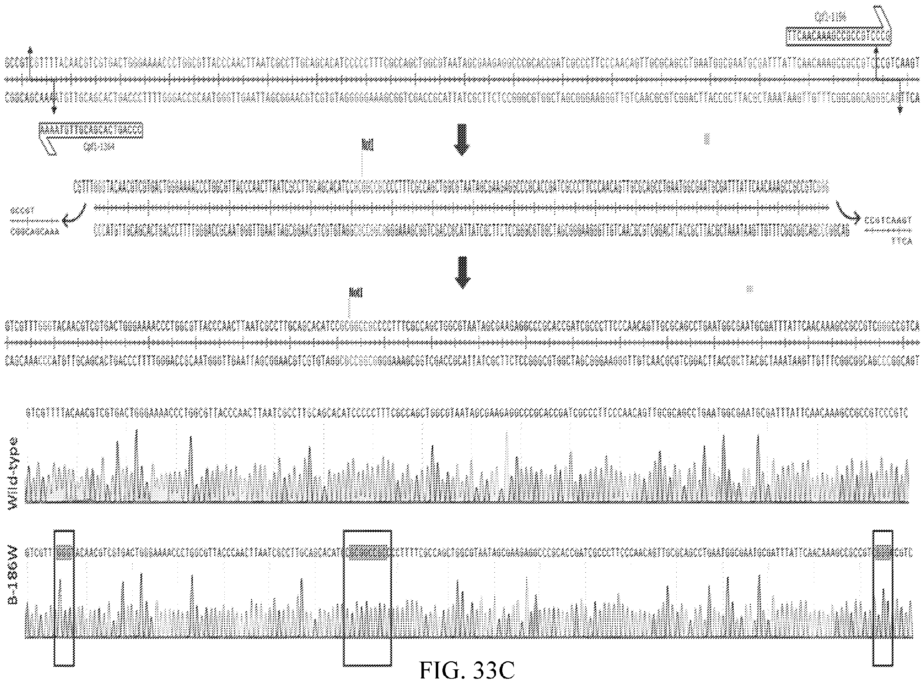

FIG. 33A shows an 81 base replacement. Two Cpfl RNP binding sites 81 base pairs apart are shown along the wild-type lacZ gene sequence with the resulting two staggered cut sites illustrated by two staggered arrows. The DNA segment between the two cut sites is then removed and a duplexed 81 base replacement fragment is integrated into the gene at the cut sites. The wild type lacZ gene sequence is shown above the sequence of a perfect 81 base replacement. The boxes indicate the inserted NotI restriction enzyme site and the barcode sequence (TT). FIG. 33B shows a 136 base replacement. Two Cpfl RNP binding sites 136 base pairs apart are shown along the wild-type lacZ gene sequence with the resulting two staggered cut sites illustrated by two staggered arrows. The DNA segment between the two cut sites is then removed and a duplexed 136 base replacement fragment is integrated into the gene at the cut sites. The wild type lacZ gene sequence is shown above the sequence of a perfect 136 base replacement. The boxes indicate the inserted NotI restriction enzyme site and the barcode sequence (AA/TT). FIG. 33C shows a 186 base replacement. Two Cpfl RNP binding sites 177 base pairs apart are shown along the wild-type lacZ gene sequence with the resulting two staggered cut sites illustrated by two staggered arrows. The DNA segment between the two cut sites is then removed and a duplexed 186 base replacement fragment is integrated into the gene at the cut sites. The wild type lacZ gene sequence is shown above the sequence of a perfect 186 base replacement. The boxes indicate the inserted NotI restriction enzyme site and the barcode sequence (GGG).

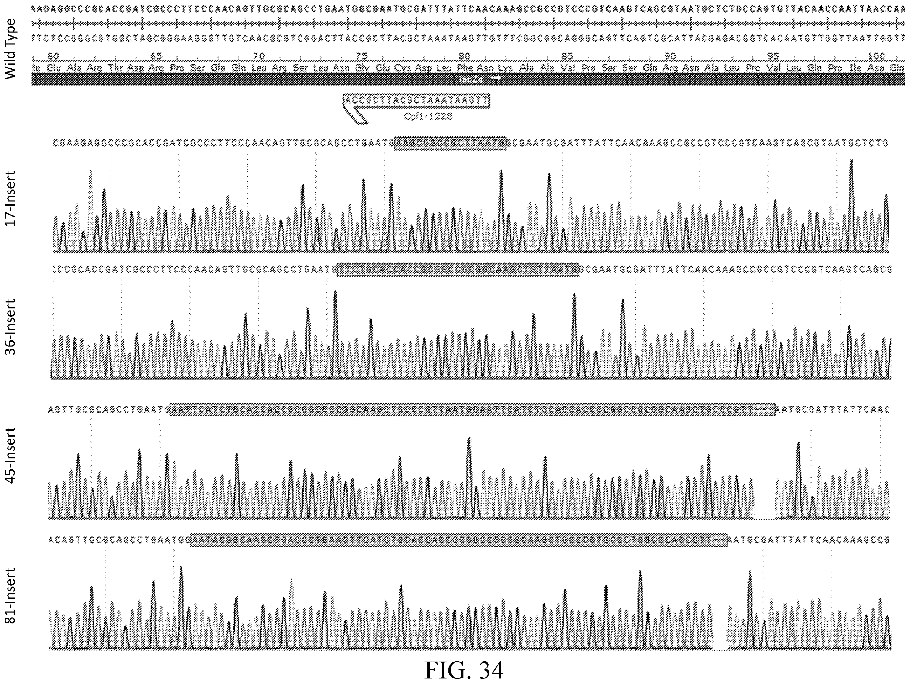

FIG. 34 shows additional sequences of fragment insertions of varied lengths in the lacZ gene. The Cpf1-1228 RNP cut site is illustrated along the sequence of the wild-type lacZ gene region. Sequences are shown from *in vitro* insertion reactions containing duplexed 17, 36, 45 and 81 base DNA fragments resulting in imperfect insertions. These sequences were from selected bacterial colonies that were transformed with isolated plasmid DNA.

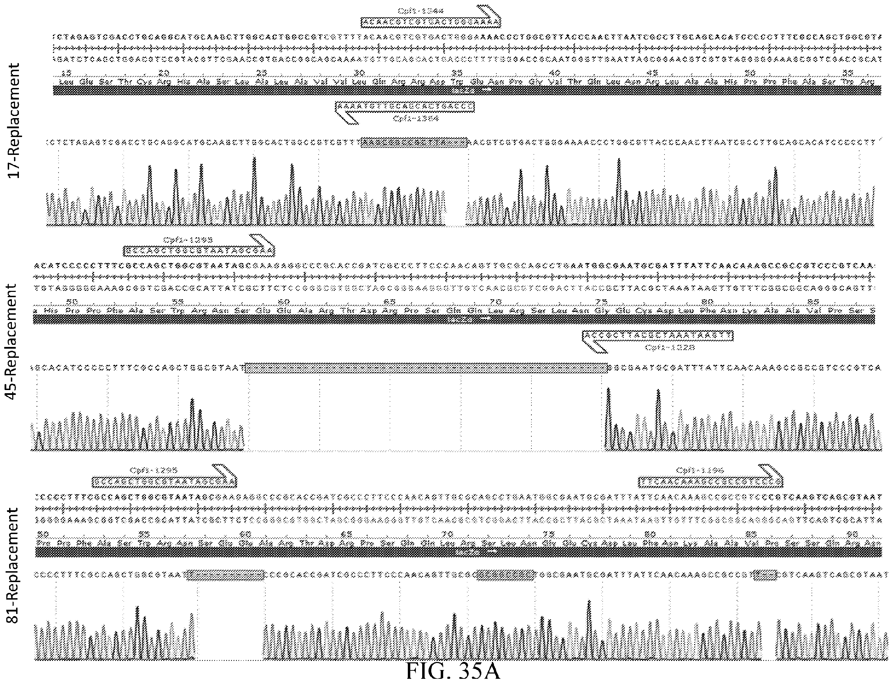

FIGs. 35A-35B illustrate additional sequences of segment replacements of varied lengths in the lacZ gene. Two Cpfl sites are shown along the sequence of the wild-type lacZ gene region for the 17, 45, 81, 136 and 186 base segment replacement reactions. These sequences were selected from bacterial colonies transformed with isolated plasmid DNA, demonstrating imperfect *in vitro* replacement reactions containing duplexed 17, 45, 81, 136 and 186 base DNA fragments.

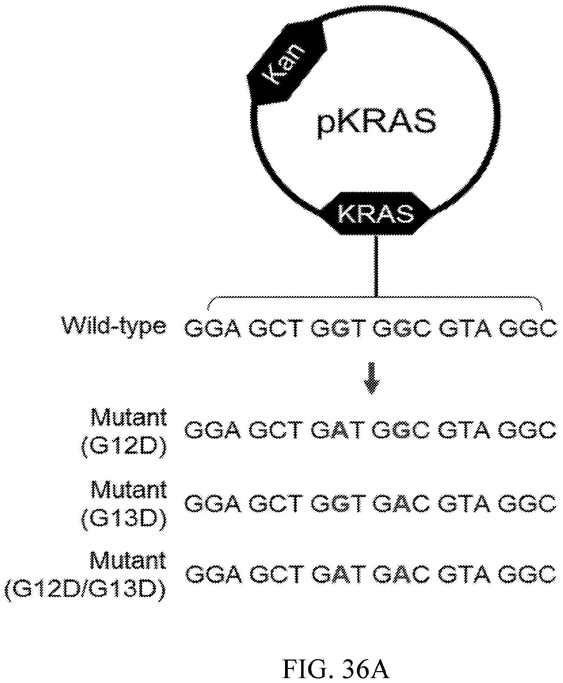

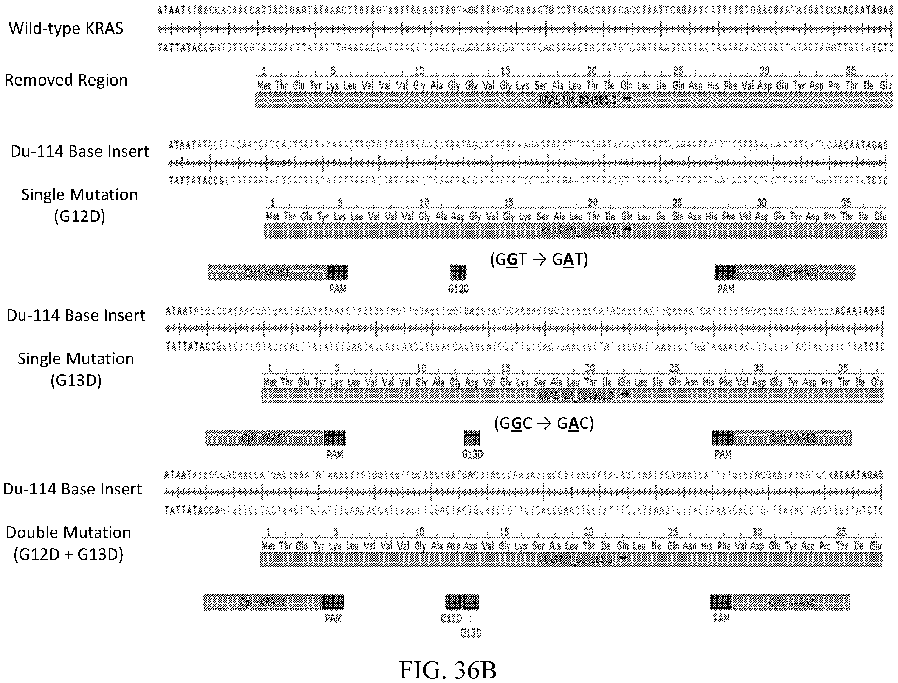

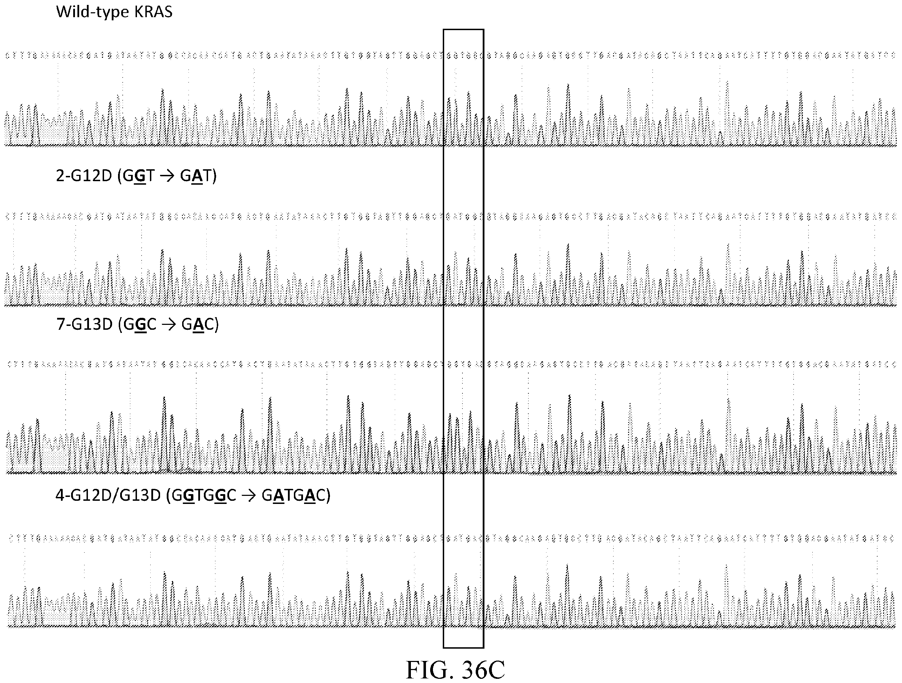

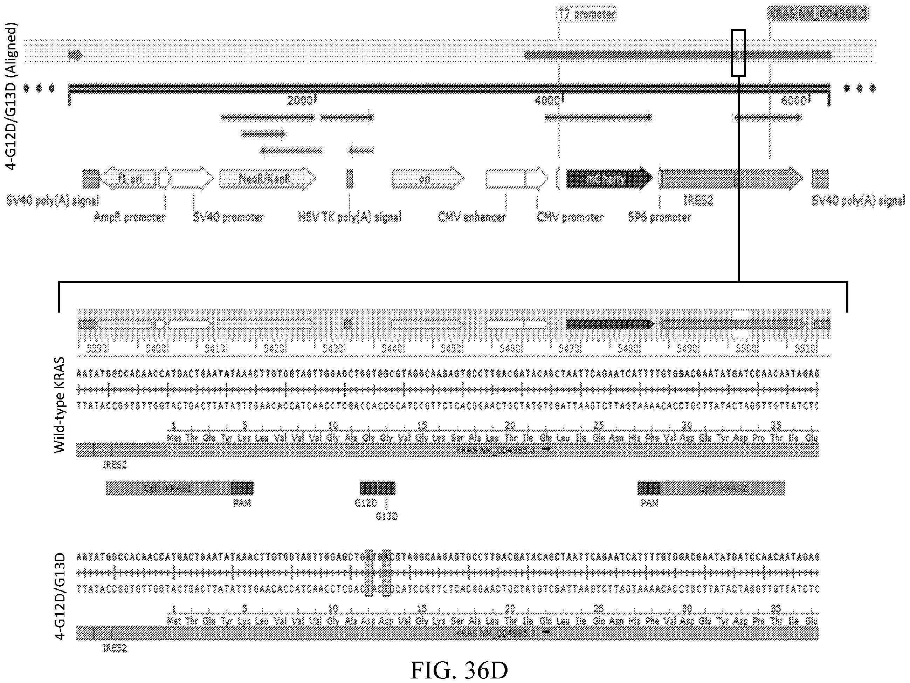

FIGs. 36A-36D are a series of images illustrating site-directed mutagenesis in the KRAS gene. FIG. 36A shows a representative plasmid map for pKRAS containing the Kanamycin resistance gene and KRAS gene of interest. Mutational variations for the G12D and G13D mutations are seen compared to the wild-type sequence of the KRAS gene. FIG. 36B shows two Cpfl RNPs 114 bases apart along the KRAS gene region spanning both the G12D and G13D mutation sites. Representations of the single mutations and double mutation variations incorporated into the duplexed 114 base replacement fragments are shown. FIG. 36C shows the sequence of the wild-type KRAS gene region and selected bacterial colonies transformed with plasmid DNA isolated from perfect *in vitro* replacement reaction containing all three mutation variations of the duplexed 114 base DNA fragment are shown with the specific mutations incorporated indicated within the red box. FIG. 36D shows confirmation that no off-target effects were produced through an aligned view of the plasmid containing the perfectly replaced G12D/G13D mutation fragment (solid grey line) to the wild-type KRAS plasmid (solid double black line), indicated the only mismatches seen are the intended two base pair changes resulting from the G12D and G13D mutations, shown within the box.

### DETAILED DESCRIPTION OF THE INVENTION

### Definitions

Unless defined otherwise, all technical and scientific terms used herein have the same meaning as commonly understood by one of ordinary skill in the art to which the invention pertains. Although any methods and materials similar or equivalent to those described herein may be used in the practice for testing of the present invention, exemplary materials and methods are described herein. In describing and claiming the present invention, the following terminology will be used.

It is also to be understood that the terminology used herein is for the purpose of describing particular embodiments only, and is not intended to be limiting.

The articles "a" and "an" are used herein to refer to one or to more than one *(i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

As used herein the term "amount" refers to the abundance or quantity of a constituent in a mixture.

As used herein, the term "bp" refers to base pair.

The term "complementary" refers to the degree of anti-parallel alignment between two nucleic acid strands. Complete complementarity requires that each nucleotide be across from its opposite. No complementarity requires that each nucleotide is not across from its opposite. The degree of complementarity determines the stability of the sequences to be together or anneal/hybridize. Furthermore various DNA repair functions as well as regulatory functions are based on base pair complementarity.

The term "CRISPR/Cas" or "clustered regularly interspaced short palindromic repeats" or "CRISPR" refers to DNA loci containing short repetitions of base sequences followed by short segments of spacer DNA from previous exposures to a virus or plasmid. Bacteria and archaea have evolved adaptive immune defenses termed CRISPR/CRISPR-associated (Cas) systems that use short RNA to direct degradation of foreign nucleic acids. In bacteria, the CRISPR system provides acquired immunity against invading foreign DNA via RNA-guided DNA cleavage.

"Cas endonuclease" refers to a CRISPR-associated endonuclease enzyme. A non-limiting example of a Cas endonuclease is Cas9. Other exemplary Cas endonucleases include but are not limited to Cpfl, Cas3, Cas8a, Cas8b, Cas10d, Cse1, Csy1, Csn2, Cas4, Cas10, Csm2, Cmr5, Fok1, T7, spCas9, Cpf2, CasY, CasX, and/or saCas9.

The "CRISPR/Cas9" system or "CRISPR/Cas9-mediated gene editing" refers to a type II CRISPR/Cas system that has been modified for genome editing/engineering. It is typically comprised of a "guide" RNA (gRNA) and a non-specific CRISPR-associated endonuclease (Cas9). "Guide RNA (gRNA)" is used interchangeably herein with "short guide RNA (sgRNA)". The gRNA is a short synthetic RNA composed of a "scaffold" sequence necessary for Cas9-binding and a user-defined ~20 nucleotide "spacer" or "targeting" sequence that defines the genomic target to be modified. The genomic target of Cas9 can be changed by changing the targeting sequence present in the gRNA.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence driven by its promoter.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an *in vitro* expression system. Expression vectors include all those known in the art, such as cosmids, plasmids (*e.g.,* naked or contained in liposomes) and viruses (*e.g.,* Sendai viruses, lentiviruses, retroviruses, adenoviruses, and adeno-associated viruses) that incorporate the recombinant polynucleotide.

"Homologous" as used herein, refers to the subunit sequence identity between two polymeric molecules, e.g., between two nucleic acid molecules, such as, two DNA molecules, two RNA molecules, a DNA and an RNA molecule, a DNA and a sgRNA molecule, or between two polypeptide molecules. When a subunit position in both of the two molecules is occupied by the same monomeric subunit; e.g., if a position in each of two DNA molecules is occupied by adenine, then they are homologous at that position. The homology between two sequences is a direct function of the number of matching or homologous positions; *e.g.,* if half (*e.g.,* five positions in a polymer ten subunits in length) of the positions in two sequences are homologous, the two sequences are 50% homologous; if 90% of the positions (e.g., 9 of 10), are matched or homologous, the two sequences are 90% homologous.

"Identity" as used herein refers to the subunit sequence identity between two polymeric molecules particularly between two amino acid molecules, such as, between two polypeptide molecules. When two amino acid sequences have the same residues at the same positions; e.g., if a position in each of two polypeptide molecules is occupied by an arginine, then they are identical at that position. The identity or extent to which two amino acid sequences have the same residues at the same positions in an alignment is often expressed as a percentage. The identity between two amino acid sequences is a direct function of the number of matching or identical positions; *e.g.,* if half (*e.g.,* five positions in a polymer ten amino acids in length) of the positions in two sequences are identical, the two sequences are 50% identical; if 90% of the positions (e.g., 9 of 10), are matched or identical, the two amino acids sequences are 90% identical.

As used herein, an "instructional material" includes a publication, a recording, a diagram, or any other medium of expression that can be used to communicate the usefulness of the compositions and methods of the invention. The instructional material of the kit of the invention may, for example, be affixed to a container which contains the nucleic acid, peptide, and/or composition of the invention or be shipped together with a container which contains the nucleic acid, peptide, and/or composition. Alternatively, the instructional material may be shipped separately from the container with the intention that the instructional material and the compound be used cooperatively by the recipient.

By the term "modified" as used herein, is meant a changed state or structure of a molecule or cell of the invention. Molecules may be modified in many ways, including chemically, structurally, and functionally. Cells may be modified through the introduction of nucleic acids.

A "mutation" as used therein is a change in a DNA sequence resulting in an alteration from a given reference sequence (which may be, for example, an earlier collected DNA sample from the same subject). The mutation can comprise deletion and/or insertion and/or duplication and/or substitution of at least one deoxyribonucleic acid base such as a purine (adenine and/or thymine) and/or a pyrimidine (guanine and/or cytosine). Mutations may or may not produce discernible changes in the observable characteristics (phenotype) of an organism (subject).

By "nucleic acid" is meant any nucleic acid, whether composed of deoxyribonucleosides or ribonucleosides, and whether composed of phosphodiester linkages or modified linkages such as phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages. The term nucleic acid also specifically includes nucleic acids composed of bases other than the five biologically occurring bases (adenine, guanine, thymine, cytosine and uracil).

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "oligonucleotide" typically refers to short polynucleotides, generally no greater than about 100 nucleotides. It will be understood that when a nucleotide sequence is represented by a DNA sequence (*i.e.,* A, T, G, C), this also includes an RNA sequence (*i.e.,* A, U, G, C) in which "U" replaces "T".

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "polynucleotide" includes DNA, cDNA, RNA, DNA/RNA hybrid, antisense RNA, siRNA, miRNA, snoRNA, genomic DNA, synthetic forms, and mixed polymers, both sense and antisense strands, and may be chemically or biochemically modified to contain non-natural or derivatized, synthetic, or semisynthetic nucleotide bases. Also, included within the scope of the invention are alterations of a wild type or synthetic gene, including but not limited to deletion, insertion, substitution of one or more nucleotides, or fusion to other polynucleotide sequences.

Conventional notation is used herein to describe polynucleotide sequences: the left-hand end of a single-stranded polynucleotide sequence is the 5'- end; the left-hand direction of a double-stranded polynucleotide sequence is referred to as the 5'-direction.

A "primer" is an oligonucleotide, usually of about 15, 20, 25, 30, 35, 40, 45 or 50 nucleotides in length, that is capable of hybridizing in a sequence specific fashion to the target sequence and being extended during the PCR.

The term "promoter" as used herein is defined as a DNA sequence recognized by the synthetic machinery of the cell, or introduced synthetic machinery, required to initiate the specific transcription of a polynucleotide sequence.

A "sample" or "biological sample" as used herein means a biological material from a subject, including but is not limited to organ, tissue, exosome, blood, plasma, saliva, urine and other body fluid. A sample can be any source of material obtained from a subject.

The term "subject" is intended to include living organisms in which an immune response can be elicited (e.g., mammals). A "subject" or "patient," as used therein, may be a human or non-human mammal. Non-human mammals include, for example, livestock and pets, such as ovine, bovine, porcine, canine, feline and murine mammals. Preferably, the subject is human.

A "targeted gene", "targeted sequence", or "target sequence" as used interchangeably herein refers to a nucleic acid sequence that is specifically targeted for mutagenesis. The nucleic acid sequence that is targeted can be in a coding (gene) or non-coding region of a genome.

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, Sendai viral vectors, adenoviral vectors, adeno-associated virus vectors, retroviral vectors, lentiviral vectors, and the like.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The invention relates to a novel method for performing *in vitro* site-directed mutagenesis using gene editing technologies. In certain embodiments, the invention includes an *in vitro* site-directed mutagenesis kit comprising a ribonucleotide particle (RNP), an oligonucleotide, a buffer, a cell extract having enzymatic activity for editing genes and instructional material for use thereof.

The invention includes a method of performing *in vitro* mutagenesis. In embodiments, the method comprises incubating a mixture comprising the RNP, a first plasmid, an oligonucleotide, a buffer, and a cell extract having enzymatic activity for editing genes, thus forming a second mutated plasmid. In yet other embodiments, the method comprises isolating the second plasmid. The method comprises administering the isolated second plasmid to a plurality of non-germ line cells. The method further comprises selecting from the plurality of cells at least one cell wherein *in vitro* mutagenesis of the target gene has occurred. The RNP may be assembled by annealing tracrRNA with crRNA then combining with Cas9. The plasmid contains a gene target. The gene target can be any gene in a cell. In one embodiment, the target gene is EGFP.

In other embodiments, the method comprises incubating a first mixture comprising an isolated ribonucleotide particle (RNP) and a plasmid, wherein the RNP comprises a crRNA and a Cpfl enzyme, wherein the crRNA is complementary to the targeted gene, and wherein the RNP generates a double stranded break in the plasmid. In yet other embodiments, the method comprises administering the re-circularized plasmid to a plurality of cells, and selecting from the plurality of cells at least one cell wherein *in vitro* mutagenesis has occurred in the targeted gene.

In certain embodiments, the invention includes a method of performing *in vitro* mutagenesis of a targeted sequence, comprising incubating a mixture comprising an isolated RNP, a plasmid, a single stranded oligonucleotide, a cell extract having enzymatic activity for editing genes. The RNP comprises a crRNA and a Cpfl enzyme. The crRNA is complementary to the targeted sequence, and the RNP generates a double stranded break in the plasmid. The single stranded oligonucleotide comprises 5' overhangs complementary to the Cpfl cut site. A re-circularized plasmid is generated and administered to a plurality of non-germ line cells. Selected from the plurality of cells is at least one cell wherein *in vitro* mutagenesis has occurred in the targeted sequence.

In certain embodiments, the double stranded oligonucleotide comprises a NotI restriction site.

The cells can be selected using any standard mean known to one of ordinary skill in the art. In one embodiment, the selecting can comprise digesting the cell with a NotI enzyme to confirm that *in vitro* mutagenesis has occurred. The cell-free extract can be derived from any cells or cell line known in the art, including but not limited to HEK, HUH-7, DLD1, HCT116, and *S. cerevisiae.*

Demonstrated herein is a new system in which an RNP particle contains crRNA and/or tracRNA as separate entities, similar to what is found in natural systems and bacteria. Coupled with the Cas9 or Cpfl protein, this system increases efficacy and precision reducing the concern of offsite mutagenesis. A series of capabilities for mutation synthesis are possible, ranging from single base point mutations, single base deletions or insertions, small insertions or deletions, and small duplications within the coding region of the target genes.

The fundamental steps for this new assay were established and validated for a wide-range of DNA sequence mutations. This focused primarily on increasing the efficacy and efficiency of creating point mutations in specific target genes. In addition, a more universal RNP-type particle is used that expands the versatility of the assay and enables precise mutagenesis at multiple sites simultaneously within the coding region of the gene.

### Structure and Composition of Oligonucleotides for Various Modifications in Target Genes

Single-stranded oligonucleotides are well studied and useful synthetic DNA molecules for gene editing because a large number of chemical modifications and variations can be incorporated into their composition. Some of these modifications enable a higher binding affinity to a duplex DNA target, ensuring that the critical reaction intermediate, the D-loop, is stable enough to direct genetic exchange. Several classes of chemical modifications can be used in this study so that the desired genetic alteration in the target gene will be created at a higher efficiency. In certain embodiments, single base nucleotide modifications, deletions, or insertions, are executed by an oligonucleotide of about 72 bases in length bearing chemically modified terminal linkages to prevent against nuclease digestion in the cell free extract. This workhorse oligonucleotide is designed so that it binds in perfect homologous register and complementarity with the target gene sequence except for a single mismatch which is created at the nucleotide in the target gene designated for change. The mismatched base pair is most efficiently corrected when it is created at the central base in the oligonucleotide during the alignment of complementary strands. For target gene alterations where double nucleotide substitutions or small insertions or deletions are desired, two types of chemical modifications in the targeting oligonucleotide are utilized. Both are designed to increase target affinity so that the section of the targeted gene can be deleted or small insertions can be placed within the gene sequence. Chemical modifications that increase binding affinity and stable DNA pairing between an incoming single stranded oligo donor (ssODN) and the duplex as it incorporates into the helix are synthesized alone or in combination into the targeting oligonucleotide.

The 2'-O-(methyl, fluoro, and so forth) group of modifications offers a significant increase in binding affinity with both RNA and DNA targets and have also been shown to increase resistance to nuclease digestion in both cell free conditions and after microinjection into cells. Typically, a series of 2'-O-methyl modifications (ranging from 3-5) are incorporated in the left and/or right arms of the workhorse vector (72-mer), as well as within the basis in the center of the molecule surrounding the target site. Another 2' modification that improves binding affinity for DNA is the addition of a fluoro- group to designated bases in the oligonucleotide. Once again, a series of fluoro-group modified bases are placed at 3' and 5' arms as well as in the central region of the targeting vector. Linked Nucleic Acid (LNA) has become a prominent modification for increasing binding affinity. LNA is a bicyclic nucleic acid that tethers the 2'-O to the 4'C to create a methylene bridge effectively locking the structure into a 3'- sugar conformation. Since the length of the oligonucleotide can be extended from about 72 to about 200 bases, where the desired end product is a 20 base insertion, lateral sections of the oligonucleotide can bear a series of these modifications to improve binding target stability. The same is true in the case where the objective is to delete 20 bases. In certain embodiments, the targeting oligonucleotide is 52 bases in length and bears lateral sections of chemical moieties that improve binding avidity. In certain embodiments, the oligonucleotide is between 25 and 200 bases in length, and any and all numbers therebetween. In this way genetic modifications beyond the single base substitution can be created with relatively high efficiency and identified through the dual targeting approach outlined herein.

The oligonucleotides may be engineered to be between about 10 nucleotides to about 200 nucleotides, or about 50 nucleotides to about 125 nucleotides, or about 60 nucleotides to about 100 nucleotides, or about 70 nucleotides to about 90 nucleotides in length. The oligonucleotide can be about 40, 45, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 160, 170, 180, 190, 200 nucleotides, or any number of nucleotides therebetween. In one embodiment, the oligonucleotide is greater than 60 nucleotides in length. In another embodiment, the oligonucleotide is greater than 70 nucleotides in length. In yet another embodiment, the oligonucleotide is about 72 nucleotides in length. In still another embodiment, the oligonucleotide is between about 25 and about 200 bases in length.

### Sources of cell free extract

There are a number of sources for the cell extracts that provide the enzymatic activity for editing genes located in expression vectors or episomal targets. While the tendency might be to isolate and purify nuclear extracts from mammalian cells solely, whole cell extracts containing cytoplasmic activities enable a higher level of gene editing, and thus are used in the experiments described herein. Mammalian cell free extracts can be obtained from any type of cell, including but not limited to HEK, HUH-7, DLD1,HCT116, and *S. cerevisiae* cells. The latter two originate from colon cancer cells while the former originates from liver. Each cell line has demonstrated a rich source for cell extracts that can catalyze gene editing activity. These cell lines are known to be deficient in one or more mismatch repair protein activities which, while somewhat counterintuitive, actually enables higher levels of gene editing. When a mismatch is created by an incoming oligonucleotide with the target gene, to enable nucleotide exchange, insertion or deletion, the natural tendency of a wild type mismatch repair pathway is to recognize and destabilize that pairing. Extensive genetic and biochemical studies were carried out to show that nucleotide exchange driven by ssODNs at the target site is enhanced in such mutant cell backgrounds (Dekker et al. Nucleic Acids Res. 31(6) e27). Preparation and utilization of cell free extracts from yeast, primarily *S. cerevisiae,* to enable genetic modification of expression vectors provides an innovative approach to modifying episomal targets. The variety of genetic backgrounds in the remarkably genetically tractable *S. cerevisiae,* provides a rich source of enzymatic activity that could be more efficient in executing single base repairs, small segment deletions, small segment insertions, or gene fragment duplications. Thus, depending on the objective, the appropriate strain can be utilized as a source for the cell free extract to achieve success in the most validated and expeditious fashion.

### Supplementation of the cell extract having enzymatic activity for editing enzymes

In certain embodiments, chemical modification of the oligonucleotide provides the most efficient strategy to produce a wide range of appropriate genetic modifications. However, should a particular series of gene alterations become problematic, cell extracts supplemented with additional genetic tools can be used. A wide variety of CRISPR/Cas9 expression constructs that are highly expressed in mammalian cell lines are available. Non-limiting examples of these constructs include plasmid p42230, which contains a human codon optimized SpCas9 and an insertion site for chimeric guide RNA, and other constructs with a pX330 backbone vector (Addgene). A particular CRISPR/Cas9 construct can be transfected into mammalian cells and expressed prior to preparing a cell free extract, thereby enriching the enzymatic activity of gene editing. Without wishing to be bound by specific theory, supplementation of the cell free extract may help direct the oligonucleotide to a specific site and indirectly enhance the frequency of editing of the target gene. In certain embodiments, if simple deletion or insertion of a DNA segment of the target gene is the experimental objective, it may be preferable to utilize the cell free extract supplemented with a CRISPR/Cas9 function initially to enable the generation of that genetic alteration. An innovative series of experiments have been performed in which a long single-stranded DNA molecule was inserted into a mammalian gene, in frame, to tag the disabled gene (Miura et al. Sci Rep. 2015, Aug 5; 5:12799). Such an approach can also be used *in vitro* using cell free extracts.

### Kits

In certain aspects, the invention provides *in vitro* mutagenesis kits for a targeted sequence. In one embodiment, the kit comprises an isolated ribonucleotide particle (RNP), a double stranded oligonucleotide, a plasmid, a cell extract having enzymatic activity for editing genes, and preferably further comprising instructional material for use thereof. The RNP comprises a crRNA and a Cpfl enzyme, wherein the crRNA is complementary to the targeted sequence, and the double stranded oligonucleotide comprises 5' overhangs complementary to the Cpfl cut site. In certain embodiments, the double stranded oligonucleotide comprises a NotI restriction site.

The crRNA is complementary to the targeted gene and the single stranded oligonucleotide comprises a NotI restriction site and 5' overhangs complementary to the Cpfl cut site.

The kits can further comprise a second RNP comprising a second crRNA complementary to a second targeted sequence.

### CRISPR/Cas

The CRISPR/Cas system is a facile and efficient system for inducing targeted genetic alterations. Target recognition by the Cas9 protein requires a 'seed' sequence within the guide RNA (gRNA) and a conserved tri-nucleotide containing protospacer adjacent motif (PAM) sequence upstream of the gRNA-binding region. The CRISPR/CAS system can thereby be engineered to cleave virtually any DNA sequence by redesigning the gRNA for use in cell lines (such as 293T cells), primary cells, and CAR T cells. The CRISPR/CAS system can simultaneously target multiple genomic loci by co-expressing a single Cas9 protein with two or more gRNAs, making this system uniquely suited for multiple gene editing or synergistic activation of target genes.

One example of a CRISPR/Cas system used to inhibit gene expression, CRISPRi, is described in U.S. Publication No.: 2014/0068797. CRISPRi induces permanent gene disruption that utilizes the RNA-guided Cas9 endonuclease to introduce DNA double stranded breaks which trigger error-prone repair pathways to result in frame shift mutations. A catalytically dead Cas9 lacks endonuclease activity. When coexpressed with a guide RNA, a DNA recognition complex is generated that specifically interferes with transcriptional elongation, RNA polymerase binding, or transcription factor binding. This CRISPRi system efficiently represses expression of targeted genes.

CRISPR/Cas gene disruption occurs when a guide nucleic acid sequence specific for a target gene and a Cas endonuclease are introduced into a cell and form a complex that enables the Cas endonuclease to introduce a double strand break at the target gene. The CRISPR system may comprise an expression vector, such as, but not limited to, an pAd5F35-CRISPR vector. The Cas expression vector may induce expression of Cas9 endonuclease. Other endonucleases may also be used, including but not limited to, T7, Cas3, Cas8a, Cas8b, Cas10d, Cse1, Csy1, Csn2, Cas4, Cas10, Csm2, Cmr5, Fok1, other nucleases known in the art, and any combination thereof. In certain embodiments, Cas9 further includes spCas9, Cpfl, Cpf2, CasY, CasX, and/or saCas9.

Inducing the Cas expression vector may comprise exposing the cell to an agent that activates an inducible promoter in the Cas expression vector. Then, the Cas expression vector includes an inducible promoter, such as one that is inducible by exposure to an antibiotic *(e.g.,* by tetracycline or a derivative of tetracycline, for example doxycycline). However, it should be appreciated that other inducible promoters can be used. The inducing agent can be a selective condition (e.g., exposure to an agent, for example an antibiotic) that results in induction of the inducible promoter. This results in expression of the Cas expression vector.

The guide nucleic acid sequence is specific for a gene and targets that gene for Cas endonuclease-induced double strand breaks. The sequence of the guide nucleic acid sequence may be within a loci of the gene. In certain embodiments, the guide nucleic acid sequence is at least 10, 11, 12, 13, 14, 15, 16, 17, 18, 19, 20, 21, 22, 23, 24, 25, 26, 27, 30, 31, 32, 33, 34, 35, 36, 37, 38, 39, 40 or more nucleotides in length.

The guide nucleic acid sequence may be specific for any gene, such as a gene that would reduce immunogenicity or reduce sensitivity to an immunosuppressive microenvironment. The guide nucleic acid sequence includes a RNA sequence, a DNA sequence, a combination thereof (a RNA-DNA combination sequence), or a sequence with synthetic nucleotides. The guide nucleic acid sequence can be a single molecule or a double molecule. The guide nucleic acid sequence may comprise a single guide RNA.

In the context of formation of a CRISPR complex, "target sequence" refers to a sequence to which a guide sequence is designed to have some complementarity, where hybridization between a target sequence and a guide sequence promotes the formation of a CRISPR complex. Full complementarity is not necessarily required, provided there is sufficient complementarity to cause hybridization and promote formation of a CRISPR complex. A target sequence may comprise any polynucleotide, such as DNA or RNA polynucleotides.Typically, in the context of a CRISPR system, formation of a CRISPR complex (comprising a guide sequence hybridized to a target sequence and complexed with one or more Cas proteins) results in cleavage of one or both strands in or near (e.g., within about 1, 2, 3, 4, 5, 6, 7, 8, 9, 10, 20, 50 or more base pairs) the target sequence. As with the target sequence, it is believed that complete complementarity is not needed, provided this is sufficient to be functional. The tracr sequence may have at least 50%, 60%, 70%, 80%, 90%, 95% or 99% of sequence complementarity along the length of the tracr mate sequence when optimally aligned. One or more vectors driving expression of one or more elements of a CRISPR system may be introduced into a host cell, such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. A single promoter may drive expression of a transcript encoding a CRISPR enzyme and one or more of the guide sequence, tracr mate sequence (optionally operably linked to the guide sequence), and a tracr sequence embedded within one or more intron sequences (e.g., each in a different intron, two or more in at least one intron, or all in a single intron).

The CRISPR enzyme may be part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Additional domains that may form part of a fusion protein comprising a CRISPR enzyme are described in US20110059502.

A tagged CRISPR enzyme may be used to identify the location of a target sequence.

Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (*e.g.* a transcript of a vector described herein), naked nucleic acid, and nucleic acid complexed with a delivery vehicle, such as a liposome. Another delivery mode for the CRISPR/Cas9 comprises a combination of RNA and purified Cas9 protein in the form of a Cas9-guide RNA ribonucleoprotein (RNP) complex (Lin et al., 2014, ELife 3:e04766). Viral vector delivery systems include DNA and RNA viruses, which have either episomal or integrated genomes after delivery to the cell (Anderson, 1992, Science 256:808-813; and Yu et al., 1994, Gene Therapy 1:13-26).

The CRISPR/Cas may be derived from a type II CRISPR/Cas system. In other embodiments, the CRISPR/Cas sytem is derived from a Cas9 protein. The Cas9 protein can be from *Streptococcus pyogenes, Streptococcus thermophilus,* or other species.

In general, CRISPR/Cas proteins comprise at least one RNA recognition and/or RNA binding domain. RNA recognition and/or RNA binding domains interact with the guiding RNA. CRISPR/Cas proteins can also comprise nuclease domains *(i.e.,* DNase or RNase domains), DNA binding domains, helicase domains, RNAse domains, protein-protein interaction domains, dimerization domains, as well as other domains. The CRISPR/Cas proteins can be modified to increase nucleic acid binding affinity and/or specificity, alter an enzymatic activity, and/or change another property of the protein. The CRISPR/Cas-like protein of the fusion protein can be derived from a wild type Cas9 protein or fragment thereof. The CRISPR/Cas can be derived from modified Cas9 protein. For example, the amino acid sequence of the Cas9 protein can be modified to alter one or more properties (e.g., nuclease activity, affinity, stability, and so forth) of the protein. Alternatively, domains of the Cas9 protein not involved in RNA-guided cleavage can be eliminated from the protein such that the modified Cas9 protein is smaller than the wild type Cas9 protein. In general, a Cas9 protein comprises at least two nuclease *(i.e.,* DNase) domains. For example, a Cas9 protein can comprise a RuvC-like nuclease domain and a HNH-like nuclease domain. The RuvC and HNH domains work together to cut single strands to make a double-stranded break in DNA (Jinek et al., 2012, Science, 337:816-821). In certain embodiments, the Cas9-derived protein can be modified to contain only one functional nuclease domain (either a RuvC-like or a HNH-like nuclease domain). For example, the Cas9-derived protein can be modified such that one of the nuclease domains is deleted or mutated such that it is no longer functional *(i.e.,* the nuclease activity is absent). In some embodiments in which one of the nuclease domains is inactive, the Cas9-derived protein is able to introduce a nick into a double-stranded nucleic acid (such protein is termed a "nickase"), but not cleave the double-stranded DNA. In any of the above-described embodiments, any or all of the nuclease domains can be inactivated by one or more deletion mutations, insertion mutations, and/or substitution mutations using well-known methods, such as site-directed mutagenesis, PCR-mediated mutagenesis, and total gene synthesis, as well as other methods known in the art.

A vector may drive the expression of the CRISPR system. The art is replete with suitable vectors that are useful in the present invention. The vectors to be used are suitable for replication and, optionally, integration in eukaryotic cells. Typical vectors contain transcription and translation terminators, initiation sequences, and promoters useful for regulation of the expression of the desired nucleic acid sequence. The vectors of the present invention may also be used for nucleic acid standard gene delivery protocols. Methods for gene delivery are known in the art, such as in U.S. Patent Nos. 5,399,346, 5,580,859 & 5,589,466. .

Further, the vector may be provided to a cell in the form of a viral vector. Viral vector technology is well known in the art and is described, for example, in Sambrook et al. (4th Edition, Molecular Cloning: A Laboratory Manual, Cold Spring Harbor Laboratory, New York, 2012), and in other virology and molecular biology manuals. Viruses, which are useful as vectors include, but are not limited to, retroviruses, adenoviruses, adeno-associated viruses, herpes viruses, Sindbis virus, gammaretrovirus and lentiviruses. In general, a suitable vector contains an origin of replication functional in at least one organism, a promoter sequence, convenient restriction endonuclease sites, and one or more selectable markers (e.g., WO 01/96584; WO 01/29058; and U.S. Patent No. 6,326,193).

### EXPERIMENTAL EXAMPLES

The materials and methods employed in these experiments are now described.

*Preparation of Cell-Free Extract:* Cell-free extract was prepared from 200 million HCT116-19 cells following the outlined technique in Cole-Strauss et al., 1999. Nucl. Acids Res. 27 (5), 1323-1330. The resulting extract was aliquoted immediately and stored at -80°C. HEK293 cells (ATCC, American Type Cell Culture) were cultured and 8 × 10⁶ cells were harvested and immediately washed in cold hypotonic buffer (20mM HEPES, 5mM KCl, 1.5mM MgCl2 1mM DTT, and 250mM sucrose). Cells were centrifuged, washed and resuspended in cold hypotonic buffer without sucrose, followed by incubation on ice for 15 minutes before being lysed by 25 strokes of a Dounce homogenizer. Cytoplasmic fraction of enriched cell lysate was incubated on ice for 60 minutes and centrifuged for 15 minutes at 12,000 g, 4°C. The supernatant was then aliquoted out and immediately frozen at -80°C. Cell-free extract concentrations were determined using a Bradford assay.

*RNP Construction:* Cas9 and tracrRNA:crRNA components were assembled as an RNP complex to accommodate eight reactions at 10 pmoles per reaction.

*In vitro Reactions:* In certain embodiments, reactions were prepared with components shown in Table 1 and incubated at 37°C for 120 minutes. In certain embodiments, *in vitro* DNA cleavage reaction mixtures contained 250ng of pHSG299 (Takara Bio Company, Shiga, Japan) or pKRAS6163 plasmid DNA and 10pmols of RNP mixed in a reaction buffer (100mM NaCl, 20mM Tris-HCl, 10mM MgCl2, 100ug/ml BSA) at a final volume of 20ul. Each reaction was incubated for 15 minutes at 37°C after which DNA was isolated from reaction mixtures and purified using silica spin columns. Secondary *in vitro* re-circularization reactions contained DNA isolated from the initial cleavage reaction and 175ug of cell-free extract supplemented with ligase mixed in a reaction buffer (20mM TRIS, 15mM MgCl2, 0.4mM DTT, and 1.0mM ATP) brought to a final volume of 35ul. Each reaction was incubated for 15 minutes at 37°C. For reactions including duplexed insertion or replacement fragments, 100pmol were added into the final reaction mixture. DNA from the final reaction mixture was then purified using silica spin columns (Qiagen, Hilden, Germany).

**Table 1: In vitro Reactions**

| | RNP (10µl) | 10x Buffer (5 µl) | peGFP (5µg) | 72NT (0.5µg) | Extract | H₂O (to 50 µl) |
|---|---|---|---|---|---|---|
| 1 | - | + | + | - | - | + |
| 2 | + | + | + | - | - | + |
| +3 | - | + | + | + | 10µg | + |
| 4 | - | + | + | + | 20µg | + |
| 5 | - | + | + | + | 30µg | + |
| 6 | + | + | + | + | 10µg | + |
| 7 | + | + | + | + | 20µg | + |
| 8 | + | + | + | + | 30µg | + |

*Isolation of Plasmid DNA:* After incubation, each reaction mixture was diluted with PB buffer at a 1:5 ratio. Plasmid DNA was isolated using silica membrane spin columns following Qiagen's QIAprep Miniprep protocol and eluted in 10µL of water. Miniprep plasmid yields ranged from 620 ng - 1.8 µg, with control mixtures yielding higher returns on plasmid than full-component mixtures.

*Transformation of Isolated Plasmids into Competent Cells:* Eight transformations were performed using all 10 µL of the varied full plasmid DNA concentrations isolated from each of the eight reaction mixtures. Transformations were performed following the heat shock technique protocol outlined by the Invitrogen OneShot TOP 10 Chemically Competent *E. coli* protocol.

*Plating of Transformed Cells and Kanamycin Selection:* Transformed cells were plated onto kanamycin plates after 1:5 and 1:100 dilutions and incubated overnight to allow kanamycin-resistant colony growth. After incubation, 30 colonies were selected over 8 of the 1:100 diluted plates, favoring the last three full-component reactions, sealed and sent out for sequencing.

*Transformation, selection, DNA isolation and analysis:* Plasmid DNA recovered from *in vitro* reactions was transformed into DH5α (Invitrogen, Carlsbad, CA) or TOP10 One Shot (Thermo Fisher Scientific Wilmington, DE) competent *E. coli* via the heat shock methodology. Competent cells were incubated on ice for 30 minutes, heat shocked for 20 seconds at 42°C, placed on ice for 2 minutes and incubated at 37°C in 1ml of SOC media for 1 hour at 225 rpm. Undiluted competent cells were plated on media containing kanamycin and incubated overnight at 37°C. Single kanamycin resistant colonies were selected, and plasmid DNA was isolated via QIAprep Spin Miniprep Kit (Qiagen, Hilden, Germany). Modifications to the plasmid DNA selected from bacterial colonies was evaluated after DNA sequencing (GeneWiz, South Plainfield, NJ). Sequence analysis and disruption patterns were evaluated using SnapGene software for alignment of sample sequences to a wild-type sequence of the relevant plasmids.

The results of the experiments are now described.

### Example 1: In vitro activity of the CRISPR/Cas9 assembled into a ribonucleoprotein (RNP) complex on purified DNA templates

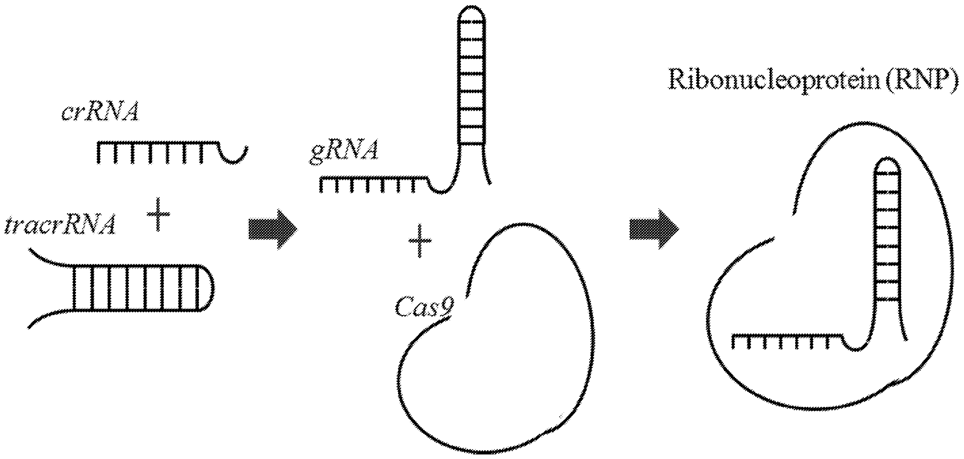

For RNP assembly, tracrRNA and (cr)isprRNA were annealed separately followed by addition of the purified Cas9 protein (FIG. 1). RNP assembly conditions and Cas9 were provided by IDT (Integrated DNA Technologies (IDT), Coralville, IA).

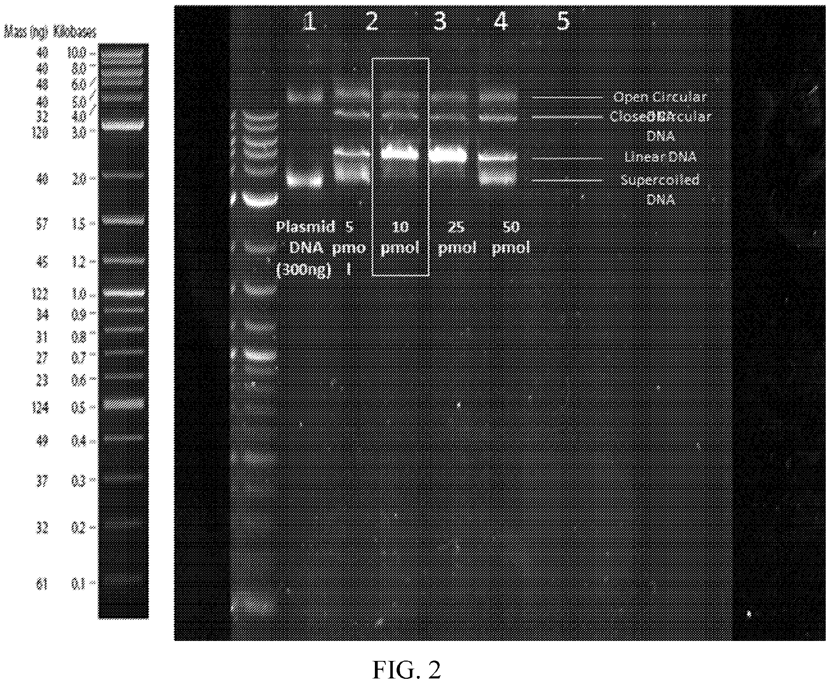

DNA cleavage activity of the purified RNP complex is shown in FIG. 2. The reaction is based on the use of a superhelical DNA plasmid molecule containing the eGFP gene, which contains the target sequence designated for cleavage by the RNP. Following the MW marker lane, lane 1 displays purified plasmid DNA in superhelical form. Lanes 2-5 show the products of reaction mixtures containing increasing amounts of the RNP complex. Even at the lowest level, double-stranded DNA cleavage was observed. At the excessive 50 pmol level, DNA cleavage activity is actually blocked due, in all likelihood, to the massive amount of Cas9 protein in the reaction. This titration provided confirmation that the RNP used in the *in vitro* mutagenesis kit was cutting the DNA efficiently.

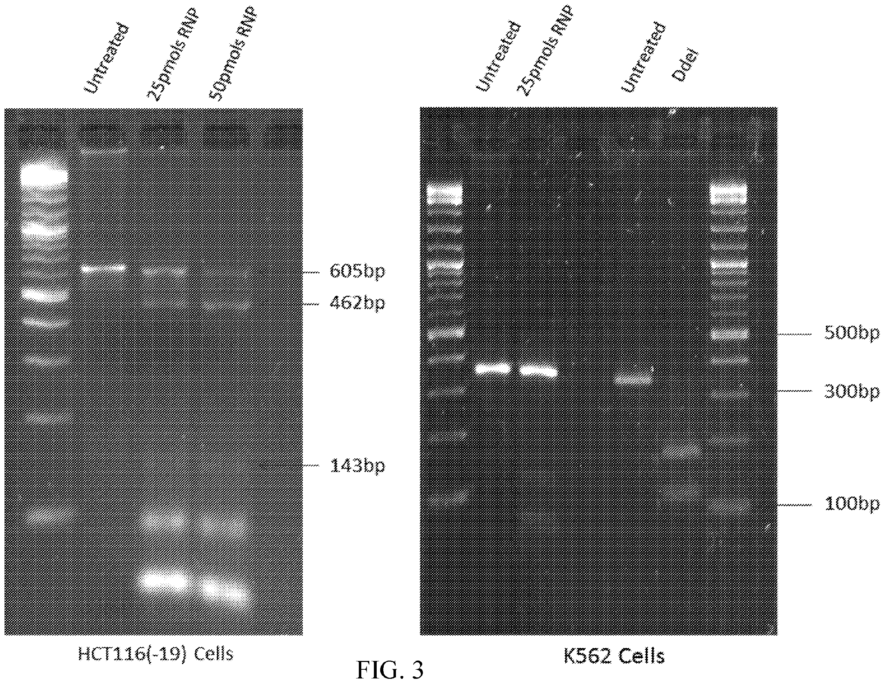

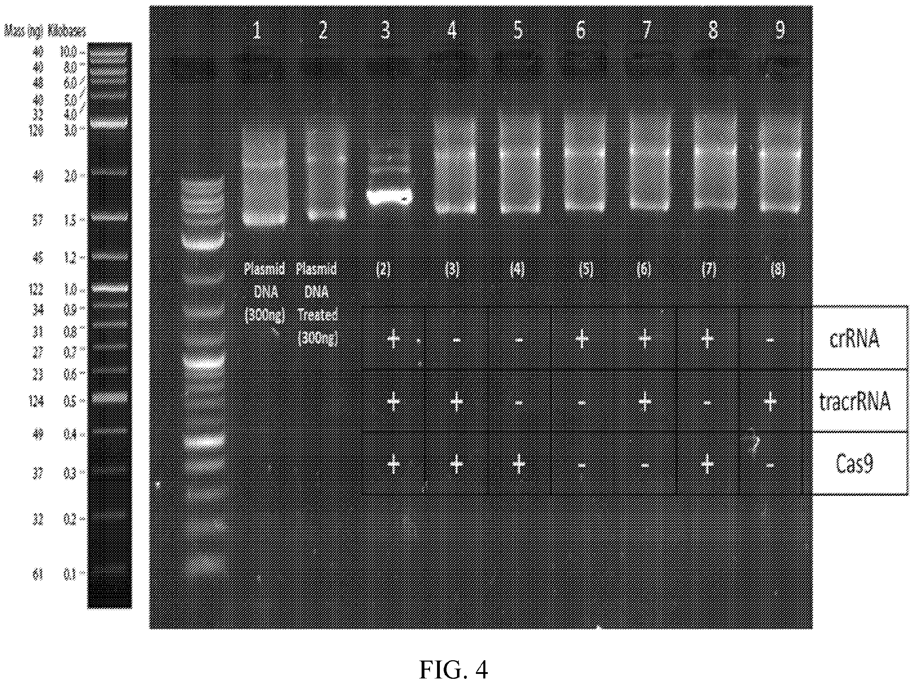

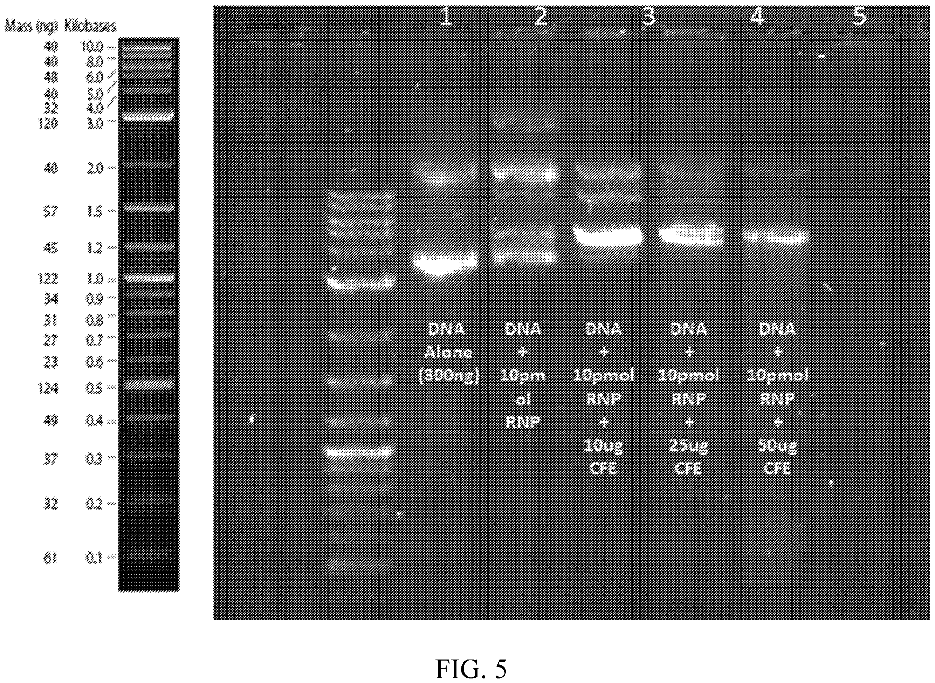

An experiment was designed to show the specificity of RNP-directed cleavage (FIG. 3). Genomic DNA was isolated from untreated HCT116-19 cells and PCR used to generate a 605 bp amplicon, which surrounds the sequence of the integrated mutant eGFP gene. The amplicon was combined with 25 pmols and 50 pmols of RNP complex respectively and incubated for 40 minutes at 37° C. In the complete reaction, two products were generated with sizes consistent with fragments predicted from the specific cut site designed for the RNP complex. As a control, the RNP complex was incubated with an amplicon generated from the HBB gene 345 base pairs in length from cell line K562. A control digest was performed on the 345 base amplicon with the restriction enzyme DdeI. The 345 base pair fragment was resistant to digestion by the RNP which was designed to cleave the eGFP target (and did so successfully elsewhere herein). Digestion with the restriction enzyme, Dde1, served as a positive control to ensure that the PCR product was capable of being cleaved. Enzymatic reactions were performed demonstrating enzyme activity in which individual components of the RNP complex were left out (FIG. 4). DNA cleavage activity was most efficacious when all components of the RNP were present (FIG. 4, lane 3). The activity of mammalian cell free extract on the product of the RNP cleavage was tested. Importantly, in a reaction which included 10 µg of cell free extract, some re-circularization of the plasmid was seen (FIG. 5, lane 3). In this particular case the cell free extract catalyzed the partial re-circularization of the linearized plasmid. Without wishing to be bound by specific theory, the linearized plasmid in all likelihood is the template for nucleotide exchange. This experiment demonstrated that superhelical DNA can be successfully cleaved by the RNP particle and partially processed back into circular form by the cell free extract. Importantly, the DNA template processed by the RNP was stable in the cell-free extract at functional dosages.

### Example 2: In vitro mutagenesis

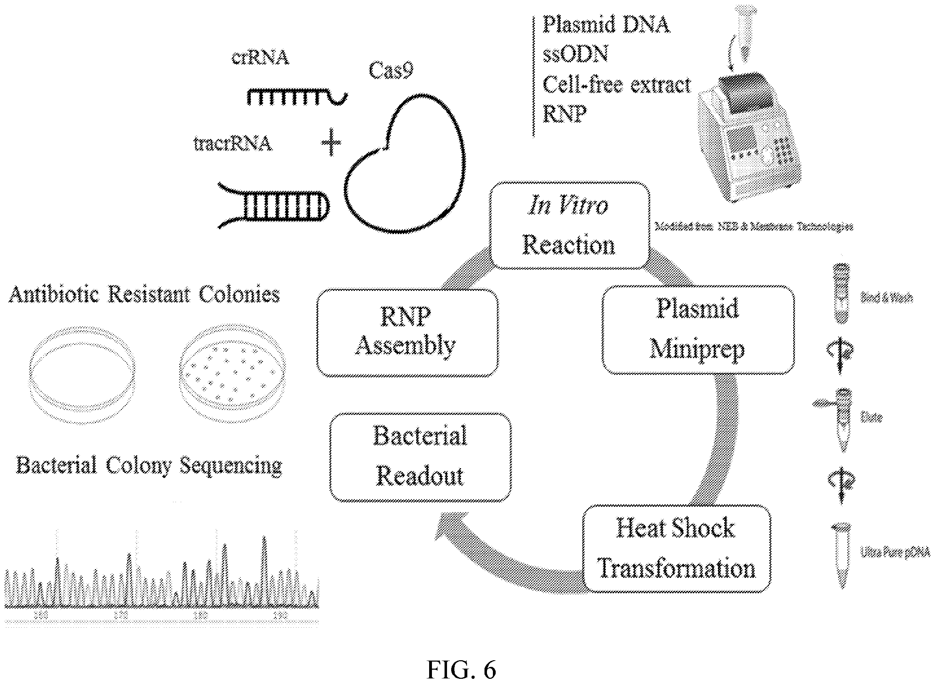

The initialization and validation strategy of the genetic readout system for the present invention is depicted in FIG. 6. The entire reaction is displayed starting with the assembly of the RNP particle followed by the addition of the single-stranded oligonucleotide, the cell free extract and the superhelical plasmid DNA template which bears the gene target - in this non-limiting example, the eGFP gene. The reaction takes place for 40 to 60 minutes after which the targeted plasmid is isolated using a standard DNA mini-prep protocol. The plasmid population is then transformed into *Escherichia coli* via the heat shock protocol and the cells plated on agar plates laden with ampicillin. The wild type ampicillin resistance gene is contained within the original plasmid and thus bacterial colonies that grow from a single transformed bacterial cell bearing ampicillin resistance can be selected. Ampicillin resistant colonies are picked, usually 50 at a time and processed for DNA sequencing. The control reaction is shown below the plates in the DNA sequence containing the eGFP gene with the gene highlighted. For the experiments demonstrated herein, the TAG codon was targeted for conversion to TAC.

Two genetic readouts are used in the present study. The first is the conversion of kanamycin sensitivity to kanamycin resistance through correction of a G residue to a T residue in the plasmid pKan (FIG. 7A). The other plasmid does not contain an antibiotic resistance gene for easy selection, but bears an ampicillin resistance gene in wild type form for screening cells that have received the plasmid after transformation (FIG. 7B). In this case, the target is an eGFP gene bearing a stop codon whereupon conversion from the third-base, G, to a C, generates the wild type eGFP. Both plasmids are used to demonstrate the versatility of this system vis-à-vis selection of antibiotic resistance or through colony screening and direct, unselected DNA sequencing.

The cell free extract is capable of catalyzing a single base repair at low levels as evidenced by a variety of combinations tested (Table 2). Addition of gene editing components individually, including the RNP or the oligonucleotide, does not catalyze site specific mutagenesis and conversion of the pKan plasmid. Simultaneous addition of the RNP, single-stranded oligonucleotide and the cell free extract catalyzed a highly significant increase in the conversion of the pKan sensitive to pKan resistant plasmid. Four experiments were carried out in triplicate to generate the data are presented in Table 3; the average range of colony numbers is presented.

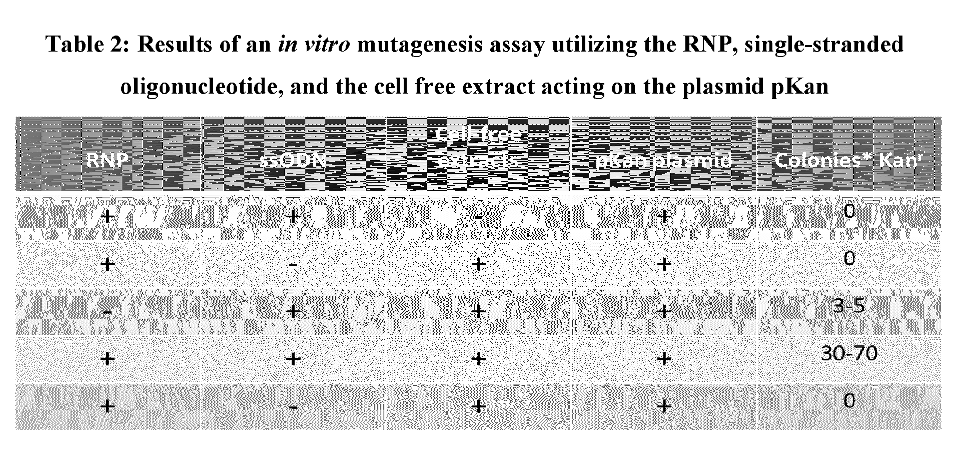

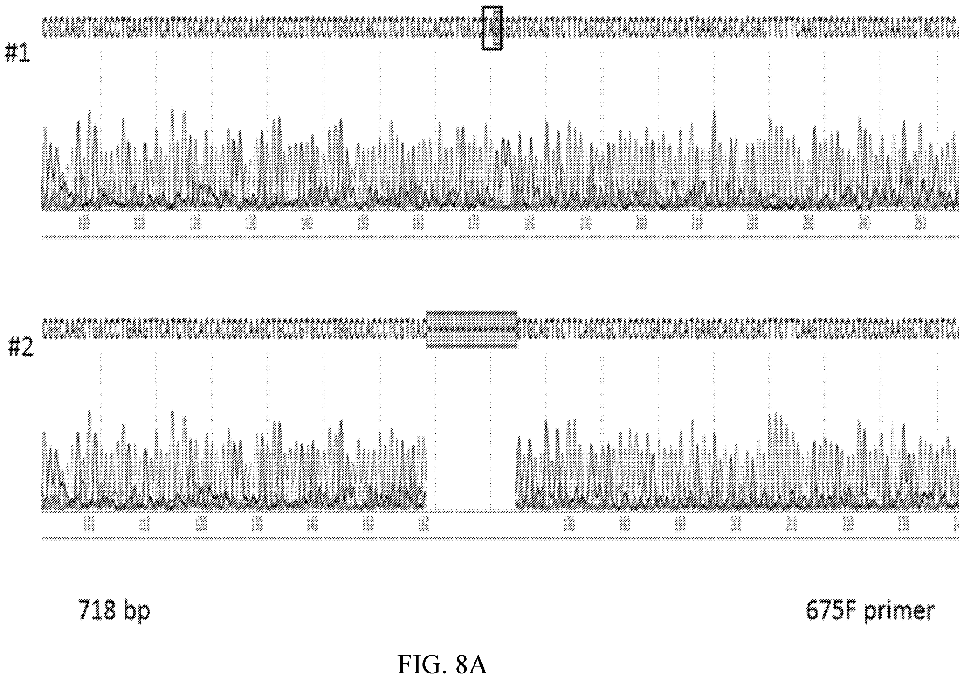

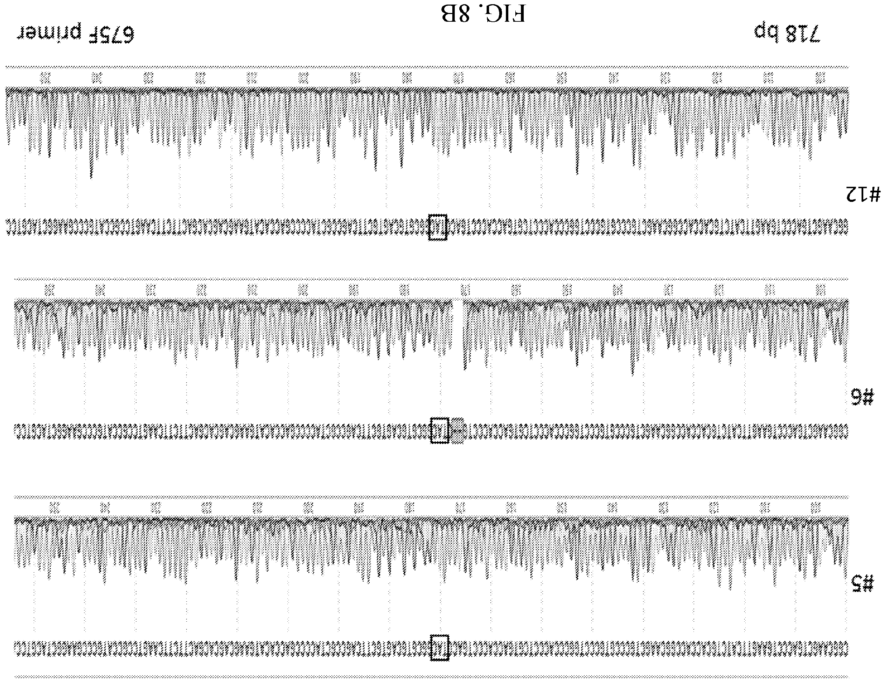

A single base alteration or mutagenesis in converting peGFP⁻ to peGFP⁺ required all reaction components including the RNP, single-stranded oligonucleotide and the cell free extract (Table 3). In the absence of the single-stranded DNA or RNP, the cell free extract catalyzed no mutagenic events within the eGFP gene (without selection). Sequences in FIGs. 8A-8B illustrate that point mutations, deletions and insertions were created in the nine out of 100 colonies analyzed. Three colonies bearing the exact correction were seen within the 100 amp resistant colonies. This is site-directed mutagenesis without selection. These results demonstrated the remarkable versatility of the *in vitro* system in that base substitution, base deletion and base insertions can all be catalyzed by this *in vitro* system within the same reaction mixture. Thus, multiple types of specific mutations can be made within the same gene at the same target site, separated and isolated as individual plasmid molecules after selection.

**Table 3: Results of site directed mutagenesis experiments in the peGFP plasmid**

| RNP | ssODN | Cell-free extracts | peGFP plasmid | Altered plasmid / 100 Amp^{r} |
|---|---|---|---|---|
| + | - | - | + | 0 |
| + | + | - | + | 0 |
| + | + | + | + | 6^{∗} |
| - | + | + | + | 0 |

| | | | | |
|---|---|---|---|---|
| ^{∗} Mutagenesis type: 3 deletions, 3 point mutations | | | | |

Representative DNA sequencing results from plasmid DNA modified *in vitro* and isolated from clones are shown in FIGs. 8A-8B. The numbering is random as these clones were not isolated in any specific order. Clone 1 illustrates isolated plasmid bearing no genetic change. The targeted base, TAG, remained intact. The majority of clones had no change at the target site. Clone 2 contained a clean deletion surrounding the target site. Clone 5, contained a successfully changed point mutation; TAG to TAC. Clone 6 contained the same G-C change except it also contained a two base deletion upstream from the target site. Clone 12 again contained a perfectly modified C at the target site of the third base of the stop codon, TAG. These experiments demonstrate the site-directed mutagenesis reaction. What is important and interesting is within the same population of isolated clones, a variety of specifically mutagenized DNA sequences could be obtained in the same reaction, including the desired point mutation change. This is not possible with any other *in vitro* mutagenesis assay.

### Example 3: Optimizing reaction conditions and development of alternative methodologies to improve frequency and accuracy

The Cas9 protein contains two nucleolytic domains buried within the binding domains of the intact protein. Genetic engineering carried out on Cas9 has now generated two variations of the wild type protein; inactive Cas9, often referred to as dead Cas9 (dCas9) and Nickase, an enzyme in which one of the two nuclease domains has been altered to inactivity. The enzyme retains the capacity to cleave one of the two strands of the double helix. Both of these Cas9 variants can be tested in a methodical fashion within the *in vitro* mutagenesis assay in order to improve the frequency and versatility of the overall reaction. The rationale is that the introduction of a single break or the unwinding of the DNA helix at the site targeted for mutagenesis by the oligonucleotide can improve the specificity and efficiency of the overall reaction.

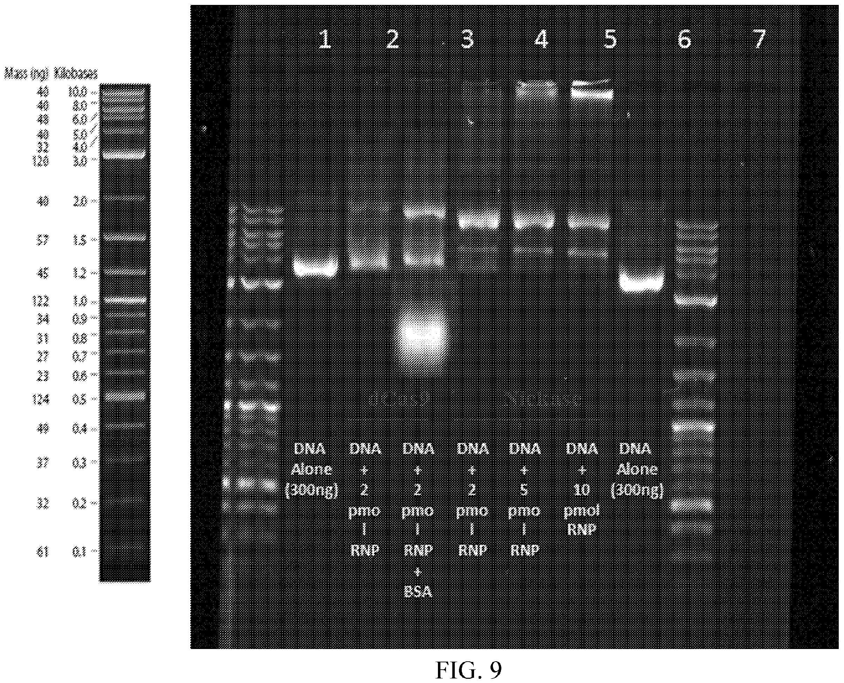

The optimization process began by examining the interaction of the dCas9 and Nickase on superhelical DNA. As illustrated in FIG. 9, dCas9 bound to and retarded migration of the super helical DNA through the agarose gel. Addition of Nickase converted superhelical DNA to an open circular or nicked form of DNA. As the dosage of Nickase was increased, several other forms of DNA appeared on the gel, particularly as a slow-moving complex visualized just below the loading wells of the gel. In the case of the dCas9 reactions, the band shift of superhelical DNA was observed in lane 1. Lane 2 displayed a reaction mixture containing BSA, as suggested by the manufacturer. Under these conditions, a fast moving molecular mass was observed migrating ahead of the superhelical substrate as well as a slower moving molecule that migrated somewhat more slowly through the gel (this upper band matched the predicted migration pattern on the spec sheet provided by the manufacturer). The two types of modified Cas9 demonstrated clear interaction with DNA and can be evaluated for gene editing activity after being assembled into the RNP complex in the *in vitro* mutagenesis assay.

### Example 4: Modifying target genes utilizing a cell-free extract system, an RNP, an oligonucleotide and a plasmid expression vector

The present invention provides a novel method to modify target genes and utilizes a cell-free extract system, a RNP, an oligonucleotide and a plasmid expression vector. Expression vectors contain the gene of interest (target gene) and, in certain embodiments, a mutated Kanamycin gene which serves as the correction marker for selection of genetically altered plasmids. Gene alteration, directed by specific oligonucleotides, is measured by utilizing a genetic readout in *E. coli.* In this embodiment, a mutant gene conferring resistance to Kanamycin inserted into the expression construct is co-targeted for correction by a standard oligonucleotide, 72 bases in length bearing specific phosphorothioate linkages. Gene editing occurs in a cell-free extract, generated from mammalian or yeast cells, and expression of the repaired Kanamycin gene is measured by the appearance of kanamycin resistance bacterial colonies on agar plates. For Kanamycin resistance, the objective is to convert a G/C base pair, (mutant) at position 4021 in the gene, to a C/G base pair (functional) concurrently with the genetic alteration in the target gene. The isolated plasmids are then electroporated into *E. coli* lacking a functional RecA protein. Bacterial strains deficient in homologous recombination and mismatch repair are used for this readout since these bacteria do not catalyze targeted nucleotide substitution on their own. This strategy minimizes background levels and enables a more efficient identification of plasmids bearing the specific targeted gene alteration. Isolation and DNA sequencing of the plasmids from *E. coli* confirms genetic alteration through the initial selection of Kanamycin resistance which signals that gene editing activity has taken place on that plasmid.

### Example 5: Nucleotide insertion

The same basic reaction workflow described herein was utilized for nucleotide insertion: DNA cleavage catalyzed by an RNP particle, addition of donor DNA, in this case a pre-annealed double strand fragment, followed by the addition of a mammalian cell free extract generated from HEK293 cells supplemented with exogenously added DNA ligase. The objective here was to insert a 15 base double-stranded fragment at the site of RNP cleavage as opposed to the intent in previous examples of creating a double strand break to execute deletion of DNA sequences contained within the plasmid. This demonstration expands the applicability of the gene editing *in vitro* mutagenesis assay into the realm of gene or DNA insertion, an important barrier to be crossed for the *in vitro* reaction.

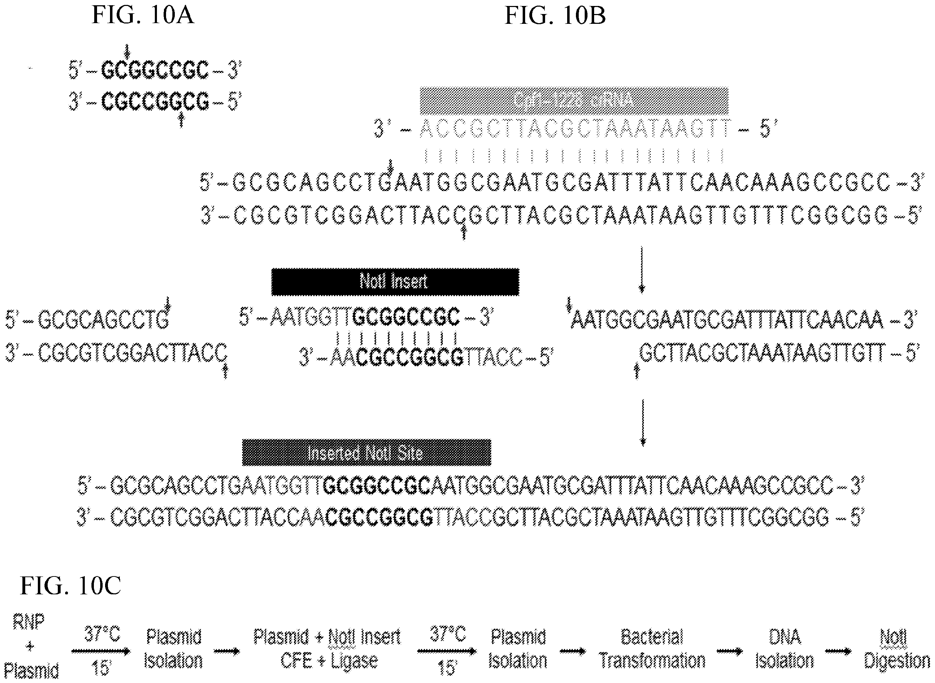

The details of the reaction itself are as follows (FIGs. 10A-10C): Two complementary single-stranded oligonucleotides were designed (5'-AATGGTT**GCGGCCGC**-3' (SEQ ID NO: 1) and 5'-CCATT**GCGGCCGC**AA-3' (SEQ ID NO: 2)) to be duplexed for a NotI restriction site insert, each with 5' overhangs complementary to one of the two overhangs generated at the Cpfl RNP staggered cut site. An *in vitro* reaction was set up to generate a staggered double stranded break on the plasmid DNA which included supercoiled plasmid DNA and Cpf1 RNP. This reaction was incubated for 15 minutes at 37°C. The linearized DNA generated from the first reaction was isolated and purified using a silica membrane column and added to a second *in vitro* reaction to insert the duplexed NotI restriction site insertion which included the purified linearized DNA, NotI site insert, and a modified HEK CFE including ligase. This reaction was incubated for 15 minutes at 37°C.

The re-circularized DNA generated from the second reaction was then isolated and purified using a silica membrane column and transformed into DH5α competent *E. coli.* Bacteria were plated onto agar plates containing Kanamycin antibiotics and incubated overnight at 37°C. Single colonies were picked from overnight plates and cultured in broth containing Kanamycin antibiotics in a shake incubator at 37°C for 16 hours. Supercoiled DNA from overnight cultures was isolated and included in a NotI digestion assay and linearized DNA containing the NotI insert was visualized on an agarose gel. DNA showing NotI enzyme cleavage was then subject to PCR amplification and sequenced to confirm the NotI site insertion.

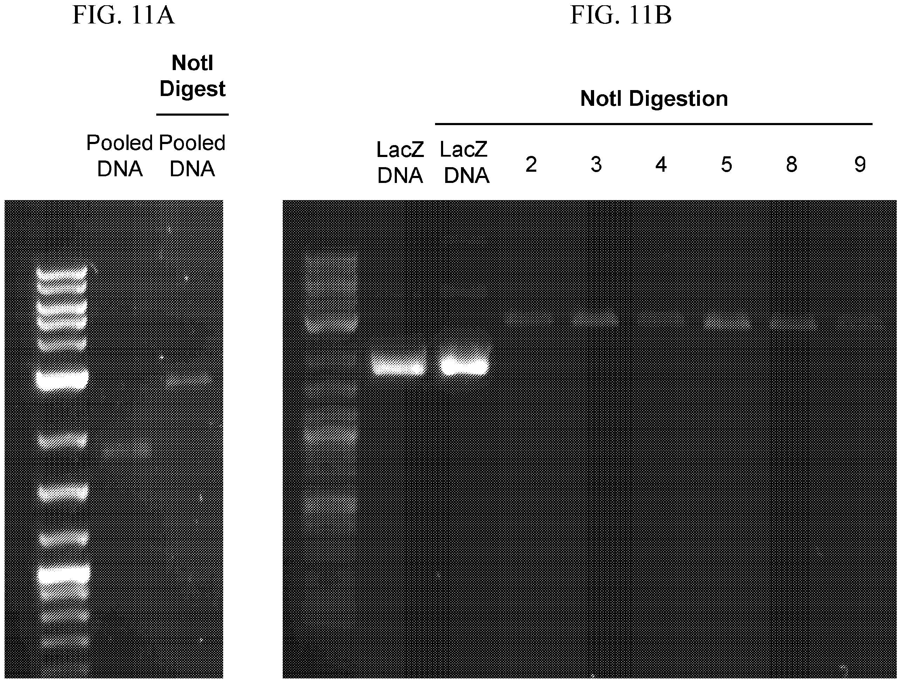

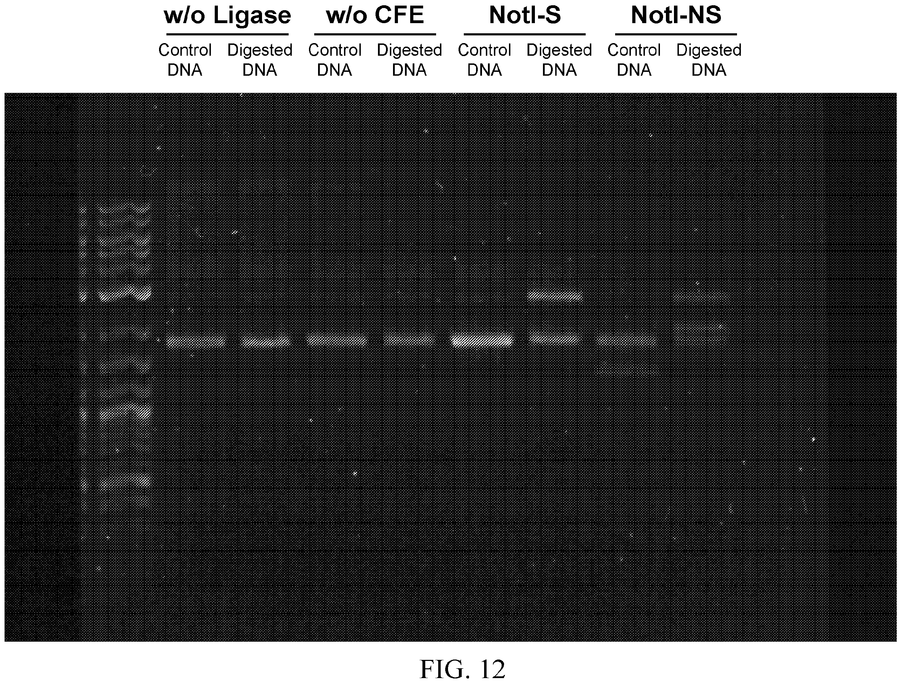

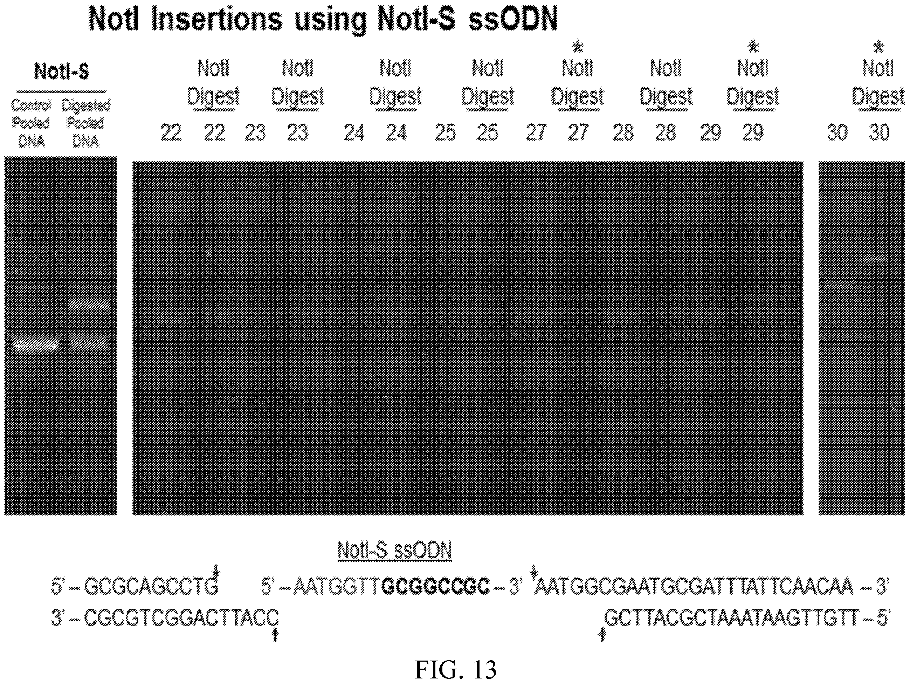

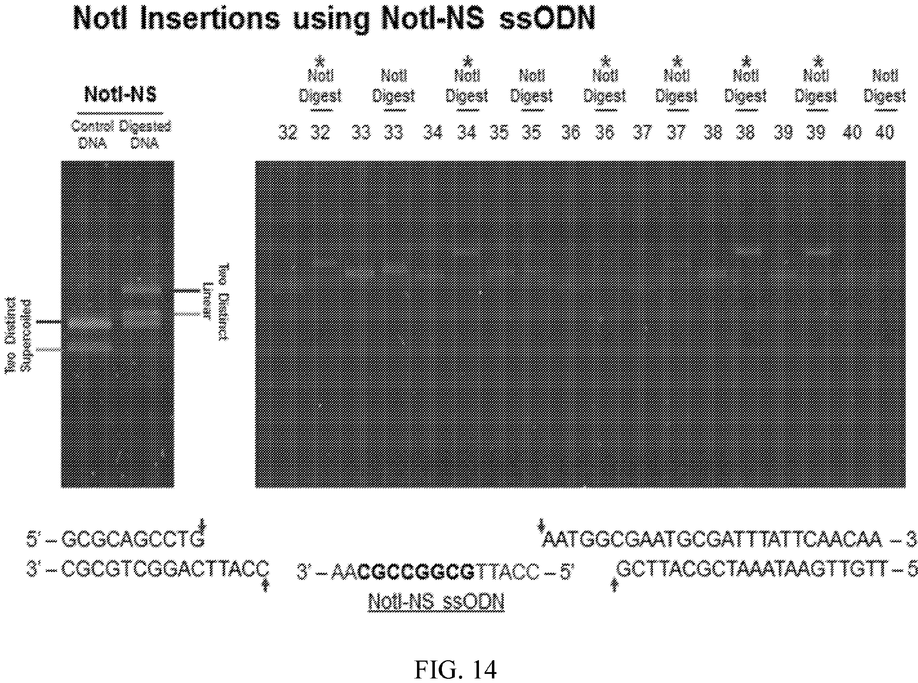

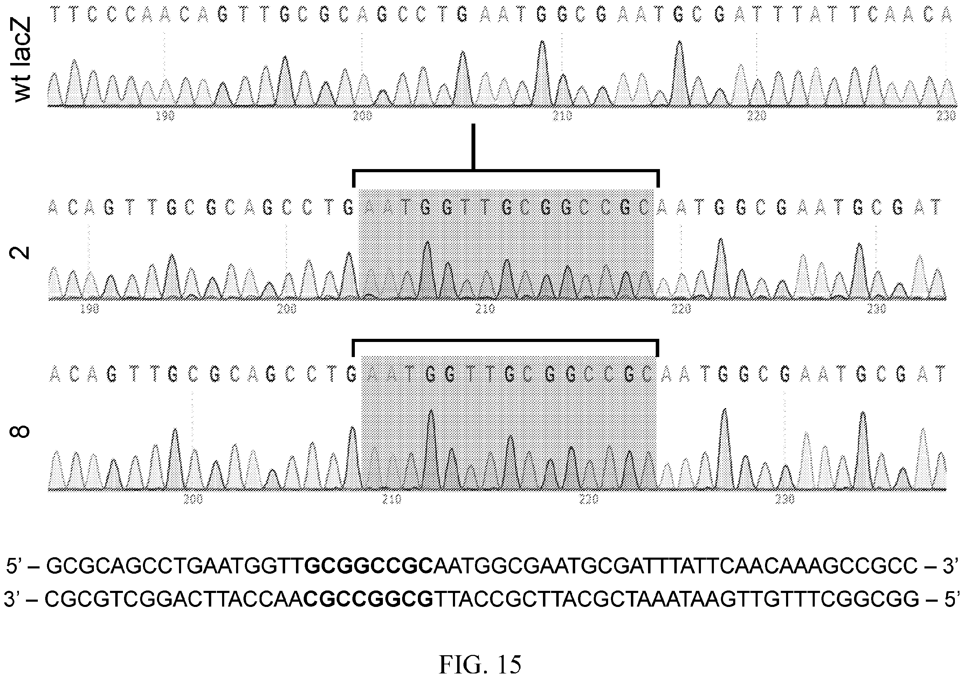

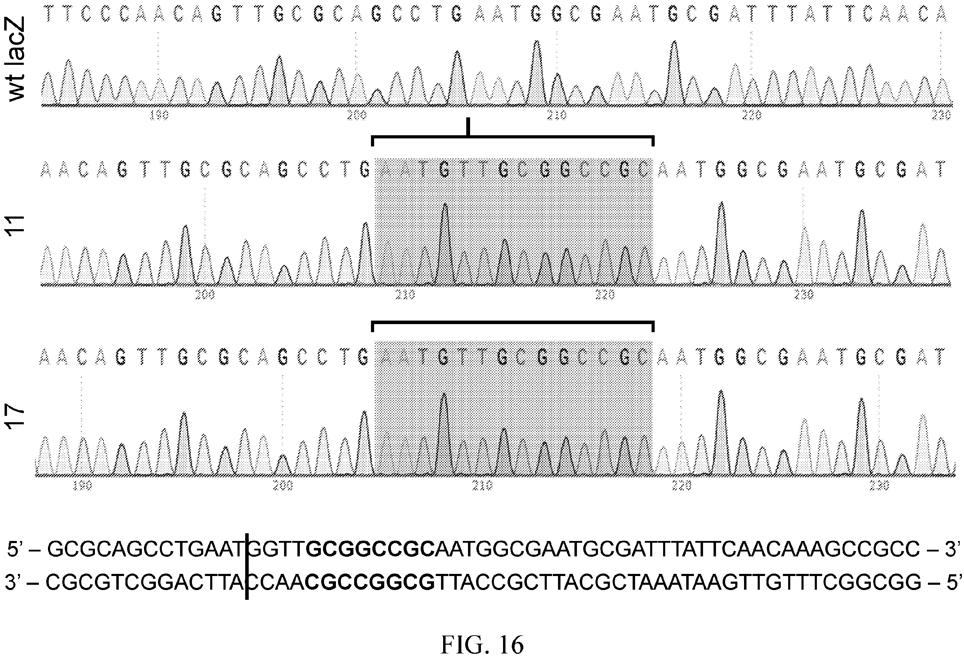

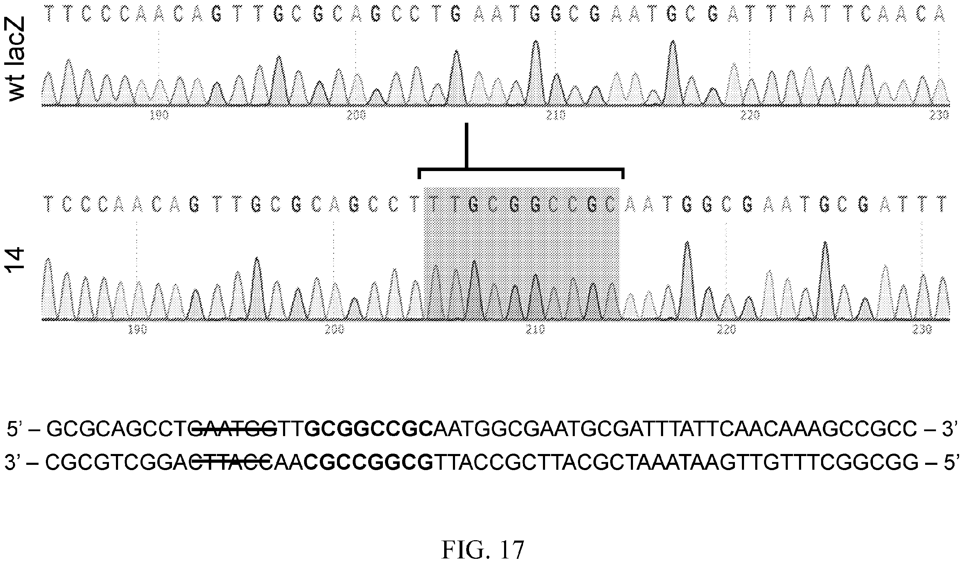

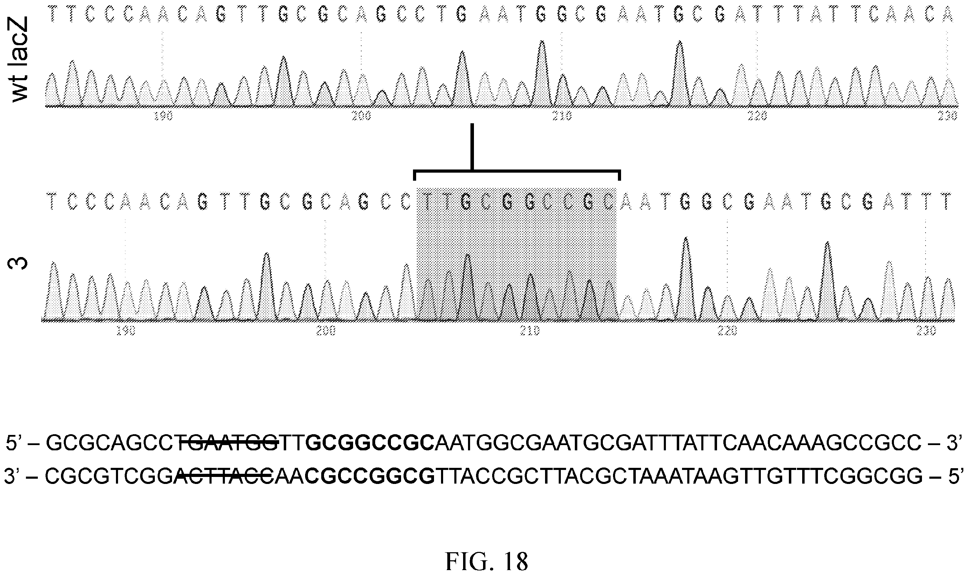

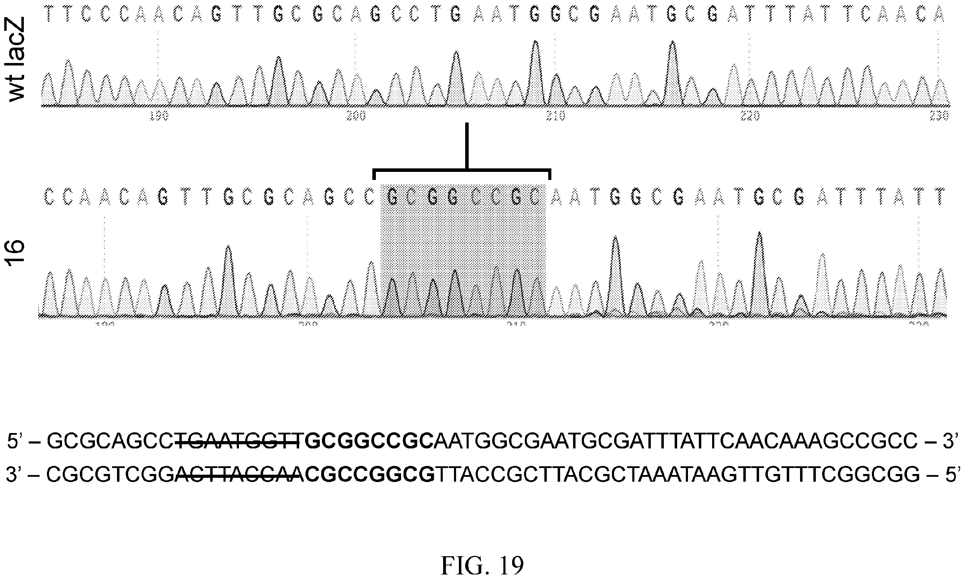

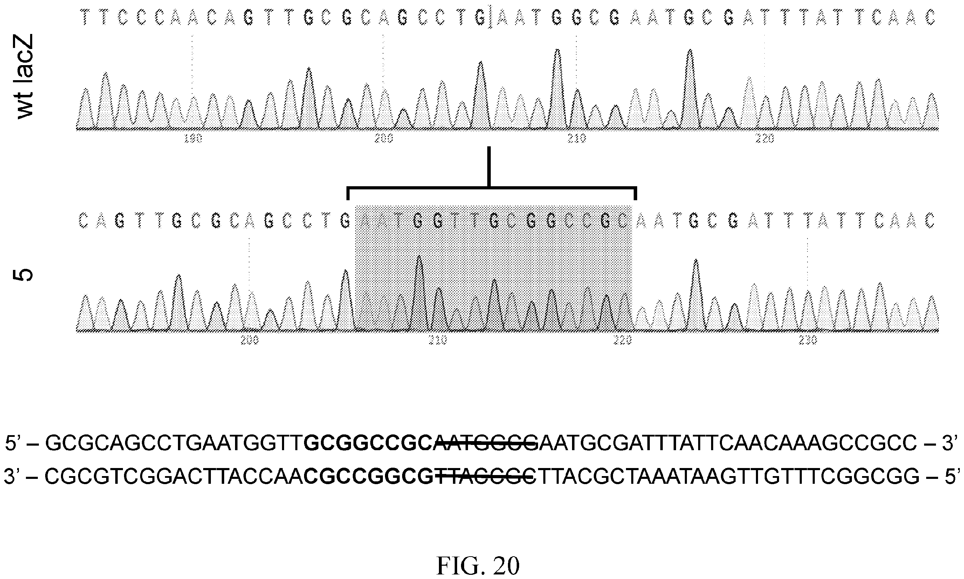

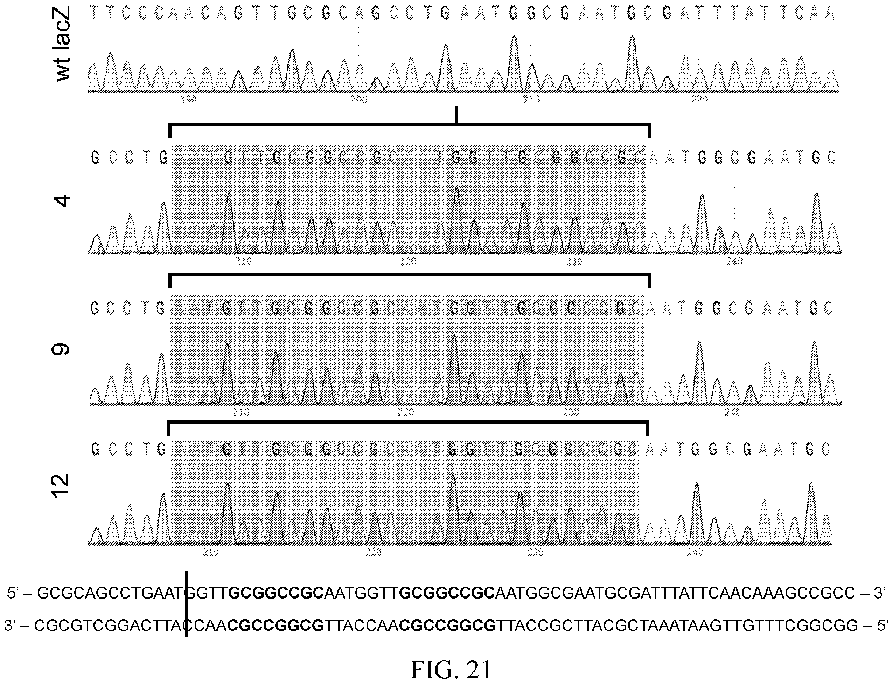

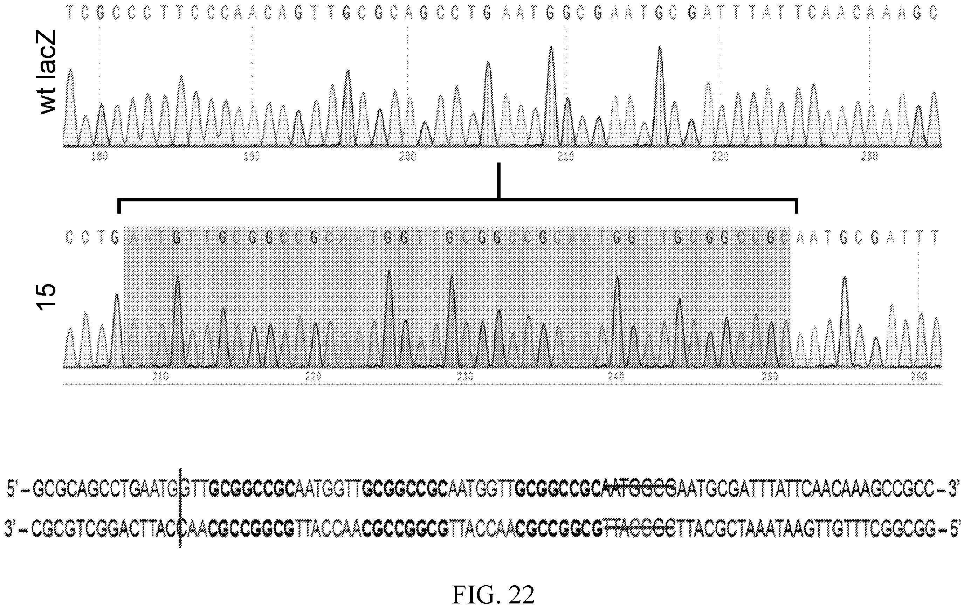

Restriction enzyme cleavage, RFLP, and DNA sequence analyses of purified plasmid DNA that has been subjected to NotI restriction digestion were performed. Results illustrated the linearization of the circular plasmid DNA. The NotI restriction site does not appear within the plasmid and thus linearization could only have occurred if the fragment had been successfully inserted through the process of *in vitro* gene editing (FIGs. 11A-14). DNA sequence analyses confirmed the insertion of the double strand fragment at the precise site intended and directed by the RNP particle (FIGs. 15-22). Several distinct DNA sequences were observed, some of which exhibit a perfect and clean insertion of the intended fragment.

### Example 6: DNA cleavage and modification induced by Cas9 or Cpf1 RNP particles

A fully integrated cell-free system for studying the process of gene editing was constructed herein. FIG. 23A illustrates the initialization of the reaction using Cas9 or Cpfl assembled into an RNP particle to direct site-specific cleavage of plasmid DNA, followed by the addition of a mammalian cell-free extract supplemented with DNA ligase. The reaction was incubated at 37°C for 15 minutes, and the plasmid DNA was recovered and transformed into *E*. *coli* for subsequent colony selection and plasmid DNA analyses. The cell-free extract provides the catalytic activities including DNA resection, phosphorylation and ligation, among others, needed for the processing of the linearized plasmid. In the presence of exogenously added donor DNA, fragments of DNA can be inserted with precision at the cleavage site.

A variety of Cas9 and Cpfl sites were targeted along the lacZ gene region as directed by the appropriate guide RNA sequences displayed in FIG. 23B. The Cpfl site used primarily in these experiments is surrounded by a box with the associated cut site illustrated by a staggered arrow. The Cas9 site used in this study is illustrated by the bar which depicts the position of crRNA binding. The lacZ gene embedded in the plasmid pHSG299 provides an attractive template for this assay because, the destruction of the lacZ gene through DNA deletion or insertion can result in the production of a non-functional β-galactose protein unable to metabolize X-gal. When plasmids containing a disrupted lacZ gene are introduced into bacteria cultured in the presence of X-gal, a well-established change in the color, from blue to white, is seen.

FIGs. 24A-24C demonstrate the *in vitro* activity of the assembled RNP particles. First, FIG. 24A illustrates a simple reaction in which cleavage of supercoiled pHSG299 is induced by RNP variants *in vitro.* The nuclease activity on pHSG299 is evident at each of the varying Cpfl cleavage sites as well as the wild-type and associated nickase Cas9 cleavage sites. Secondly, a dose dependency for both Cas9 and Cpfl is presented in FIG. 24B, with Cpfl displaying cleavage of supercoiled DNA at a lower picomole level. Cleavage activity by Cas9 RNPs revealed a 'threshold' limit for DNA cleavage in which complete linearization of supercoiled DNA was not seen until 5pmol of RNP was present. A very slight linear DNA band began to appear in the presence of 2pmol and all reactions with 5pmol or more showed nearly all DNA to be completely cleaved. In contrast, cleavage activity by Cpfl RNPs revealed more of a 'gradient' increase in DNA cleavage. A very slight linear DNA band began to appear in the presence of as low as 0.5pmol of RNP and increased incrementally until 1pmol was present and all reactions with 2pmol or more showed nearly all DNA to be completely cleaved. The difference in cleavage patterns seen between Cas9 and Cpfl *in vitro* may reflect the enhanced catalytic activity of Cpfl within this system. These results demonstrated the utility of both Cas9 and Cpfl in combination with specific guide RNA to act as a genetic tool for DNA structural modification.

A mammalian cell-free extract was previously utilized to investigate the mechanism and regulation of gene editing directed by ssODNs (Engstrom et al. BioEssays 31, 159-168 (2009)). Much of the data that led to the elucidation and re-construction of that gene editing pathway originated from studies carried out *in vitro* (Cole-Strauss. et al. Nucleic Acids Res. 27, 1323-30 (1999)). The same experimental workflow was employed in developing the cell-free, CRISPR-directed gene editing system. FIG. 24C illustrates the catalytic activity of the extract on linearized DNA generated by Cpfl (see FIG. 24B). Extracts, prepared from cell sources, displayed varying degrees of DNA resection activity on the linearized plasmid. Each source of extract provided suitable levels of nuclease activity reducing the size of the linearized plasmid as a function of increasing extract concentration. The activity exhibited by an extract prepared from HEK-293 cells was of particular interest because these cells are believed to have appropriate levels of DNA repair and DNA recombination enzymatic activities.

### Example 7: Specific DNA deletion directed by gene editing components in a-cell free system

As described herein, a CRISPR-based gene editing system was established that could be used to elucidate the molecular pathways and regulatory circuitry surrounding genome modification in human cells. Toward this end, the DNA cleavage activity on pHSG299 was taken advantage of in an *in vitro* reaction involving Cas9 RNP, pHSG299 and the mammalian cell-free extract. The main objective was to create a specific deletion at the break site followed by recirculation and genetic readout in bacteria. FIG. 25A outlines the genetic target with the associated genetic tools and FIG. 25B illustrates the results. Despite concerted efforts and analyses of 18 clones, no modified plasmid molecules bearing sequence deletions generated from reactions catalyzed by the Cas9 RNP were identified. Several preliminary experiments were carried out in which the nuclease activity of a Cas9 RNP particle was combined with two separate exonucleases, T7 and Exo I. In only one case with a unique combination of enzymes was a resected DNA sequence observed; this reaction was not robust. This negative outcome may be due to the fact that Cas9 cleavage results in the generation of blunt-ended double stranded DNA breaks which might be less amenable to DNA resection *in vitro,* unlike *in vivo.*

Next, this reaction was repeated and the nuclease Cpfl substituted in place of Cas9. FIG. 25C demonstrates successful gene editing of the lacZ gene in pHSG299. Multiple bacterial clones transformed with plasmid recovered from the *in vitro* reaction were found to harbor sequence deletions surrounding the targeted site, indicated by the staggered arrow in FIG. 25A. Representative sequence panels are shown to demonstrate the type of DNA deletions found within the clonal population, including clones in which DNA sequences were found to be unaltered. Twenty-two out of the forty-one sequences analyzed displayed DNA alterations surrounding the cleavage site created by the Cpfl RNP. These data demonstrated the development of an experimental system in which Cpfl catalyzes gene editing *in vitro.* Modified plasmids in bacterial colonies exhibited various shades of blue; DNA deletions were found in colonies colored white, pale blue and deep blue. Thus, blue colonies could not be eliminated from the screening process and it could not be ruled out that these colonies contained altered DNA sequences.

### Example 8: Specific donor DNA insertion directed by RNP in a cell-free system

The experimental system was expanded by attempting to carry out DNA insertion via homology directed repair (HDR) at the designated Cpfl cleavage site. To do so, two short, complimentary DNA molecules were synthesized that, upon annealing, created a double-stranded DNA fragment containing a cleavage site for the restriction enzyme NotI, with no inherent site in pHSG299. The NotI restriction site and experimental system is displayed in FIGs. 26A and 26B, respectively, and the experimental protocol outlined in FIG. 26C. In an effort to examine discrete gene editing events, the homologous regions between the integrated arms of the exogenously added fragment and the native lacZ gene region were distinguished by including a two base pair "barcode" (TT: AA) at an upstream position relative to the NotI site. As an intermediate step, and to check for the presence of the inserted fragment, plasmid DNA was isolated from selected bacterial colonies and digested with the restriction enzyme NotI. As shown in FIG. 26D, a number of purified plasmid DNA samples were digested by NotI, generating linearized plasmid DNA as predicted; fragment insertion was dependent on the presence of cell-free extract within the reaction mixture, as NotI cleavage was not observed in its absence.

Plasmid DNA samples exhibiting NotI restriction were amplified by PCR and sequenced to verify DNA insertion. FIGs. 27A-27C display the array of DNA fragment insertion patterns found within the NotI positive population with the wild type sequence presented above the sequence panels. FIG. 27A displays the sequences of several clones that contain a precise and perfect insertion at the designated site of cleavage. Approximately 15% of the plasmid DNA, isolated from colonies transformed with DNA and recovered from *in vitro* reactions containing the duplexed NotI insertion fragment, was found to contain the perfect insertion. Other insertion patterns included one in which two NotI fragment insertions were observed accompanied by a single base pair deletion (FIG. 27B); in all three sequences, the one base deletion occurred at the last nucleotide positioned on the 5' overhang end upstream from the insertion site. FIG. 27C displays a DNA sequence in which three NotI fragment insertions accompanied by multiple deletions surrounding the inserted NotI fragment; a single base pair deletion upstream from the first inserted fragment and a seven base pairs deletion downstream from the third inserted fragment. Approximately 8% of the plasmid DNA had no alteration in the DNA sequence as seen in FIG. 27D.

### Example 9: Specific DNA insertion promoted by single-stranded DNA molecules

The possibility that HDR and fragment insertion could be directed by single-stranded DNA molecules, instead of by the annealed double strand DNA fragment, was examined. FIG. 28A and 28B, provide a diagram of the single-stranded molecules used in this experiment and their orientation/position relative to the target site; these single-stranded molecules comprised the double-stranded fragment used in the previous experiments described herein (see FIG. 26B). Each molecule has a ten base overhang, with a five base region complementary to the Cpfl staggered cut site overhangs. Following the same experimental protocol outlined in FIG. 4C, the purified DNA recovered from reaction mixtures was transformed into bacterial cells and plasmid DNA was isolated from selected bacterial colonies. NotI restriction digestion was carried out on the isolated DNA samples and the results are presented in FIG. 28C. Focusing first on DNA plasmids isolated from a reaction containing the single-stranded oligonucleotide which integrates into the sense strand, NotI-S, it was observed that several products containing an insertion can be cleaved by NotI enzyme digestion. Samples from a reaction containing the single-stranded oligonucleotide, NotI-NS, which integrates into the nonsense strand, also generated product molecules bearing the NotI restriction site. Taken together, a population of plasmid molecules that can be successfully cleaved by incubation with NotI was observed, indicating the successful insertion of at least part of the single-stranded DNA molecule that contains the NotI site.

DNA sequencing was carried out across the region of interest for NotI positive plasmid samples recovered from both of the *in vitro* single-stranded DNA molecule insertion reactions. A representative panel for each is presented in FIG. 28D. Sequencing data confirmed the RFLP analysis and revealed a heterogeneous population of sequence inserts within both categories of clones. Importantly, in 50% of the sequences analyzed from reactions driven by NotI-S, a perfect single insertion of the intended molecule was seen. Perfect insertion of the fragment was not detected when NotI-NS served as the single-stranded donor molecule. In all cases where no perfect insertion was detected, each clone contained a variable amount of DNA modification, often in the form of a deletion. The strand bias exhibited in this HDR-directed reaction will likely provide insight into the mechanism of fragment insertion in the *in vitro* gene editing.

### Example 10: Insertion of 186-bp fragment

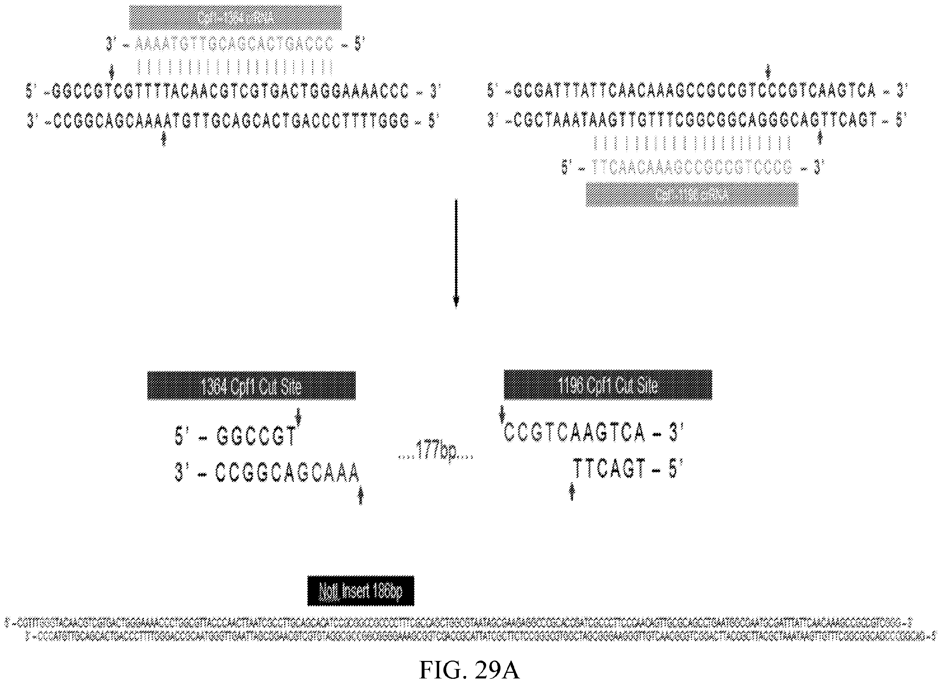

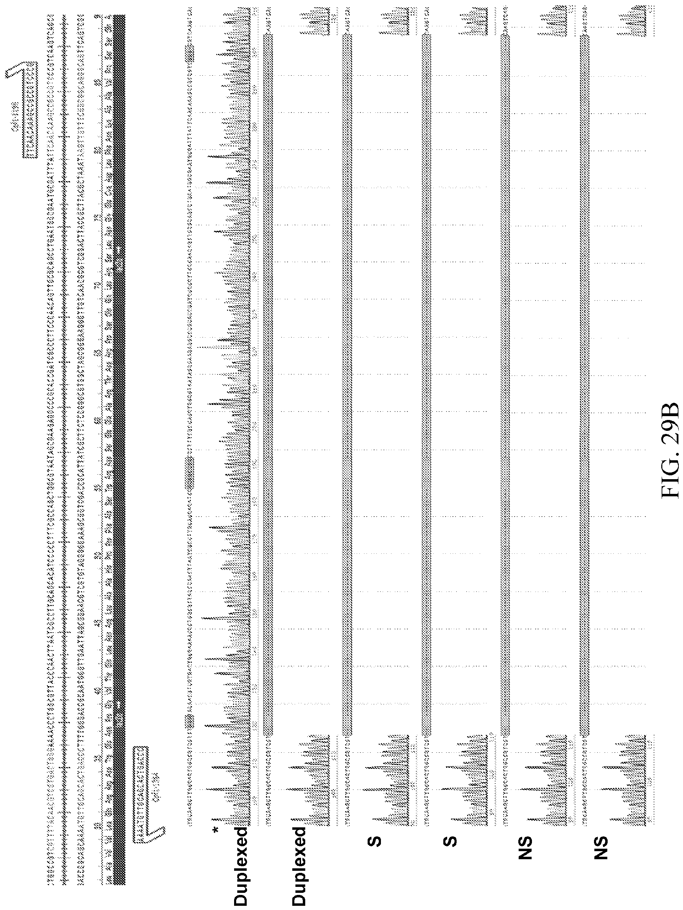

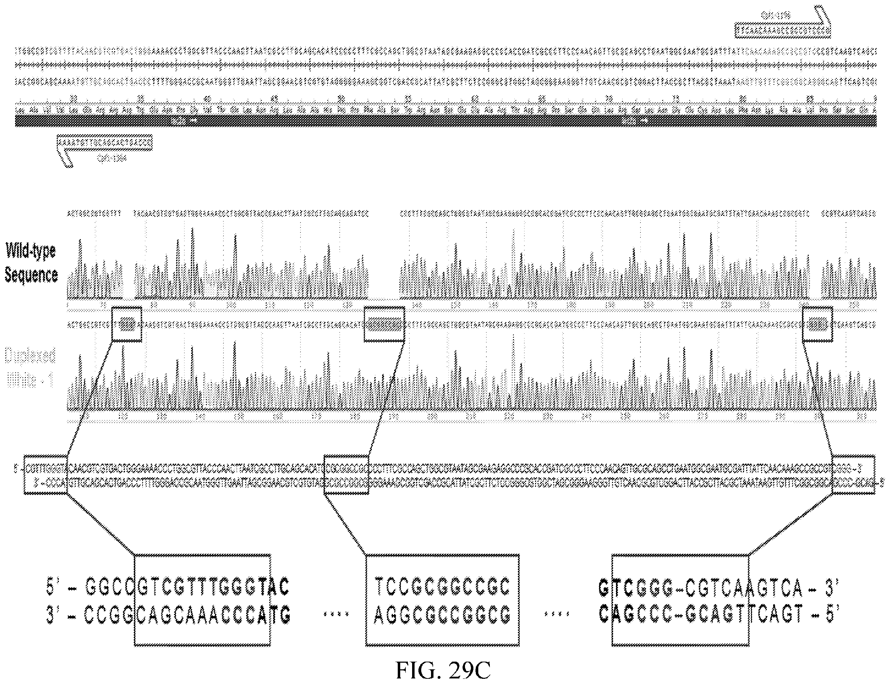

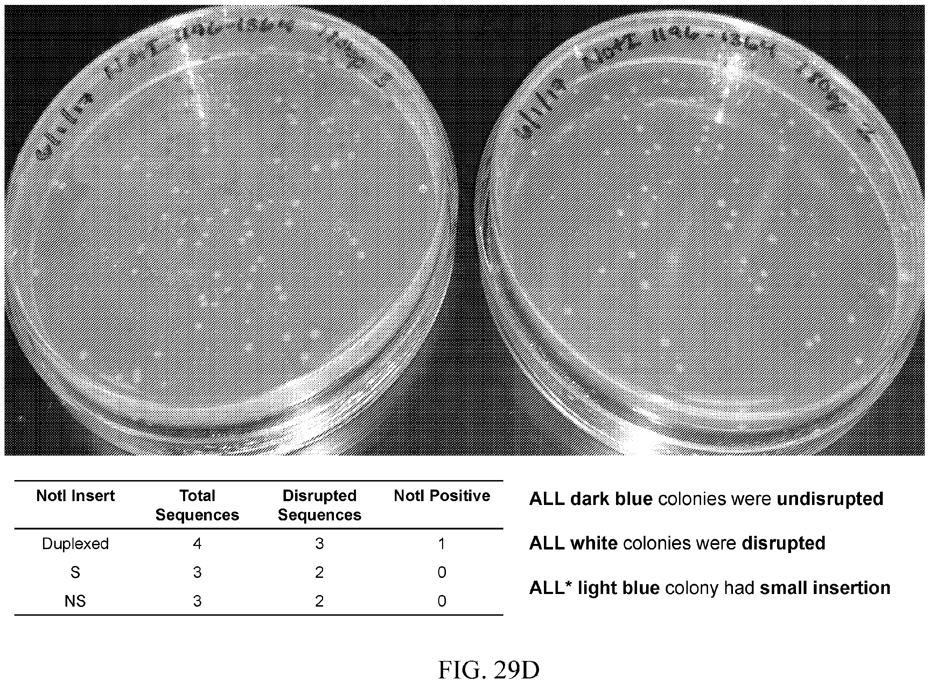

In this experiment, two Cpfl nucleases, cutting at different sites, were used to excise a fragment from the parent plasmid and replace it with a fragment of 186 base pairs, created by the annealing of two complementary oligonucleotides with complementary overhangs as indicated in FIG. 29A. The sequences presented in FIGs. 29B-29C display the sequences obtained for the *in vitro* reaction with readout in bacteria. FIG. 29D illustrates the type of outcomes obtained, including mostly deletions and one successful insertion clone. Importantly, whereas previous data presented herein used only one Cpfl enzyme, in this experiment two Cpfl enzymes were successfully used to drop out a fragment of a plasmid and successfully insert a similarly sized donor DNA fragment.

### Example 11: Site-specific DNA insertion

The site-specific DNA cleavage activity of an RNP complex, containing the Cpfl nuclease, was combined with a mammalian cell-free extract that can catalyze DNA resection, DNA insertion, and gene segment replacement within a plasmid using donor DNA fragments (FIG. 23A). Plasmid DNA containing CRISPR-directed modifications were recovered from *in vitro* reactions and transformed into competent *E. coli* which were then plated on agar laden with kanamycin. Modified plasmid DNA was identified initially through antibiotic resistance and phenotypic changes (*i.e.* color) and/or directly by DNA sequence analyses. Two targeting systems were utilized; plasmid pHSG299 which contains an intact and functional copy of the lacZ gene and plasmid pKRAS6163 which contains an intact and functional copy of the oncogene KRAS. Insertion or replacement reactions occurring within the lacZ gene results in a change of color from blue to white indicative of gene editing activity. Insertion or replacement in pKRAS6163 requires DNA sequence analysis to identify successful gene editing activity.

This *in vitro* system demonstrated great versatility in enabling the insertion of a donor DNA fragment at a single Cpfl RNP cleavage site or the replacement of a gene segment with a donor DNA fragment through the action of two Cpfl RNP complexes at distinct cleavage sites. In addition, this system also produced plasmid molecules containing site-specific deletions through the resection of the DNA ends created by the action of the RNP complex. Thus, a multiplicity of gene editing reactions were occurring simultaneously, competitively recapitulating how gene editing tools act when introduced into cells. These three pathways are depicted schematically in FIG. 30; insertion required a single cut and replacement required a double cut at the target site. In both insertion and replacement reactions, the distinct cleavage pattern of Cpfl RNPs resulted in DNA overhangs on the 5' ends at each cut site. Donor DNA fragments were then generated by designing two complementary oligonucleotides that, once annealed, harbored the double-stranded insertion or replacement fragment flanked on each 5' end by 5 base overhangs with homology to the staggered ends. The homology of the 5 base overhangs between the donor DNA fragments and the Cpfl cleavage sites facilitated integration of the fragments during the insertion and replacement reactions.

As described herein, a CRISPR-based gene editing system that could be used to elucidate the molecular pathways and regulatory circuitry surrounding genome modification in human cells was established. Cpfl was utilized to initiate the cleavage of supercoiled DNA followed by the addition of the cell-free extract as described herein. FIG. 31 illustrates successful gene editing of the lacZ gene in pHSG299. Multiple bacterial clones transformed with plasmid recovered from the *in vitro* reaction were found to harbor sequence deletions surrounding the targeted site, indicated by the staggered arrow. Twenty-two out of the forty-one sequences analyzed displayed DNA alterations through DNA resection surrounding the cleavage site created by the Cpfl RNP. These data demonstrated Cpfl catalyzed, site-specific DNA deletion can be carried out in this *in vitro* system.

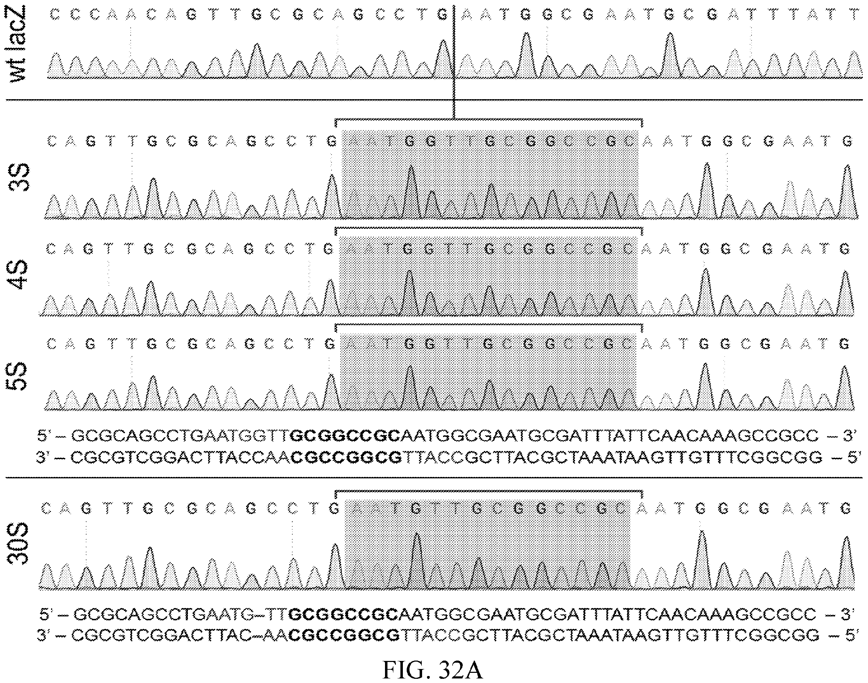

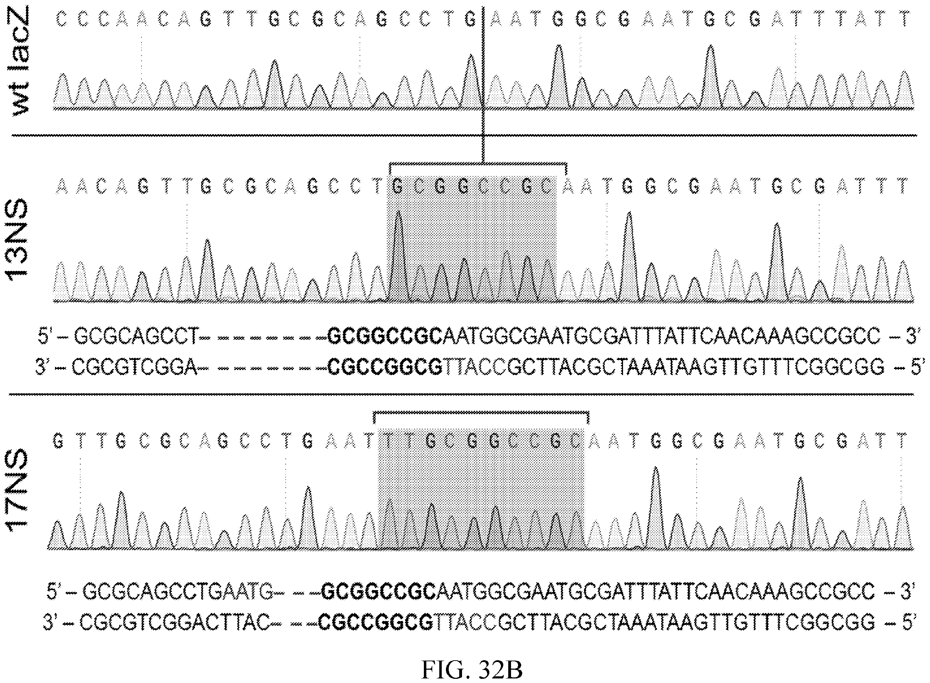

The possibility that this system could reflect the reaction being carried out in mammalian cells wherein homology-directed repair, catalyzed by a single-stranded DNA fragment, is taking place was explored. Single-stranded DNA templates of the preferred substrates for homology-directed repair and successful incorporation of these fragments has been seen reproducibly, whether through imprecise or precise alignment. By designing a system that could recapitulate these reactions in a controllable environment, the function of some of the controlling factors of homology-directed repair in mammalian cells could be identified and elucidated. FIGs. 28A-28B provide a diagram of the experimental strategy and the single-stranded molecules used in this experiment including their orientation relative to the target insertion site. Each molecule has a ten base overhang, with a five base region complementary to the Cpfl staggered cut site overhangs. In an effort to examine discrete gene editing events, the homologous regions between the integrated arms of the exogenously added fragment and the native lacZ gene region were distinguished by a two base pair "barcode" (TT:AA) included at an upstream position relative to the NotI site. As an intermediate step, and to check for the presence of the inserted fragment, plasmid DNA was isolated from selected bacterial colonies and digested with the NotI restriction enzyme. Following the same experimental protocol outlined in FIG. 23A, the purified DNA recovered from reaction mixtures was transformed into bacterial cells and plasmid DNA was then isolated from selected bacterial colonies. NotI restriction digestion was carried out on the isolated DNA samples (FIG. 28C) and clonal DNA isolates that were cleaved by the restriction enzyme were processed for DNA sequence analysis. A representative panel for each is presented in FIG. 28D. Additional DNA sequence data are shown in FIGs. 32A-32B. Sequencing data confirmed the RFLP analysis and revealed a heterogeneous population of sequence inserts within both categories of clones. Importantly, in 50% of the sequences analyzed from reactions driven by the single-stranded oligonucleotide integrating into the sense strand, NotI-S, a perfect single insertion of the intended molecule was seen. When the single-stranded oligonucleotide integrating into the nonsense strand, NotI-NS, served as the donor molecule, perfect insertion of the fragment was not detected. In all cases where no perfect insertion was detected, each clone contained a variable amount of DNA modification, often in the form of a deletion.

### Example 12: Site-specific gene segment replacement

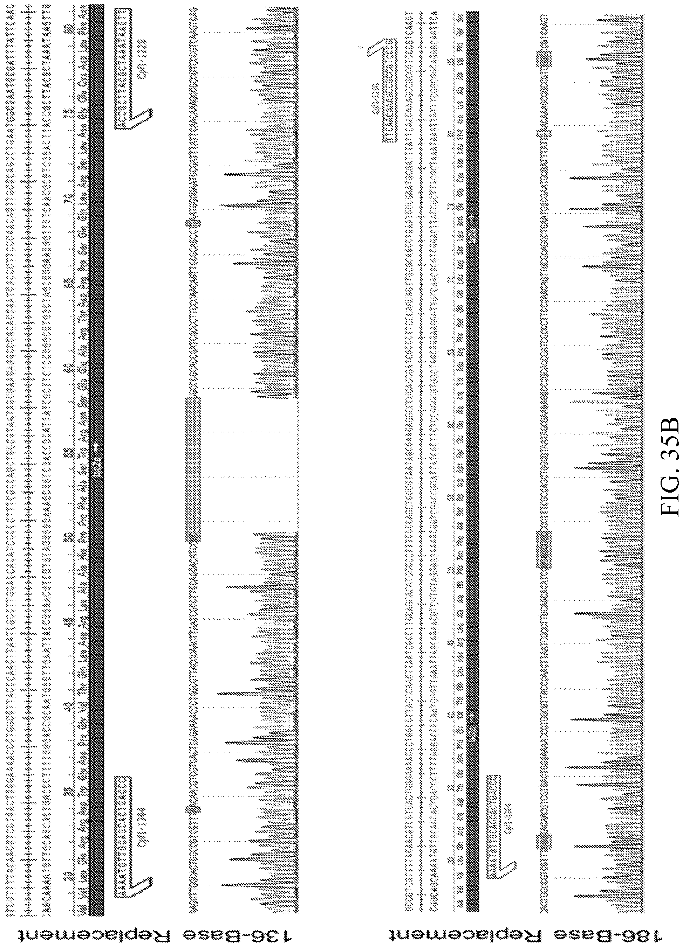

Herein it was demonstrated that both site-specific deletion, perhaps reflecting a non-homologous end joining reaction, and site-specific insertion of the single-stranded DNA fragment, perhaps reflecting homology-directed repair, was possible in this *in vitro* system. The capability of the system to catalytically support the precise replacement of a segment of a gene was tested. One long-term objective of gene editing is to successfully replace a functional copy of the disabled gene or a dysfunctional exon. The same reaction strategy was carried out as was done for assessment of insertion (see FIG. 23A), except in this reaction two Cpfl RNPs were used to generate two cuts at different positions along the lacZ gene to lift out a segment of the gene. Separate reactions were designed to replace gene segments of 81, 136, and 177 base pairs and individual populations of 81, 136 and 186 base donor DNA fragments were added, respectively, to each of the separate replacement reactions. With regard to reactions containing the 81 base DNA donor fragment, 20% of the recovered colonies contained a perfect replacement, in frame, preserving the coding region of the lacZ gene (FIG. 33A). Sixty six percent of the 136 base replacement reactions resulted in plasmids containing a perfect replacement (FIG. 33B) and 10% of the 186 base replacement reactions harbored plasmids bearing a perfect replacement (FIG. 33C). A barcode, AA/TT/GGG, was incorporated into each of the donor DNA fragments to ensure that the homology of the 5' overhangs were distinguishable from the native gene segment. The same experiment was also carried out with donor DNA fragments with lengths of 17, 36, and 45 bases; none of these donor DNA fragments produced precise replacements (FIG. 35A) although they did generate imprecise replacements of varying lengths and composition. These results demonstrated that this *in vitro* gene editing system can catalyze perfect gene segment replacements with donor DNA fragments of lengths ranging from approximately 81 to 186 bases. It's also important to note that site-specific deletions were also found in the population of isolated plasmids produced from reactions containing donor DNA segments (FIG. 35B) confirming the fact that both deletion/resection and replacement reactions were taking place in the same *in vitro* system.

### Example 13: Site-directed mutagenesis

The success of DNA replacement of a section of the lacZ gene with a high degree of precision prompted testing of a unique application of *in vitro* gene editing. Perfect gene segment replacement was observed at a notable frequency, particularly when using donor DNA fragments with lengths between 81-186 bases. It was decided to target the KRAS gene and reengineer well-known oncogenic mutations that are found within the coding region. Two prominent mutations appeared within the first 13 codons; at position 35, a G to A transversion changes the amino acid produced from these codons from glycine to aspartic acid. (FIG. 36A). In the adjacent codon 13, a second G to A transversion again converts a glycine to aspartic acid residue. Each of these mutations, either as single site changes or as a dual site change, can have serious impacts in re-directing the function of KRAS protein. Therefore, all three mutations were generated in a single reaction mixture by multiplexing with three donor DNA fragments bearing the G12D, G13D and a combination of G12D and G13D mutations (FIG. 36B). A donor DNA fragment of 114 bases was utilized because the optimization reaction results indicated that successful and efficient replacement was achievable with fragments ranging from 81 to 186 bases (FIG. 33A-33C). The reaction was carried out as described in FIG. 23A with one change: the molar relationship between the donor DNA fragment and the RNP was maintained, so that in this reaction, only 33% of each of the three different donor fragments was present in the reaction. The results are presented in Figure 36C. Site-specific mutagenesis of the KRAS gene was isolated and confirmed by analyzing plasmid DNA isolated independently from 14 bacterial colonies. Within this population, both individual mutations and the dual mutation were found; 78% of the plasmids sequenced were found to contain either the individual mutations or the dual mutation and 50% were perfectly replaced. These results revealed that this *in vitro* gene editing reaction can be used to multiplex precise gene segment replacement and generate a population of site-specific mutations from a single reaction mixture. In addition, to confirm that this reaction is truly site-specific and is not generating unwanted off-site mutations within the coding region of the KRAS gene, the plasmids were sequenced upstream and downstream past the coding region of the KRAS gene. The map of the sequencing strategy and the position of the replacement site within the coding region of KRAS are displayed in FIG. 36D. No change in the DNA sequence was observed at any other location along the gene apart from the intended mutations at the targeted site on the reengineered plasmid DNA isolated from an *in vitro* reaction containing the G12D/G13D donor DNA fragment displayed in FIG. 36C. These results indicated that this *in vitro* gene editing system can be used for the generation of multiple site-specific mutations within the coding region of a eukaryotic gene without the generation of detectable off-site mutations.
## Claims

1. A method for performing in vitro mutagenesis of a targeted sequence, the method comprising:
incubating a mixture comprising an isolated ribonucleotide particle (RNP), a first plasmid, an oligonucleotide, and a cell extract having enzymatic activity for editing genes,
wherein the RNP comprises a crRNA and a Cas endonuclease,
wherein the first plasmid comprises a nucleotide sequence of the targeted sequence, and
wherein the oligonucleotide comprises a nucleotide sequence that is complementary to the targeted sequence but contains at least one mismatched nucleotide,
thus generating a second, mutated plasmid;
administering the second plasmid to a plurality of non-germ line cells; and
selecting from the plurality of cells at least one cell wherein in vitro mutagenesis has occurred in the targeted sequence.

2. The method of claim 1, wherein the Cas endonuclease is selected from the group consisting of Cas9, Cas3, Cas8a, Cas8b, CaslOd, Csel, Csyl, Csn2, Cas4, CaslO, Csm2, Cmr5, Fokl, T7, Cpfl, Cpf2, CasY, and CasX, preferably wherein the Cas9 endonuclease is spCas9 or saCas9.

3. The method of claim 1, wherein the RNP further comprises a tracrRNA, preferably wherein a single RNA construct comprises the tracrRNA and the crRNA.

4. The method of claim 1, wherein each oligonucleotide is independently between about 25 and about 200 bases in length, preferably wherein each oligonucleotide is independently about 72 bases in length.

5. The method of claim 1, wherein the in vitro mutagenesis comprises at least one mutation in the nucleotide sequence of the targeted sequence selected from the group consisting of a single base nucleotide modification, a deletion, and an insertion.

6. The method of claim 1, wherein each oligonucleotide independently further comprises a chemically modified terminal linkage.

7. The method of claim 1, wherein the cell extract is selected from the group consisting of whole cell extract, cell-free extract, nuclear extract, and cytoplasmic extract, preferably:
wherein the cell extract is a eukaryotic cell extract, preferably:
wherein the eukaryotic cell extract is a mammalian cell extract, more preferably wherein the mammalian cell extract is derived from at least one cell selected from the group consisting of HEK, HUH-7, DLDl, and HCT116; or
wherein the eukaryotic cell extract is derived from S. cerevisiae.

8. The method of claim 1, wherein:
(a) the mixture comprises (i) a plurality of RNPs, each RNP comprising a crRNA complementary to a different targeted sequence as compared to crRNAs of other RNPs of the plurality, and (ii) a plurality of oligonucleotides, each oligonucleotide comprising a nucleotide sequence that is complementary to a different targeted sequence as compared to other oligonucleotides of the plurality and containing at least one mismatched nucleotide as compared to its different targeted sequence;
b) a plurality of second plasmids is generated;
(c) the plurality of second plasmids is administered to the plurality of cells; and
(d) in vitro mutagenesis has occurred in the different targeted sequences,
preferably:
wherein the first plasmid comprises one or more nucleotide sequence(s) of the different targeted sequences; or
further comprising a plurality of plasmids, each plasmid comprising one or more nucleotide sequence(s) of the different targeted sequences.

9. The method of claim 1, further comprising a second RNP comprising a second crRNA complementary to a second targeted sequence.

10. An in vitro mutagenesis kit for a targeted sequence, the kit comprising an isolated ribonucleotide particle (RNP), an oligonucleotide, a plasmid, and a cell extract having enzymatic activity for editing genes,
wherein the RNP comprises a crRNA and a Cas endonuclease,
wherein the plasmid comprises a nucleotide sequence of a targeted sequence, and
wherein the oligonucleotide comprises a nucleotide sequence that is complementary to the targeted sequence but contains at least one mismatched nucleotide as to the targeted sequence therein,
preferably:
wherein the Cas endonuclease is selected from the group consisting of Cas9, Cas3, Cas8a, Cas8b, CaslOd, Csel, Csyl, Csn2, Cas4, CaslO, Csm2, Cmr5, Fokl, T7, Cpf1, Cpf2, CasY, and CasX, more preferably wherein the Cas9 endonuclease is spCas9 or saCas9.

11. The kit of claim 10, further comprising a second RNP comprising a second crRNA complementary to a second targeted sequence.

12. The kit of claim 10, wherein the RNP further comprises a tracrRNA, preferably wherein a single RNA construct comprises the tracrRNA and the crRNA.

13. The kit of claim 10, wherein each oligonucleotide is independently between about 25 and about 200 bases in length, preferably wherein each oligonucleotide is independently about 72 bases in length.

14. The kit of claim 10, wherein each oligonucleotide independently further comprises a chemically modified terminal linkage.

15. The kit of claim 10, wherein the cell extract is selected from the group consisting of whole cell extract, cell-free extract, nuclear extract, and cytoplasmic extract, preferably:
wherein the cell extract is a eukaryotic cell extract, preferably:
wherein the eukaryotic cell extract is a mammalian cell extract, more preferably wherein the mammalian cell extract is derived from at least one cell selected from the group consisting of HEK, HUH-7, DLDl, and HCT116; or
wherein the eukaryotic cell extract is derived from *S. cerevisiae.*

## Patentansprüche

1. Verfahren zur Durchführung einer *in vitro*-Mutagenese einer Zielsequenz, wobei das Verfahren umfasst:
Inkubieren einer Mischung, die ein isoliertes Ribonukleotidpartikel (RNP), ein erstes Plasmid, ein Oligonukleotid und einen Zellextrakt mit enzymatischer Aktivität zum Editieren von Genen umfasst,
worin das RNP eine crRNA und eine Cas-Endonuklease umfasst,
worin das erste Plasmid eine Nukleotidsequenz der Zielsequenz umfasst, und
worin das Oligonukleotid eine Nukleotidsequenz umfasst, die komplementär zu der Zielsequenz ist, aber mindestens ein *Mismatch-*Nukleotid enthält,
wodurch ein zweites, mutiertes Plasmid erzeugt wird;
Verabreichen des zweiten Plasmids an mehrere Nicht-Keimbahnzellen; und
Auswählen von mindestens einer Zelle, in der eine *in-vitro*-Mutagenese in der Zielsequenz stattgefunden hat, aus den mehreren Zellen.

2. Verfahren nach Anspruch 1, worin die Cas-Endonuklease ausgewählt ist aus der Gruppe bestehend aus Cas9, Cas3, Cas8a, Cas8b, Cas10d, Cse1, Csy1, Csn2, Cas4, Cas10, Csm2, Cmr5, Fokl, T7, Cpfl, Cpf2, CasY und CasX, vorzugsweise wobei die Cas9-Endonuklease spCas9 oder saCas9 ist.

3. Verfahren nach Anspruch 1, worin die RNP ferner eine tracrRNA umfasst, vorzugsweise worin ein einzelnes RNA-Konstrukt die tracrRNA und die crRNA umfasst.

4. Verfahren nach Anspruch 1, worin jedes Oligonukleotid unabhängig zwischen etwa 25 und etwa 200 Basen lang ist, vorzugsweise worin jedes Oligonukleotid unabhängig etwa 72 Basen lang ist.

5. Verfahren nach Anspruch 1, wobei die *in vitro*-Mutagenese mindestens eine Mutation in der Nukleotidsequenz der Zielsequenz umfasst, die ausgewählt ist aus der Gruppe bestehend aus einer Einzelbasen-Nukleotidmodifikation, einer Deletion und einer Insertion.

6. Verfahren nach Anspruch 1, worin jedes Oligonukleotid unabhängig voneinander zudem eine chemisch modifizierte terminale Bindung umfasst.

7. Verfahren nach Anspruch 1, worin der Zellextrakt ausgewählt ist aus der Gruppe bestehend aus Ganzzellextrakt, zellfreiem Extrakt, Kernextrakt und Zytoplasmaextrakt, vorzugsweise:
worin der Zellextrakt ein eukaryotischer Zellextrakt ist, vorzugsweise:
worin der eukaryotische Zellextrakt ein Säugetierzellextrakt ist, noch bevorzugter, worin der Säugetierzellextrakt von mindestens einer Zelle abgeleitet ist, die ausgewählt ist aus der Gruppe bestehend aus HEK, HUH-7, DLD1 und HCT116; oder
worin der eukaryotische Zellextrakt von *S. cerevisiae* abgeleitet ist.

8. Verfahren nach Anspruch 1, wobei:
(a) das Gemisch umfasst (i) mehrere RNPs, worin jedes RNP eine crRNA umfasst, die im Vergleich zu crRNAs anderer der mehreren RNPs zu einer unterschiedlichen Zielsequenz komplementär ist, und (ii) mehrere Oligonukleotide, worin jedes Oligonukleotid eine Nukleotidsequenz umfasst, die im Vergleich zu anderen der mehreren Oligonukleotide einer unterschiedlichen Zielsequenz komplementär ist und im Vergleich zu ihrer unterschiedlichen Zielsequenz mindestens ein *Mismatch*-Nukleotid enthält;
b) mehrere zweite Plasmide erzeugt werden;
(c) die mehreren zweiten Plasmide den mehreren Zellen verabreicht werden; und
(d) eine *in vitro*-Mutagenese in den verschiedenen Zielsequenzen stattgefunden hat, vorzugsweise:
worin das erste Plasmid eine oder mehrere Nukleotidsequenzen der verschiedenen Zielsequenzen umfasst; oder
umfassend mehrere erste Plasmide, worin jedes Plasmid eine oder mehrere Nukleotidsequenzen der verschiedenen Zielsequenzen umfasst.

9. Verfahren nach Anspruch 1, welches ferner ein zweites RNP, das eine zweite crRNA umfasst, die komplementär zu einer zweiten Zielsequenz ist.

10. *In-*v*itro*-Mutagenese-Kit für eine Zielsequenz, worin das Kit ein isoliertes Ribonukleotidpartikel (RNP), ein Oligonukleotid, ein Plasmid und einen Zellextrakt mit enzymatischer Aktivität zum Editieren von Genen umfasst,
worin das RNP eine crRNA und eine Cas-Endonuklease umfasst,
worin das Plasmid eine Nukleotidsequenz einer Zielsequenz umfasst, und
worin das Oligonukleotid eine Nukleotidsequenz umfasst, die komplementär zu der Zielsequenz ist, aber mindestens ein *Mismatch*-Nukleotid in Bezug auf die Zielsequenz enthält,
vorzugsweise:
worin die Cas-Endonuklease ausgewählt ist aus der Gruppe bestehend aus Cas9, Cas3, Cas8a, Cas8b, Cas10d, Cse1, Csy1, Csn2, Cas4, Cas10, Csm2, Cmr5, Fok1, T7, Cpfl, Cpf2, CasY und CasX, besonders bevorzugt worin die Cas9-Endonuklease spCas9 oder saCas9 ist.

11. Kit nach Anspruch 10, welches ferner ein zweites RNP umfasst, das eine zweite crRNA umfasst, die komplementär zu einer zweiten Zielsequenz ist.

12. Kit nach Anspruch 10, worin die RNP ferner eine tracrRNA umfasst, vorzugsweise worin ein einziges RNA-Konstrukt die tracrRNA und die crRNA umfasst.

13. Kit nach Anspruch 10, worin jedes Oligonukleotid unabhängig zwischen etwa 25 und etwa 200 Basen lang ist, vorzugsweise worin jedes Oligonukleotid unabhängig etwa 72 Basen lang ist.

14. Kit nach Anspruch 10, worin jedes Oligonukleotid unabhängig voneinander zudem eine chemisch modifizierte terminale Bindung umfasst.

15. Kit nach Anspruch 10, worin der Zellextrakt ausgewählt ist aus der Gruppe bestehend aus Ganzzellextrakt, zellfreiem Extrakt, Kernextrakt und Zytoplasmaextrakt, vorzugsweise:
worin der Zellextrakt ein eukaryotischer Zellextrakt ist, vorzugsweise:
worin der eukaryotische Zellextrakt ein Säugetierzellextrakt ist, besonders bevorzugt, wobei der Säugetierzellextrakt von mindestens einer Zelle abgeleitet ist, die ausgewählt ist aus der Gruppe bestehend aus HEK, HUH-7, DLD1 und HCT116; oder
worin der eukaryotische Zellextrakt von *S. cerevisiae* abgeleitet ist.

## Revendications

1. Procédé pour réaliser une mutagenèse *in vitro* d'une séquence ciblée, le procédé comprenant:
l'incubation d'un mélange comprenant une particule de ribonucléotide isolée (RNP), un premier plasmide, un oligonucléotide, et un extrait cellulaire ayant une activité enzymatique pour l'édition de gènes,
dans laquelle la RNP comprend un crRNA et une endonucléase Cas,
dans lequel le premier plasmide comprend une séquence nucléotidique de la séquence ciblée, et
dans lequel l'oligonucléotide comprend une séquence nucléotidique qui est complémentaire de la séquence ciblée mais contient au moins un nucléotide *mismatch,*
générant ainsi un second plasmide muté;
l'administration du second plasmide à une pluralité de cellules qui ne sont pas des lignées germinales; et
sélectionner parmi la pluralité de cellules au moins une cellule dans laquelle une mutagenèse *in vitro* s'est produite dans la séquence ciblée.

2. Procédé selon la revendication 1, dans lequel l'endonucléase Cas est choisie dans le groupe constitué de Cas9, Cas3, Cas8a, Cas8b, Cas10d, Csel, Csy1, Csn2, Cas4, Cas10, Csm2, Cmr5, Fok1, T7, Cpfl, Cpf2, CasY et CasX, de préférence dans lequel l'endonucléase Cas9 est spCas9 ou saCas9.

3. Procédé selon la revendication 1, dans lequel le RNP comprend en outre un tracrRNA, de préférence dans lequel une construction de RNA unique comprend le tracrRNA et le crRNA.

4. Procédé selon la revendication 1, dans lequel chaque oligonucléotide est indépendamment entre environ 25 et environ 200 bases en longueur, de préférence dans lequel chaque oligonucléotide est indépendamment environ 72 bases en longueur.

5. Procédé selon la revendication 1, dans lequel la mutagenèse *in vitro* comprend au moins une mutation dans la séquence nucléotidique de la séquence ciblée choisie dans le groupe constitué par une modification nucléotidique à base unique, une délétion et une insertion.

6. Procédé selon la revendication 1, dans lequel chaque oligonucléotide comprend en outre indépendamment une liaison terminale chimiquement modifiée.

7. Procédé selon la revendication 1, dans lequel l'extrait cellulaire est choisi dans le groupe constitué par l'extrait cellulaire entières, l'extrait acellulaire, l'extrait nucléaire et l'extrait cytoplasmique, de préférence:
dans lequel l'extrait cellulaire est un extrait cellulaire eucaryote, de préférence:
dans lequel l'extrait cellulaire eucaryote est un extrait cellulaire de mammifères, plus préférablement dans lequel l'extrait cellulaire de mammifères est dérivé d'au moins une cellule choisie dans le groupe constitué par HEK, HUH-7, DLD1 et HCT116; ou
dans lequel l'extrait cellulaire eucaryote est dérivé de *S. cerevisiae.*

8. Procédé selon la revendication 1, dans lequel:
(a) le mélange comprend (i) une pluralité de RNP, chaque RNP comprenant un crRNA complémentaire d'une séquence ciblée différente par rapport aux crRNA des autres RNP de la pluralité, et (ii) une pluralité d'oligonucléotides, chaque oligonucléotide comprenant une séquence nucléotidique qui est complémentaire d'une séquence ciblée différente par rapport aux autres oligonucléotides de la pluralité et contenant au moins un nucléotide *mismatch* par rapport à sa séquence ciblée différente;
b) une pluralité de seconds plasmides est générée;
(c) la pluralité de seconds plasmides est administrée à la pluralité de cellules; et
(d) une mutagenèse *in vitro* s'est produite dans les différentes séquences ciblées,
de préférence:
dans lequel le premier plasmide comprend une ou plusieurs séquences nucléotidiques des différentes séquences ciblées; ou
comprenant en outre une pluralité de plasmides, chaque plasmide comprenant une ou plusieurs séquences nucléotidiques des différentes séquences ciblées.

9. Procédé selon la revendication 1, comprenant en outre un second RNP comprenant un second crRNA complémentaire d'une seconde séquence ciblée.

10. Kit de mutagenèse *in vitro* pour une séquence ciblée, le kit comprenant une particule ribonucléotidique (RNP) isolée, un oligonucléotide, un plasmide, et un extrait cellulaire ayant une activité enzymatique pour éditer des gènes,
dans laquelle la RNP comprend un crRNA et une endonucléase Cas,
dans lequel le plasmide comprend une séquence nucléotidique d'une séquence ciblée, et
dans lequel l'oligonucléotide comprend une séquence nucléotidique qui est complémentaire de la séquence ciblée mais contient au moins un nucléotide *mismatch* à la séquence ciblée,
de préférence:
dans lequel l'endonucléase Cas est choisie dans le groupe constitué par Cas9, Cas3, Cas8a, Cas8b, Cas10d, Csel, Csy1, Csn2, Cas4, Cas10, Csm2, Cmr5, Fok1, T7, Cpfl, Cpf2, CasY et CasX, plus préférablement dans lequel l'endonucléase Cas9 est spCas9 ou saCas9.

11. Kit selon la revendication 10, comprenant en outre un second RNP comprenant un second crRNA complémentaire d'une seconde séquence ciblée.

12. Kit selon la revendication 10, dans lequel le RNP comprend en outre un tracrRNA, de préférence dans lequel une construction RNA unique comprend le tracrRNA et le crRNA.

13. Kit selon la revendication 10, dans lequel chaque oligonucléotide est indépendamment entre environ 25 et environ 200 bases en longueur, de préférence dans lequel chaque oligonucléotide est indépendamment environ 72 bases en longueur.

14. Kit de la revendication 10, dans lequel chaque oligonucléotide comprend en outre indépendamment une liaison terminale chimiquement modifiée.

15. Kit selon la revendication 10, dans lequel l'extrait cellulaire est choisi dans le groupe constitué par l'extrait cellulaire entières, l'extrait acellulaire, l'extrait nucléaire et l'extrait cytoplasmique, de préférence:
dans lequel l'extrait cellulaire est un extrait cellulaire eucaryote, de préférence:
dans lequel l'extrait cellulaire eucaryote est un extrait cellulaire de mammifères, plus préférablement dans lequel l'extrait cellulaire de mammifères est dérivé d'au moins une cellule choisie dans le groupe constitué par HEK, HUH-7, DLD1 et HCT116; ou
dans lequel l'extrait cellulaire eucaryote est dérivé de *S. cerevisiae.*
